# EUROPÄISCHE PATENTSCHRIFT

(11) **EP 1 392 695 B1**
(45) Veröffentlichungstag und Bekanntmachung des Hinweises auf die Patenterteilung: **04.10.2006**
(21) Anmeldenummer: 02766635.3
(22) Anmeldetag: 23.04.2002
(51) Int. Cl.: C07D 487/04, A01N 43/90

(54) **TRIAZOLOPYRIMIDINE**
TRIAZOLOPYRIMIDINES
TRIAZOLOPYRIMIDINES

(30) Priorität: 27.04.2001 DE 10121102
(43) Veröffentlichungstag der Anmeldung: 03.03.2004
(73) Patentinhaber: Bayer CropScience AG, 40789 Monheim (DE)
(72) Erfinder: GEBAUER, Olaf, 50737 Köln (DE); GREUL, Jörg, Nico, 42799 Leichlingen (DE); HEINEMANN, Ulrich, 42799 Leichlingen (DE); ELBE, Hans-Ludwig, 42329 Wuppertal (DE); KRÜGER, Bernd-Wieland, 51467 Bergisch Gladbach (DE); DUNKEL, Ralf, 40789 Monheim (DE); VOERSTE, Arnd, 50677 Köln (DE); EBBERT, Ronald, 40789 Monheim (DE); WACHENDORFF-NEUMANN, Ulrike, 56566 Neuwied (DE); KUCK, Karl-Heinz, 40764 Langenfeld (DE); KITAGAWA, Yoshinori, Moka-shi, Tochigi 321-4305 (JP)
(86) Internationale Anmeldenummer: PCT/EP2002/004441
(87) Internationale Veröffentlichungsnummer: WO 2002/088127

(56) Entgegenhaltungen:
- EP-A- 0 834 513
- US-A- 4 981 507
- US-A- 5 965 561

## Beschreibung

Die vorliegende Erfindung betrifft neue Triazolopyrimidine, mehrere Verfahren zu deren Herstellung und deren Verwendung zur Bekämpfung von unerwünschten Mikroorganismen.

Es ist bereits bekannt geworden, dass bestimmte Triazolopyrimidine fungizide Eigenschaften besitzen (vgl. EP-A 0 550 113, WO 94-20 501, EP-A 0 613 900, US-A 5 612 345, EP-A 0 834 513, WO 98-46 607 und WO 98-46 608). Die Wirksamkeit dieser Stoffe ist gut, lässt aber bei niedrigen Aufwandmengen in manchen Fällen zu wünschen übrig.

Es wurden nun neue Triazolopyrimidine der Formel 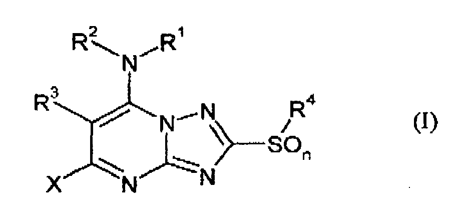 in welcher
- R¹: für Amino, für jeweils gegebenenfalls substituiertes Alkyl, gegebenenfalls substituiertes Alkenyl, gegebenenfalls substituiertes Alkinyl, gegebenenfalls substituiertes Cyloalkyl, gegebenenfalls substituiertes Alkoxy, gegebenenfalls substituiertes Alkenyloxy, gegebenenfalls substituiertes Alkinyloxy, gegebenenfalls substituiertes Cycloalkyloxy, gegebenenfalls substituiertes Alkylamino, gegebenenfalls substituiertes Dialkylamino, gegebenenfalls substituiertes Alkenylamino, gegebenenfalls substituiertes Alkinylamino, gegebenenfalls substituiertes Cycloalkylamino, gegebenenfalls substituiertes N-Cycloalkyl-N-alkyl-amino, gegebenenfalls substituiertes Alkylidenamino, gegebenenfalls substituiertes Heterocyclyl oder für einen Rest der Formel -S-R⁵ steht, worin
R⁵ für gegebenenfalls substituiertes Alkyl, gegebenenfalls substituiertes Alkenyl, gegebenenfalls substituiertes Alkinyl oder gegebenenfalls substituiertes Cycloalkyl steht,
- R²: für Wasserstoff, gegebenenfalls substituiertes Alkyl, gegebenenfalls substituiertes Alkenyl, gegebenenfalls substituiertes Alkinyl oder gegebenenfalls substituiertes Cycloalkyl steht,
oder
- R¹ und R²: gemeinsam mit dem Stickstoffatom, an das sie gebunden sind, für einen gegebenenfalls substituierten heterocyclischen Ring stehen,
- R³: für gegebenenfalls substituiertes Aryl steht,
- R⁴: für gegebenenfalls substituiertes Alkyl, gegebenenfalls substituiertes Alkenyl oder gegebenenfalls substituiertes Alkinyl steht,
- X: für Halogen steht und
- n: für 0, 1 oder 2 steht,
sowie Säureadditions-Salze von denjenigen Verbindungen der Formel (I),
in denen
- R¹: für Amino steht,
gefunden.

Die erfindungsgemäßen Verbindungen können je nach Substitutionsmuster gegebenenfalls als Mischungen verschiedener möglicher isomerer Formen, insbesondere von Stereoisomeren, wie z.B. E- und Z-, threo- und erythro-, sowie optischen Isomeren, gegebenenfalls aber auch von Tautomeren vorliegen. Ist R³ an beiden Atomen, die der Bindungsstelle benachbart sind, ungleich substituiert, können die betreffenden Verbindungen in einer besonderen Form der Stereoisomerie, als Atropisomere, vorliegen.

Weiterhin wurde gefunden, dass sich Triazolopyrimidine der Formel (I) herstellen lassen, indem man
a) Dihalogen-triazolopyrimidine der Formel 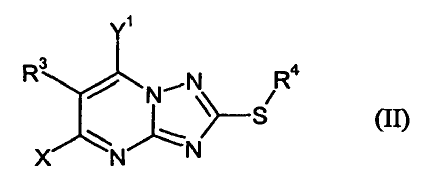 in welcher
   R³, R⁴ und X die oben angegebenen Bedeutungen haben und
   - Y¹: für Halogen steht,
   mit Aminen der Formel 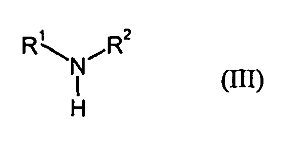 in welcher
   R¹ und R² die oben angegebenen Bedeutungen haben,
   gegebenenfalls in Gegenwart eines Verdünnungsmittels und gegebenenfalls in Gegenwart eines Säureakzeptors umsetzt,
   oder
b) Triazolopyrimidine der Formel 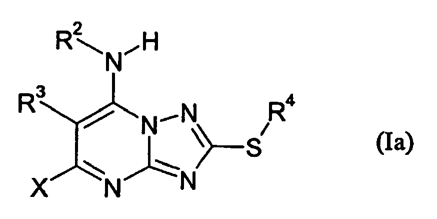 in welcher
   R², R³, R⁴ und X die oben angegebenen Bedeutungen haben,
   mit Sulfensäurehalogeniden der Formel

   Y²-S-R⁵ (IV)

   in welcher
   - R⁵: die oben angegebenen Bedeutungen hat und
   - Y²: für Halogen steht,
   gegebenenfalls in Gegenwart eines Verdünnungsmittels und gegebenenfalls in Gegenwart eines Säureakzeptors umsetzt,
   oder
c) Triazolopyrimidine der Formel 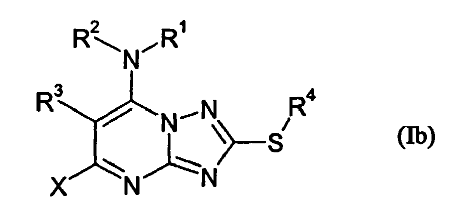 in welcher
   R¹, R², R³, R⁴ und X die oben angegebenen Bedeutungen haben,
   mit Sauerstoff abgebenden Oxidationsmitteln gegebenenfalls in Gegenwart eines Verdünnungsmittels und gegebenenfalls in Gegenwart eines Katalysators umsetzt,
und gegebenenfalls an die so erhaltenen Verbindungen der Formel (I),
in denen
- R¹: für Amino steht,
eine Säure addiert.

Schließlich wurde gefunden, dass sich die neuen Triazolopyrimidine der Formel (I) bzw. deren Säureadditions-Salze sehr gut zur Bekämpfung von unerwünschten Mikroorganismen eignen. Sie zeigen vor allem eine starke fungizide Wirksamkeit und lassen sich sowohl im Pflanzenschutz als auch im Materialschutz verwenden.

Überraschenderweise besitzen die erfindungsgemäßen Triazolopyrimidine der Formel (I) eine wesentlich bessere mikrobizide Wirksamkeit als die konstitutionell ähnlichsten, vorbekannten Stoffe gleicher Wirkungsrichtung.

Die erfindungsgemäßen Triazolopyrimidine sind durch die Formel (I) allgemein definiert.
- R¹: steht bevorzugt für Amino;
für gegebenenfalls durch Halogen, Cycloalkyl, Cyano, Phenyl, Heterocyclyl, Alkoxy mit 1 bis 4 Kohlenstoffatomen, Alkylthio mit 1 bis 4 Kohlenstoffatomen, Oxo, Hydroxoimino und/oder Alkoximino mit 1 bis 4 Kohlenstoffatomen substituiertes Alkyl mit 1 bis 6 Kohlenstoffatomen,
für gegebenenfalls durch Hydroxy, Alkenyloxy mit 2 bis 4 Kohlenstoffatomen, Alkoxycarbonyl mit 1 bis 4 Kohlenstoffatomen substituiertes Alkyl mit 1 bis 6 Kohlenstoffatomen,
für gegebenenfalls durch Halogen, Cycloalkyl, Cyano, Phenyl und/oder Heterocyclyl substituiertes Alkenyl mit 2 bis 6 Kohlenstoffatomen,
für gegebenenfalls durch Halogen, Cycloalkyl, Cyano, Phenyl und/oder Heterocyclyl substituiertes Alkinyl mit 2 bis 6 Kohlenstoffatomen,
für gegebenenfalls durch Halogen, Cycloalkyl, Cyano, Phenyl und/oder Heterocyclyl substituiertes Cycloalkyl mit 3 bis 7 Kohlenstoffatomen,
für gegebenenfalls durch Halogen, Cyloalkyl, Cyano, Phenyl und/oder Heterocyclyl substituiertes Alkoxy mit 1 bis 7 Kohlenstoffatomen,
für gegebenenfalls durch Halogen, Cyloalkyl, Cyano, Phenyl und/oder Heterocyclyl substituiertes Alkenyloxy mit 2 bis 6 Kohlenstoffatomen,
für gegebenenfalls durch Halogen, Cyloalkyl, Cyano, Phenyl und/oder Heterocyclyl substituiertes Alkinyloxy mit 2 bis 6 Kohlenstoffatomen,
für gegebenenfalls durch Halogen, Cyloalkyl, Cyano, Phenyl und/oder Heterocyclyl substituiertes Cycloalkyloxy mit 3 bis 7 Kohlenstoffatomen,
für gegebenenfalls durch Halogen, Cyloalkyl, Cyano, Phenyl und/oder Heterocyclyl substituiertes Alkylamino mit 1 bis 7 Kohlenstoffatomen,
für gegebenenfalls durch Halogen, Cyloalkyl, Cyano, Phenyl und/oder Heterocyclyl substituiertes Dialkylamino mit 1 bis 7 Kohlenstoffatomen in jedem der Alkylreste,
für gegebenenfalls durch Halogen, Cyloalkyl, Cyano, Phenyl und/oder Heterocyclyl substituiertes Alkenylamino mit 2 bis 6 Kohlenstoffatomen,
für gegebenenfalls durch Halogen, Cyloalkyl, Cyano, Phenyl und/oder Heterocyclyl substituiertes Alkinylamino mit 2 bis 6 Kohlenstoffatomen,
für gegebenenfalls durch Halogen, Cyloalkyl, Cyano, Phenyl und/oder Heterocyclyl substituiertes Cycloalkylamino mit 3 bis 7 Kohlenstoffatomen,
für gegebenenfalls durch Halogen, Cyloalkyl, Cyano, Phenyl und/oder Heterocyclyl substituiertes N-Cycloalkyl-N-alkyl-amino mit 3 bis 7 Kohlenstoffatomen im Cycloalkylteil und 1 bis 7 Kohlenstoffatomen in Alkylteil,
für gegebenenfalls durch Halogen, Cyloalkyl, Cyano, Phenyl und/oder Heterocyclyl substituiertes Alkylidenamino mit 2 bis 6 Kohlenstoffatomen,
für gegebenenfalls durch Halogen, Alkyl, Cycloalkyl, Cyano, Phenyl und/oder Heterocyclyl substituiertes Heterocyclyl mit 5 oder 6 Ringgliedern oder
für -S-R⁵, worin
R⁵ für gegebenenfalls durch Halogen, Cycloalkyl, Cyano, Phenyl und/oder Heterocyclyl substituiertes Alkyl mit 1 bis 6 Kohlenstoffatomen,
für gegebenenfalls durch Halogen, Cycloalkyl, Cyano, Phenyl und/oder Heterocyclyl substituiertes Alkenyl mit 2 bis 6 Kohlenstoffatomen,
für gegebenenfalls durch Halogen, Cycloalkyl, Cyano, Phenyl und/oder Heterocyclyl substituiertes Alkinyl mit 2 bis 6 Kohlenstoffatomen oder
für gegebenenfalls durch Halogen, Cycloalkyl, Cyano, Phenyl und/oder Heterocyclyl substituiertes Cycloalkyl mit 3 bis 7 Kohlenstoffatomen steht, wobei die zuvor genannten Heterocyclyl-Reste einfach bis dreifach, gleichartig oder verschieden substituiert sein können durch Halogen, Alkyl mit 1 bis 4 Kohlenstoffatomen, Halogenalkyl mit 1 oder 2 Kohlenstoffatomen und 1 bis 5 Halogenatomen, Alkoxy mit 1 bis 4 Kohlenstoffatomen, Alkylthio mit 1 bis 4 Kohlenstoffatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen und/oder Phenyl,
und wobei die zuvor genannten Phenyl-Reste einfach bis dreifach, gleichartig oder verschieden substituiert sein können durch
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen; jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen,
oder einfach substituiert sein können durch zweifach in ortho-Stellung verknüpftes Alkylen mit 3 oder 4 Kohlenstoffatomen oder Dioxyalkylen mit 1 oder 2 Kohlenstoffatomen, wobei diese Reste einfach bis vierfach, gleichartig oder verschieden substituiert sein können durch Halogen, Alkyl mit 1 bis 4 Kohlenstoffatomen und/oder Halogenalkyl mit 1 bis 4 Kohlenstoffaromen und 1 bis 9 gleichen oder verschiedenen Halogenatomen.
- R²: steht bevorzugt für Wasserstoff,
für gegebenenfalls durch Halogen, Alkoxy mit 1 bis 4 Kohlenstoffatomen, Alkylthio mit 1 bis 4 Kohlenstoffatomen, Oxo, Hydroximino, Alkoximino mit 1 bis 4 Kohlenstoffatomen und/oder Cycloalkyl mit 3 bis 6 Kohlenstoffatomen substituiertes Alkyl mit 1 bis 4 Kohlenstoffatomen,
für gegebenenfalls durch Halogen und/oder Cycloalkyl mit 3 bis 6 Kohlenstoffatomen substituiertes Alkenyl mit 2 bis 4 Kohlenstoffatomen oder
für gegebenenfalls durch Halogen und/oder Cycloalkyl mit 3 bis 6 Kohlenstoffatomen substituiertes Alkinyl mit 2 bis 4 Kohlenstoffatomen oder
für gegebenenfalls durch Halogen und/oder Alkyl mit 1 bis 4 Kohlenstoffatomen substituiertes Cycloalkyl mit 3 bis 6 Kohlenstoffatomen steht,
- R¹ und R²: stehen bevorzugt gemeinsam mit dem Stickstoffatom, an das sie gebunden sind, für einen 3- bis 6-gliedrigen heterocyclischen Ring, der einfach bis dreifach, gleichartig oder verschieden substituiert sein kann durch
Alkyl mit 1 bis 4 Kohlenstoffatomen,
Halogenalkyl mit 1 bis 4 Kohlenstoffatomen und 1 bis 9 gleichen oder verschiedenen Halogenatomen,
Fluor, Chlor, Brom, Iod, Hydroxy, Cyano, Oxo;
Alkylcarbonyl mit 1 bis 4 Kohlenstoffatomen,
Alkoxycarbonyl mit 1 bis 4 Kohlenstoffatomen,
Aminoalkyl mit 1 bis 4 Kohlenstoffatomen,
Alkylcarbonylamino mit 1 bis 4 Kohlenstoffatomen oder
an dem ein Phenylring anelliert ist.
- R³: steht bevorzugt für Phenyl, das einfach bis fünffach, gleichartig oder verschieden substituiert sein kann durch
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen,
oder einfach substituiert sein kann durch zweifach in ortho-Stellung verknüpftes Alkylen mit 3 oder 4 Kohlenstoffatomen oder Dioxyalkylen mit 1 oder 2 Kohlenstoffatomen, wobei diese Reste einfach bis vierfach, gleichartig oder verschieden substituiert sein können durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen.
- R⁴: steht bevorzugt für gegebenenfalls durch Halogen und/oder Cyano substituiertes Alkyl mit 1 bis 6 Kohlenstoffatomen,
für gegebenenfalls durch Halogen und/oder Cyano substituiertes Alkenyl mit 3 bis 6 Kohlenstoffatomen,
für gegebenenfalls durch Halogen und/oder Cyano substituiertes Alkinyl mit 3 bis 6 Kohlenstoffatomen oder
für gegebenenfalls durch Halogen substituiertes Aralkyl mit 6 bis 10 Kohlenstoffatomen im Arylteil und 1 bis 4 Kohlenstoffatomen im Arylteil und 1 bis 4 Kohlenstoffatomen im Alkylteil steht,
- X: steht bevorzugt für Fluor, Chlor oder Brom.
- n: steht bevorzugt für 0, 1 oder 2.
- R¹: steht besonders bevorzugt für Methyl, Ethyl, n-Propyl, i-Propyl, n-, i-, s- oder t-Butyl, Methoxymethyl, 2-Methoxy-ethyl, Methylthio-methyl, 2-Methylthiomethyl, Hydroximino-methyl, Methoximino-methyl, Acetylmethyl, 2-(Hydroximino)-propyl, 2-(Methoximino)propyl,
oder
für 2-Ethoxycarbonyl-ethyl, 2-Vinyloxy-ethyl, 2-Hydroxyimino-ethyl, 2-Methoxyimino-ethyl, 2-Hydroxyethyl, 2-Chlorethyl, 2-Methyl-1-propyl, 1,2-Dimethylpropyl, Thienylmethyl, amino;
oder
für Allyl, 2-Methyl-prop-2-enyl, Propargyl, 2,2,2-Trifluorethyl, 1-(Trifluormethyl)-ethyl, 3,3,3-Trifluorpropyl, Cyclopentyl, Cyclohexyl, Cyclopropylmethyl, Cyclobutylmethyl, Cyclopentylmethyl oder Cyclohexylmethyl, Methoxy, Ethoxy, n- oder i-Propoxy, n-, i-, s- oder t-Butoxy, Allyloxy, Propargyloxy, Cyclopropyloxy, Cyclobutyloxy, Cyclopentyloxy oder Cyclohexyloxy, Difluormethoxy, Trifluormethoxy, Difluorchlormethoxy, Trifluorethoxy, Methylamino, Ethylamino, n- oder i-Propylamino, n-, i-, s- oder t-Butylamino, Dimethylamino, Diethylamino, Trifluorethylamino, Cyclohexylmethylamino, 2-Cyanethylamino, Allylamino, 1-Cyclopropylethylamino, Cyclopropylamino, Cyclobutylamino, Cyclopentylamino, Cyclohexylamino, 1-Methylethylidenamino, Benzyloxy, Piperidinyl, Morpholinyl, Pyridylmethoxy, Thiazolylmethoxy oder für -S-R⁵ steht, worin
R⁵ für Methyl, Ethyl, n- oder i-Propyl, Difluormethyl, Difluorchlormethyl, Dichlorfluormethyl oder Trifluormethyl steht, wobei die zuvor genannten Thiazolyl- und Pyridyl-Reste im Falle von Thiazolyl einfach oder zweifach und im Falle von Pyridyl einfach bis dreifach, jeweils gleichartig oder verschieden substituiert sein können durch Fluor, Chlor Brom, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, Difluormethoxy, Trifluormethoxy, Difluorchlormethoxy, Trifluorethoxy, Difluormethylthio, Difluorchlormethylthio, Dichlorfluormethylthio, Trifluormethylthio und/oder Phenyl,
und wobei das zuvor genannte Benzyloxy im Phenylteil einfach bis dreifach, gleichartig oder verschieden substituiert sein kann durch
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl,
oder einfach substituiert sein kann durch zweifach in ortho-Stellung verknüpftes Propan-1,3-diyl, Methylendioxy oder Ethylendioxy, wobei diese Reste einfach bis vierfach, gleichartig oder verschieden substituiert sein können durch Fluor, Chlor, Methyl, Ethyl, n-Propyl, i-Propyl und/oder Trifluormethyl,
- R²: steht besonders bevorzugt für Wasserstoff, Methyl, Ethyl, n- oder i-Propyl, n-, i-, s- oder t-Butyl, Cyclopropyl, Methoxy-methyl, 2-Methoxy-ethyl, Methylthio-methyl, 2-Methylthio-methyl, Hydroximino-methyl, Methoximino-methyl, Acetylmethyl, 2-Hydroximino-propyl, 2-Methoximino-propyl,
oder
für Allyl, Propargyl, 2,2,2-Trifluorethyl, 1-(1,1,1-Trifluormethyl)ethyl, Cyclopropylmethyl, Cyclobutylmethyl, Cyclopentylmethyl oder Cyclohexylmethyl.
- R¹ und R²: stehen besonders bevorzugt gemeinsam mit dem Stickstoffatom, an das sie gebunden sind,
für gegebenenfalls durch Methyl, Ethyl oder Trifluorethyl substituiertes Pyrrolyl, Piperidinyl, Morpholinyl oder Piperazinyl,
für einfach bis dreifach durch Fluor, Chlor, Brom, Hydroxy, Oxo, Methoxy, Amino, Methylamino, Dimethylamino, Acetylamino, Chlormethyl, Dichlormethyl, Fluormethyl, Trifluormethyl, Aminomethyl, Methoxycarbonyl, Methylcarbonyl, Morpholinyl, 1,3-Dioxolanyl, tert.-Butoxycarbonylamino substituiertes Piperidinyl,
für durch Phenyl anelliertes Piperidinyl,
für unsubstituiertes oder einfach bis dreifach durch Hydroxy, Dimethylaminom, Acetylamino, 1,3-Propandiyl substituiertes Pyrrolidinyl,
für unsubstituiertes oder durch Methyl substituiertes Piperazin, Tetrahydrothiazin oder Tetrahydropyridin.
- R³: steht besonders bevorzugt für Phenyl steht, das einfach bis dreifach, gleichartig oder verschieden substituiert sein kann durch
Fluor, Chlor, Brom, Cyano, Nitro, Formyl, Methyl, Ethyl, n- oder i-Propyl, n-, i-, s- oder t-Butyl, Allyl, Propargyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Allyloxy, Propargyloxy, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Iodpropargyloxy, Methylamino, Ethylamino, n- oder i-Propylamino, Dimethylamino, Diethylamino, Acetyl, Propionyl, Acetyloxy, Methoxycarbonyl, Ethoxycarbonyl, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl,
oder einfach substituiert sein kann durch zweifach in ortho-Stellung verknüpftes Propan-1,3-diyl, Methylendioxy oder Ethylendioxy, wobei diese Reste einfach bis vierfach, gleichartig oder verschieden substituiert sein können durch Fluor, Chlor, Methyl, Ethyl, n-Propyl i-Propyl und/oder Trifluormethyl,
- R⁴: steht besonders bevorzugt für Methyl, Ethyl, n- oder i-Propyl, n-, i-, s- oder t-Butyl, Allyl, Propargyl, Benzyl oder Chlorbenzyl.
- X: steht besonders bevorzugt für Fluor oder Chlor.

Ganz besonders bevorzugt sind diejenigen Verbindungen der Formel (I), in denen
R¹, R², R⁴, X und n die zuvor genannten bevorzugten Bedeutungen haben und
- R³: für 2-substituiertes, 2,4-disubstituiertes, 2,6-disubstituiertes oder 2,4,6-trisubstituiertes Phenyl steht.

Ganz besonders bevorzugt sind Verbindungen der Formel (I), in denen
- R⁴: für Methyl und
- X: für Chlor steht und
R¹, R², R³ und n die vorstehend genannten Bedeutungen haben.

Ganz besonders bevorzugt sind Verbindungen der Formel (I), in denen
- R¹ und R²: gemeinsam mit dem Stickstoffatom, an welches sie gebunden sind, für gegebenenfalls substituiertes Piperidinyl stehen, wobei die möglichen Substituenten bereits vorstehend erwähnt werden und
R³, R⁴, X und n die vorstehend genannten Bedeutungen haben.

Ganz besonders bevorzugt sind Verbindungen der Formel (I), in denen
- R²: für Wasserstoff, Methyl, Ethyl oder iso-Propyl steht und
R¹, R³, R⁴, X und n eine der vorstehend genannten Bedeutungen haben.

Die zuvor genannten Reste-Definitionen können untereinander in beliebiger Weise kombiniert werden. Außerdem können auch einzelne Bedeutungen entfallen.

Bevorzugte erfindungsgemäße Verbindungen sind auch Additionsprodukte aus Säuren und denjenigen Triazolopyrimidinen der Formel (I), in denen
- R¹: für Amino steht und
R², R³, R⁴, X und n diejenigen Bedeutungen haben, die für diese Reste bzw. den Index n als bevorzugt genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäuer, mono- und bifunktionelle Carbonsäuren und Hydroxycarbonsäuren, wie z.B. Essigsäure, Maleinsäure, Bernsteinsäure, Fumarssäure, Weinsäure, Zitronensäure, Salicylsäure, Sorbinsäure und Milchsäure, sowie Sulfonsäuren, wie z.B. p-Toluolsulfonsäure, 1,5-Naphthalindisulfonsäure, Saccharin und Thiosaccharin.

Verwendet man 5,7-Dichlor-2-(methylsulfanyl)-6-(2,4,6-trifluorphenyl)-[1,2,4]-triazolo[1,5-a]pyrimidin und 4-Trifluormethyl-piperidin als Ausgangsstoffe, so kann der Verlauf des erfindungsgemäßen Verfahrens (a) durch das folgende Formelschema veranschaulicht werden.

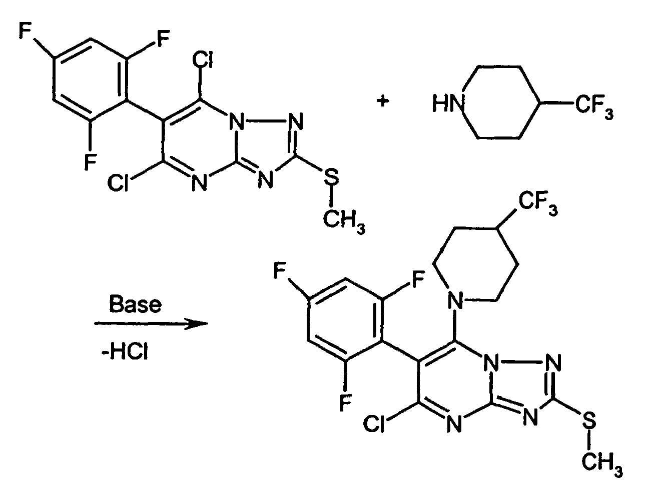

Verwendet man 5-Chlor-2-(methylsulfanyl)-N-[(1S)-2,2,2-trifluor-1-methyl-ethyl]-6-(2,4,6-trifluorphenyl)-[1,2,4]-triazolo-[1,5-a]pyrimidin-7-amin und Dichlorfluormethan-sulfenylchlorid als Ausgangsstoffe, so kann der Verlauf des erfindungsgemäßen Verfahrens (b) durch das folgende Formelschema veranschaulicht werden.

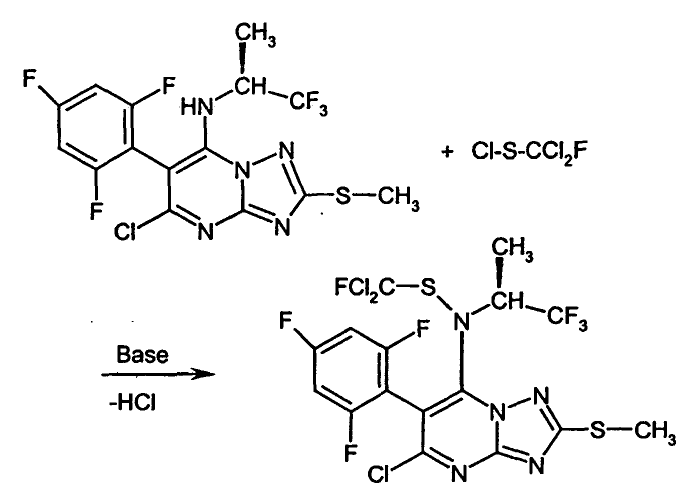

Verwendet man 5-Chlor-2-(methyl-sulfanyl)-N-[(1S)-2,2,2-trifluor-1-methyl-ethyl]-6-(2,4,6-trifluorphenyl)-[1,2,4]-triazolo[1,5-a]pyrimidin-7-amin als Ausgangsstoff und einen Überschuss an Wasserstoffperoxid als Oxidationsmittel, so kann der Verlauf des erfindungsgemäßen Verfahrens (c) durch das folgende Formelschema veranschaulicht werden:

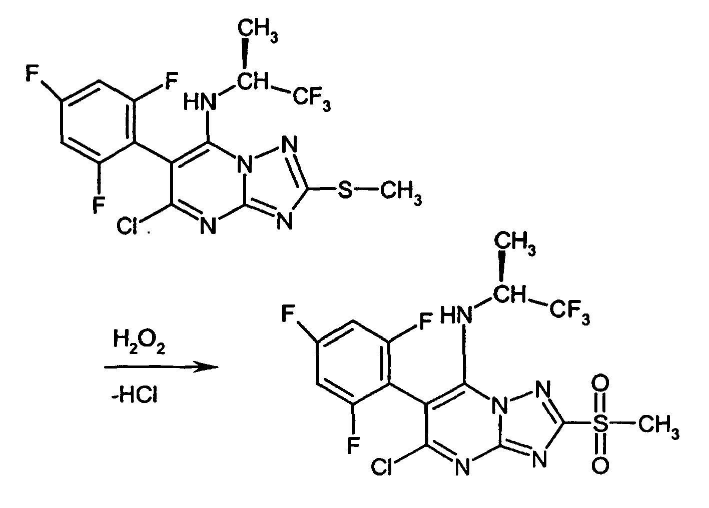

Die bei der Durchführung des erfindungsgemäßen Verfahrens (a) als Ausgangsstoffe benötigten Dihalogen-triazolo-pyrimidine sind durch die Formel (II) allgemein definiert. In dieser Formel haben R³, R⁴ und X vorzugsweise diejenigen Bedeutungen, die bereits im Zusammenhang mit der Beschreibung der erfindungsgemäßen Stoffe der Formel (I) für diese Reste als bevorzugt genannt wurden. Y¹ steht vorzugsweise für Fluor, Chlor oder Brom, besonders bevorzugt für Fluor oder Chlor.

Die Dihalogen-triazolopyrimidine der Formel (II) sind neu. Auch diese Stoffe eignen sich zur Bekämpfung von unerwünschten Mikroorganismen.

Die Dihalogen-triazolopyrimidine lassen sich herstellen, indem man
d) Dihydroxy-triazolo-pyrimidine der Formel 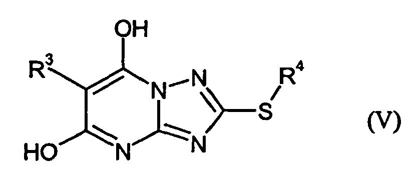 in welcher
   R³ und R⁴ die oben angegebenen Bedeutungen haben,
   mit Halogenierungsmitteln, gegebenenfalls in Gegenwart eines Verdünnungsmittels umsetzt.

Die bei der Durchführung des Verfahrens (d) als Ausgangsstoffe benötigten Dihydroxy-triazolopyrimidine sind durch die Formel (V) allgemein definiert. In dieser Formel haben R³ und R⁴ vorzugsweise diejenigen Bedeutungen, die bereits im Zusammenhang mit der Beschreibung der erfindungsgemäßen Stoffe der Formel (I) für diese Reste als bevorzugt genannt wurden.

Auch die Dihydroxy-triazolopyrimidine der Formel (V) sind bisher noch nicht bekannt. Sie lassen sich herstellen, indem man
e) Arylmalonester der Formel 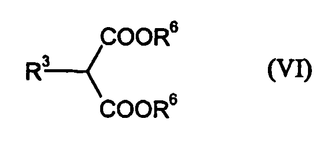 in welcher
   - R³: die oben angegebenen Bedeutungen hat und
   - R⁶: für Alkyl steht mit 1 bis 4 Kohlenstoffatomen steht,
   mit Aminotriazolen der Formel 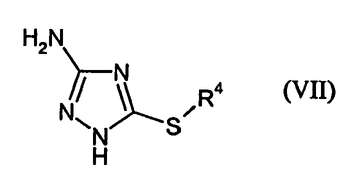 in welcher
   - R⁴: die oben angegebenen Bedeutungen hat,
   gegebenenfalls in Gegenwart eines Verdünnungsmittels und gegebenenfalls in Gegenwart eines Säurebindemittels umsetzt.

Die bei der Durchführung des Verfahrens (e) als Ausgangsstoffe benötigten Arylmalonester sind durch die Formel (VI) allgemein definiert. In dieser Formel hat R³ vorzugsweise diejenigen Bedeutungen, die bereits im Zusammenhang mit der Beschreibung der erfindungsgemäßen Stoffe der Formel (I) für diesen Rest als bevorzugt genannt wurden. R⁶ steht vorzugsweise für Methyl oder Ethyl.

Die Arylmalonester der Formel (VI) sind bekannt oder lassen sich nach bekannten Methoden herstellen (vgl. US-A 6 156 925).

Die bei der Durchführung des Verfahrens (e) weiterhin als Ausgangsstoffe benötigten Aminotriazole sind durch die Formel (VII) allgemein definiert. In dieser Formel hat R⁴ vorzugsweise diejenigen Bedeutungen, die bereits im Zusammenhang mit der Beschreibung der erfindungsgemäßen Stoffe der Formel (I) für diesen Rest als bevorzugt genannt wurden.

Die Aminotriazole der Formel (VII) sind bekannt oder können nach bekannten Methoden hergestellt werden (vgl. J. Heterocycl. Chem. (1982), 19(5), 1157-64).

Als Verdünnungsmittel kommen bei der Durchführung des Verfahrens (e) alle für derartige Umsetzungen üblichen, inerten organischen Solventien infrage. Vorzugsweise verwendbar sind Alkohole, wie Methanol, Ethanol, n-Propanol, i-Propanol, n-Butanol und tert.-Butanol.

Als Säurebindemittel kommen bei der Durchführung des Verfahrens (e) alle für derartige Umsetzungen üblichen anorganischen und organischen Basen in Betracht. Vorzugsweise verwendbar sind tertiäre Amine, wie Tributylamin oder Pyridin. Im Überschuss eingesetztes Amin kann auch als Verdünnungsmittel fungieren.

Die Temperaturen können bei der Durchführung des Verfahrens (e) in einem größeren Bereich variiert werden. Im Allgemeinen arbeitet man bei Temperaturen zwischen 20°C und 200°C, vorzugsweise zwischen 50°C und 180°C.

Bei der Durchführung des Verfahrens (e) arbeitet man im Allgemeinen unter Atmosphärendruck. Es ist allerdings auch möglich, unter erhöhtem oder vermindertem Druck zu arbeiten.

Bei der Durchführung des Verfahrens (e) setzt man Arylmalonester der Formel (VI) und Aminotriazol der Formel (VII) im Allgemeinen in äquimolaren Mengen um. Es ist aber auch möglich, die eine oder andere Komponente in einem Überschuss zu verwenden. Die Aufarbeitung erfolgt nach üblichen Methoden.

Als Halogenierungsmittel kommen bei der Durchführung des Verfahrens (d) alle für den Ersatz von Hydroxygruppen durch Halogen üblichen Komponenten in Betracht. Vorzugsweise verwendbar sind Phosphortrichlorid, Phosphotribormid, Phosphorpentachlorid, Phosphoroxychlorid, Thionylchlorid, Thionylbromid oder deren Gemische. Die entsprechenden Fluor-Verbindungen der Formel (II) lassen sich aus den Chlor- oder Brom-Verbindungen durch Umsetzung mit Kaliumfluorid herstellen.

Als Verdünnungsmittel kommen bei der Durchführung des Verfahrens (d) alle für derartige Halogenierungen üblichen Solventien infrage. Vorzugsweise verwendbar sind halogenierte aliphatische oder aromatische Kohlenwasserstoffe, wie Chlorbenzol. Als Verdünnungsmittel kann aber auch das Halogenierungsmittel selbst, z.B. Phosphoroxychlorid oder ein Gemisch von Halogenierungsmitteln fungieren.

Die Temperaturen können auch bei der Durchführung des Verfahrens (d) in einem größeren Bereich variiert werden. Im Allgemeinen arbeitet man bei Temperaturen zwischen 0°C und 150°C, vorzugsweise zwischen 10°C und 120°C.

Bei der Durchführung des Verfahrens (d) arbeitet man im Allgemeinen unter Atmosphärendruck. Es ist aber auch möglich, unter erhöhtem Druck zu arbeiten.

Bei der Durchführung des Verfahrens (d) setzt man Dihydroxy-triazolopyrimidin der Formel (V) im Allgemeinen mit einem Überschuss an Halogenierungsmittel um. Die Aufarbeitung erfolgt nach üblichen Methoden.

Die weiterhin zur Durchführung des erfindungsgemäßen Verfahrens (a) als Ausgangsstoffe benötigten Amine sind durch die Formel (III) allgemein definiert. In ieser Formel (III) haben R¹ und R² vorzugsweise diejenigen Bedeutungen, die bereits im Zusammenhang mit der Beschreibung der erfindungsgemäßen Verbindungen der Formel (I) für R¹ und R² als bevorzugt angegeben wurden.

Die Amine der Formel (III) sind teilweise bekannt.

Neu sind Amine der Formel 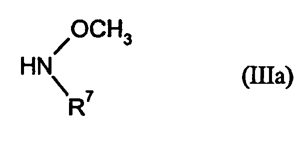 in welcher
- R⁷: für Isobutyl, 2-Methoxyethyl oder für 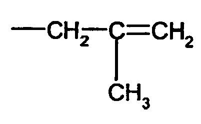
steht.

Die Amine der Formel (IIIa) lassen sich herstellen, indem man
f) in einer ersten Stufe N-Methoxycarbaminsäure-ethylester der Formel 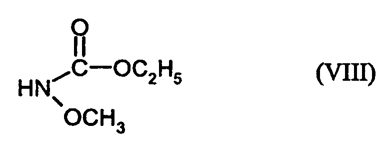 mit Halogenverbindungen der Formel

   R⁷-X¹ (IX)

   in welcher
   - R⁷: die oben angegebenen Bedeutungen hat und
   - X¹: für Brom oder Iod steht,
   in Gegenwart einer Base und in Gegenwart eines Verdünnungsmittels umsetzt und die entstehenden Carbamate der Formel 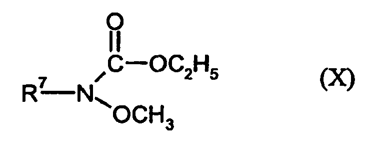 in welcher
   - R⁷: die oben angegebenen Bedeutungen hat,
   in einer zweiten Stufe mit Kaliumhydroxid in Gegenwart von Ethanol und Wasser umsetzt.

Neu sind auch Amine der Formel 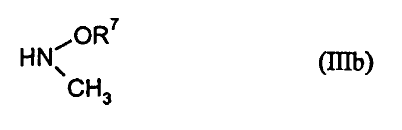 in welcher
- R⁷: die oben angegebenen Bedeutungen hat.

Die Amine der Formel (IIIb) lassen sich herstellen, indem man
g) in einer ersten Stufe N-Hydroxy-N-methyl-carbaminsäure-ethylester der Formel 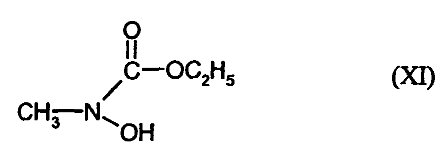 mit Halogenverbindungen der Formel

   R⁷-X¹ (IX)

   in welcher
   - R⁷ und X¹: die oben angegebenen Bedeutungen haben,
   in Gegenwart einer Base und in Gegenwart eines Verdünnungsmittels umsetzt und die entstehenden Carbamate der Formel 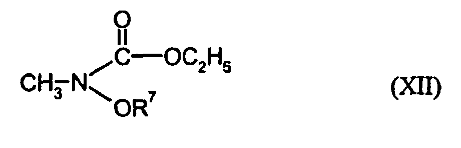 in welcher
   - R⁷: die oben angegebenen Bedeutungen hat,
   in einer zweiten Stufe mit Kaliumhydroxid in Gegenwart von Ethanol und Wasser umsetzt.

Neu sind auch Trifluorisopropylamine der Formel 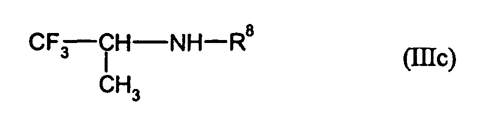 in welcher
- R⁸: für Methyl, Ethyl oder Propyl steht.

Die Trifluorisopropylamine der Formel (IIIc) lassen sich herstellen, indem
h) in einer ersten Stufe N-Trifluorisopropyl-carbaminsäure-ethylester der Formel 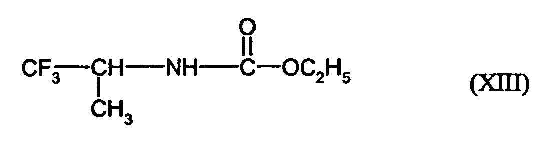 mit Halogenverbindungen der Fofrmel

   R⁸-X¹ (XIV)

   in welcher
   - R⁸ und X¹: die oben angegebenen Bedeutungen haben,
   in Gegenwart einer Base und in Gegenwart eines Verdünnungsmittels umsetzt und die entstehenden Carbamate der Formel 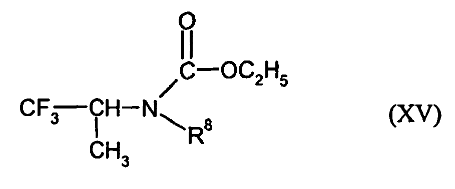 in welcher
   - R⁸: die oben angegebenen Bedeutungen hat,
   in einer zweiten Stufe mit Kaliumhydroxid in Gegenwart von Ethanol und Wasser umsetzt.

Neu ist schließlich auch das 3-Trifluormethyl-3-amino-propen der Formel

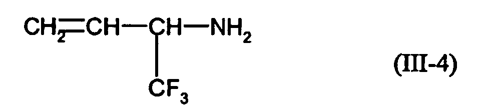

Das 3-Trifluormethyl-3-amino-propen der Formel (IIId) lässt sich herstellen, indem man
(i) das Carbamat der Formel 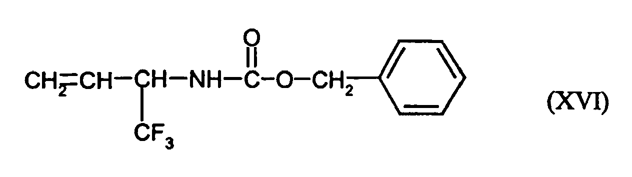 mit wässriger Salzsäure umsetzt.

Die bei der Durchführung der erfindungsgemäßen Verfahren (f) - (i) als Ausgangsstoffe benötigten Verbindungen der Formeln (VIII), (IX), (XI), (XIII), (XIV) und (XVI) sind bekannt oder lassen sich nach bekannten Methoden herstellen.

Bei der Durchführung der ersten Stufe der erfindungsgemäßen Verfahren (f), (g) und (h) kommen jeweils alle für derartige Umsetzungen üblichen anorganischen und organischen Säureakzeptoren in Frage.

Bei der Durchführung der ersten Stufe der erfindungsgemäßen Verfahren (f), (g) und (h) kommen jeweils alle für derartige Umsetzungen üblichen anorganischen und organischen Säureakzeptoren in Frage.

Vorzugsweise verwendbar sind Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat und Natriumhydrogencarbonat, und ausserdem Ammonium-Verbindungen, wie Ammoniumhydroxid, Ammoniumacetat und Ammoniumcarbonat.Als organische Basen seien genannt: tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel kommen bei der Durchführung der ersten Stufe der erfindungsgemäßen Verfahren (f), (g) und (h) jeweils alle üblichen inerten organischen Solventien in Betracht. Vorzugsweise verwendbar sind Ether, wie Diethylether, Dioxan, Tetrahydrofuran, 1,2-Dimethoxyethan, 1,2-Diethoxyethan oder Anisol; Amide, wie N,N-Dimethylformamid, N,N-Dimethylacetamid, N-Methylformanilid oder N-Methylpyrrolidon; Sulfone, wie Sulfolan; Alkohole wie Methanol, Ethanol, Isopropanol, tert.Butanol, n-Butanol.

Die Reaktionstemperaturen können bei der Durchführung der ersten Stufe der erfindungsgemäßen Verfahren (f), (g) und (h) jeweils innerhalb eines größeren Bereiches variiert werden. Im Allgemeinen arbeitet man bei Temperaturen zwischen 0°C und 150°C, vorzugsweise zwischen 10°C und 100°C.

Bei der Durchführung der ersten Stufe der erfindungsgemäßen Verfahren (f), (g) und (h) arbeitet man im Allgemeinen jeweils unter Atmosphärendruck. Es ist jedoch auch möglich, unter erhöhtem Druck oder, sofern keine niedrig siedenden Komponenten an der Umsetzung beteiligt sind, unter vermindertem Druck zu arbeiten.

Bei der Durchführung der ersten Stufe der erfindungsgemäßen Verfahren (f), (g) und (h) setzt man
- auf 1 mol an N-Methoxy-carbaminsäure-ethylester der Formel (VIII) im Allgemeinen 0,5 bis 15 mol, vorzugsweise 1 bis 5 mol an Halogenverbindung der Formel (IX) ein, bzw.
- auf 1 mol an N-Hydroxy-N-methyl-carbaminsäure-ethylester der Formel (XI) im Allgemeinen 0,5 bis 15 mol, vorzugsweise 1 bis 5 mol an Halogenverbindung der Formel (IX) ein, bzw.
- auf 1 mol an N-Trifluorisopropyl-carbaminsäure-ethylester der Formel (XIII) im Allgemeinen 0,5 bis 15 mol, vorzugsweise 1 bis 5 mol an Halogenverbindung der Formel (XIV) ein.

Die Aufarbeitung erfolgt jeweils nach üblichen Methoden, beispielsweise durch Extraktion und anschließende Trocknung oder durch Fällung mit anschließender Filtration und Trocknung. Gegebenenfalls noch vorhandene Verunreinigungen können nach üblichen Methoden entfernt werden.

Die bei der Durchführung der ersten Stufe der erfindungsgemäßen Verfahren (f), (g) und (h) als Zwischenprodukte erhaltenen Verbindungen der Formeln (X), (XII) und (XV) sind neu.

Auch bei der Durchführung der zweiten Stufe der erfindungsgemäßen Verfahren (f), (g) und (h) können die Reaktionstemperaturen jeweils innerhalb eines größeren Bereiches variiert werden. Im Allgemeinen arbeitet man bei Temperaturen zwischen 0°C und 100°C, vorzugsweise zwischen 10°C und 80°C.

Auch bei der Durchführung der zweiten Stufe der erfindungsgemäßen Verfahren (f), (g) und (h) arbeitet man im Allgemeinen jeweils unter Atmosphärendruck. Es ist jedoch wiederum möglich, jeweils auch unter erhöhtem Druck oder, sofern die zu isolierenden Produkte keine sehr niedrigen Siedepunkt aufweisen, unter vermindertem Druck zu arbeiten.

Bei der Durchführung der zweiten Stufe der erfindungsgemäßen Verfahren (f), (g) und (h) setzt man auf 1 mol an einer Verbindung der Formel (X), (XII) oder (XV) jeweils einen Überschuß, vorzugsweise bis zu 10 mol an Kaliumhydroxid ein. Die Aufarbeitung erfolgt nach üblichen Methoden. Dabei werden die Amine zweckmäßigerweise im Allgemeinen durch Hinzufügen von Säure, vorzugsweise wässriger Salzsäure, in Form ihrer Salze isoliert.

Bei der Durchführung des erfindungsgemäßen Verfahrens (i) können die Reaktionstemperaturen ebenfalls in einem größeren Bereich variiert werden. Im Allgemeinen arbeitet man bei Temperaturen zwischen 10°C und 150°C, vorzugsweise bei Rückflußtemperatur.

Im Allgemeinen arbeitet man bei der Durchführung des erfindungsgemäßen Verfahrens (i) unter Atmosphärendruck. Es ist aber auch möglich, unter erhöhtem Druck zu arbeiten.

Bei der Durchführung des erfindungsgemäßen Verfahrens (i) setzt man auf 1 mol an Carbamat der Formel (XVI) einem Überschuß, vorzugsweise bis zu 10 mol an wässriger Salzsäure ein. Die Aufarbeitung erfolgt wiederum nach üblichen Methoden.

Die bei der Durchführung des erfindungsgemäßen Verfahrens (b) als Ausgangsstoffe benötigten Triazolopyrimidine sind durch die Formel (Ia) allgemein definiert. In dieser Formel haben R², R³, R⁴ und X vorzugsweise diejenigen Bedeutungen, die bereits im Zusammenhang mit der Beschreibung der erfindungsgemäßen Stoffe der Formel (I) für diese Reste als bevorzugt genannt wurden.

Bei den Triazolopyrimidinen der Formel (Ia) handelt es sich um erfindungsgemäße Stoffe. Sie lassen sich nach dem erfindungsgemäßen Verfahren (a) herstellen.

Die weiterhin zur Durchführung des erfindungsgemäßen Verfahrens (b) als Ausgangsstoffe benötigten Sulfensäurehalogenide sind durch die Formel (IV) allgemein definiert. In dieser Formel hat R⁵ vorzugsweise diejenigen Bedeutungen, die bereits im Zusammenhang mit der Beschreibung der erfindungsgemäßen Verbindungen der Formel (I) für diesen Rest als bevorzugt genannt wurden.
- Y²: steht vorzugsweise für Fluor, Chlor oder Brom, besonders bevorzugt für Chlor.

Die Sulfensäurehalogenide der Formel (IV) sind bekannt oder lassen sich nach bekannten Methoden herstellen.

Die bei der Durchführung des erfindungsgemäßen Verfahrens (c) als Ausgangsstoffe benötigten Triazolopyrimidine sind durch die Formel (Ib) allgemein definiert. In dieser Formel haben R¹, R², R³, R⁴ und X vorzugsweise diejenigen Bedeutungen, die bereits im Zusammenhang mit der Beschreibung der erfindungsgemäßen Stoffe der Formel (I) für diese Reste als bevorzugt genannt wurden.

Bei den Triazolopyrimidinen der Formel (Ib) handelt es sich um erfindungsgemäße Stoffe. Sie lassen sich nach den erfindungsgemäßen Verfahren (a) oder (b) herstellen.

Als Oxidationsmittel kommen bei der Durchführung des erfindungsgemäßen Verfahrens (c) alle üblichen, zur Abgabe von Sauerstoff geeigneten Substanzen in Betracht. Vorzugsweise verwendbar sind Wasserstoffperoxid, dessen Salze und dessen Addukte mit z.B. Harnstoff, außerdem t-Butylperoxid, Persäuren oder deren Salze, wie Perameisensäure, Peressigsäure, Perpropionsäure, Perbenzoesäure, 3-Chlorperbenzoesäure, Kaliumhydrogenpersulfat, Kaliumperoxodisulfat, Natriumperborat, Natriumpercarbonat oder Natriumperoxodisulfat, weiterhin auch Kaliumpermanganat, oder Natriumperrhenat, ferner auch chlorige- oder hypochlorige Säure oder deren Metallsalzlösungen in Wasser.

Als Verdünnungsmittel kommen bei der Durchführung des erfindungsgemäßen Verfahrens (a) alle üblichen, inerten organischen Solventien in Betracht. Vorzugsweise verwendbar sind aliphatische, alicyclische oder aromatische Kohlenwasserstoffe, wie Hexan, Heptan, Cyclohexan oder Toluol; halogenierte Kohlenwasserstoffe, wie Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Dioxan, Tetrahydrofuran, 1,2- Dimethoxyethan oder 1,2-Diethoxyethan; Amide, wie N,N-Dimethylformamid, N,N-Dimethylacetamid oder N-Methylpyrrolidon; Ester wie Essigsäuremethylester oder Essigsäureethylester, Sulfoxide, wie Dimethylsulfoxid; Sulfone, wie Sulfolan.

Als Säureakzeptoren kommen bei der Durchführung des erfindungsgemäßen Verfahrens (a) alle für derartige Umsetzungen üblichen Säurebindemittel in Frage. Vorzugsweise verwendbar sind tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im Allgemeinen arbeitet man bei Temperaturen zwischen 0°C und 150°C, vorzugsweise bei Temperaturen zwischen 0°C und 80°C.

Sowohl bei der Durchführung des erfindungsgemäßen Verfahrens (a) als auch des Verfahrens (b) arbeitet man im allgemeinen unter Atmosphärendruck. Es ist aber auch möglich, unter erhöhtem oder vermindertem Druck, im allgemeinen zwischen 0,1 und 10 bar, zu arbeiten.

Bei der Durchführung des erfindungsgemäßen Verfahrens (a) setzt man auf 1 mol an Dihalogen-triazolo-pyrimidin der Formel (II) im allgemeinen 0,5 bis 10 mol, vorzugsweise 0,8 bis 2 mol an Amin der Formel (III) ein. Die Aufarbeitung erfolgt nach üblichen Methoden.

Als Verdünnungsmittel kommen bei der Durchführung des erfindungsgemäßen Verfahrens (b) alle üblichen, inerten organischen Solventien in Betracht. Vorzugsweise verwendbar sind Ether, wie Diethylether, Dioxan, Tetrahydrofuran, 1,2-Dimethoxyethan, 1,2-Diethoxyethan oder Anisol; Amide, wie N,N-Dimethylformamid, N,N-Dimethylacetamid, N-Methylformanilid oder N-Methylpyrrolidon; Sulfone, wie Sulfolan.

Als Säureakzeptoren kommen bei der Durchführung des erfindungsgemäßen Verfahrens (b) alle für derartige Umsetzungen üblichen anorganischen und organischen Basen infrage. Vorzugsweise verwendbar sind Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat und Natriumhydrogencarbonat, und außerdem Ammonium-Verbindungen, wie Ammoniumhydroxid, Ammoniumacetat und Ammoniumcarbonat.

Auch bei der-Durchführung des erfindungsgemäßen Verfahrens (b) können die Reaktionstemperaturen innerhalb eines größeren Bereiches variiert werden. Im allgemeinen arbeitet man bei Temperaturen zwischen -40°C und +120°C, vorzugsweise zwischen -20°C und +50°C.

Bei der Durchführung des erfindungsgemäßen Verfahrens (b) setzt man auf 1 mol an Triazolopyrimidin der Formel (Ia) im Allgemeinen 1 bis 15 mol, vorzugsweise 1 bis 8 mol an Sulfensäurehalogenid der Formel (IV) ein. Die Aufarbeitung erfolgt nach üblichen Methoden.

Als Verdünnungsmittel kommen bei der Durchführung des erfindungsgemäßen Verfahrens (c) alle für derartige Umsetzungen üblichen, inerten organischen Solventien infrage. Vorzugsweise verwendbar sind alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Säuren, wie Ameisensäure, Essigsäure oder Propionsäure; Nitrile, wie Acetonitril, Propionitril, n- oder i-Butyronitril oder Benzonitril; Amide, wie N, N-Dimethylformamid, N, N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Als Katalysatoren kommen bei der Durchführung des erfindungsgemäßen Verfahrens (c) alle für derartige Umsetzungen üblichen Reaktionsbeschleuniger infrage. Vorzugsweise verwendbar sind Salze von Metallen der IV., V. und VI. Nebengruppe des Periodensystems der Elemente. Als Beispiele hierfür seien Natrium(meta)vanadat, Natriummolybdat und Natriumwolframat genannt.

Das erfindunsgemäße Verfahren (c) kann in einer besonderen Variante auch in einem Zweiphasen wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-C₁₃/C₁₅-alkylammoniumchlorid, Trimethyl-C₁₃/-C₁₅-alkylammoniumbromid, Dibenzyl-dimethyl-ammoniummethylsulfat, Dimethyl-C₁₂/C₁₄-alkyl-benzylammoniumchlorid, Dimethyl-C₁₂/C₁₄-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Die Reaktionstemperaturen können auch bei der Durchführung des erfindungsgemäßen Verfahrens (c) in einem größeren Bereich variiert werden. Im allgemeinen arbeitet man bei Temperaturen zwischen 0°C und 150°C, vorzugsweise zwischen 0°C und 80°C.

Auch bei der Durchführung des erfindungsgemäßen Verfahrens (c) arbeitet man im Allgemeinen unter Atmosphärendruck. Es ist aber auch möglich, unter erhöhtem Druck zu arbeiten.

Ist bei der Durchführung des erfindungsgemäßen Verfahrens (c) die Herstellung von Triazolopyrimidinen der Formel (I) beabsichtigt, in denen n für 1 steht, so setzt man auf 1 mol an Triazolopyridin der Formel (Ib) im Allgemeinen 1 bis 1,5 Äquivalente, vorzugsweise 1 bis 1,2 Äquivalente, an Oxidationsmittel ein.

Ist bei der Durchführung des erfindungsgemäßen Verfahrens (c) die Herstellung von Triazolopyrimidinen der Formel (I) beabsichtigt, in denen n für 2 steht, so setzt man auf 1 mol an Triazolopyridin der Formel (Ib) im Allgemeinen 2 bis 10 Äquivalente, vorzugsweise 2 bis 5 Äquivalente, an Oxidationsmittel ein. Die Aufarbeitung erfolgt jeweils nach üblichen Methoden.

Zur Herstellung von Säureadditions-Salzen von Triazolopyrimidinen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebendenfalls durch Waschen mit einem inerten organischen Lösungsmittel gereinigt werden.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus; Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine sehr gute stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokolation mit unerwünschten Mikroorgansimen weitgehende Resistenz gegen diese Mirkroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Krankheiten im Wein-, Obst- und Gemüseanbau, wie beispielsweise gegen Venturia- oder Podosphaera-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Mit den erfindungsgemäßen Wirkstoffen können Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die Behandlung der Pflanzen und Pflanzenteile mit den erfindungsgemäßen Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiennittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen infrage:
Fungizide:
   Aldimorph, Ampropylfos, Ampropylfos-Kalium, Andoprim, Anilazin, Azaconazol, Azoxystrobin,
   Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
   Calciumpolysulfid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram, Carpropamid,
   Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
   Ediphenphos, Epoxiconazol, Etaconazol, Ethirimol, Etridiazol,
   Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox, Fenhexamid,
   Guazatin,
   Hexachlorobenzol, Hexaconazol, Hymexazol,
   Imazalil, Imibenconazol, Iminoctadin, Iminoctadinealbesilat, Iminoctadinetriacetat, Iodocarb, Ipconazol, Iprobenfos (IBP), Iprodione, Irumamycin, Isoprothiolan, Isovaledione, Iprovalicarb,
   Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrothal-isopropyl, Nuarimol,
   Ofurace, Oxadixyl, Oxamocarb, Oxolinicacid, Oxycarboxim, Oxyfenthiin,
   Paclobutrazol, Pefurazoat, Penconazol, Pencycuron, Phosdiphen, Picoxystrobin, Pimaricin, Piperalin, Polyoxin, Polyoxorim, Probenazol, Prochloraz, Procymidon, Propamocarb, Propanosine-Natrium, Propiconazol, Propineb, Pyraclostrobin, Pyrazophos, Pyrifenox, Pyrimethanil, Pyroquilon, Pyroxyfur,
   Quinconazol, Quintozen (PCNB), Quinoxyfen,
   Schwefel und Schwefel-Zubereitungen, Spiroxamine,
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Trifloxystrobin, Triflumizol, Triforin, Triticonazol,
   Uniconazol,
   Validamycin A, Vinclozolin, Viniconazol,
   Zarilamid, Zineb, Ziram sowie
   Dagger G,
   OK-8705,
   OK-8801,
   α-(1,1-Dimethylethyl)-β-(2-phenoxyethyl)-1H-1,2,4-triazol-1-ethanol,
   α-(2,4-Dichlorphenyl)-β-fluor-b-propyl-1H-1,2,4-triazol-1-ethanol,
   α-(2,4-Dichlorphenyl)-β-methoxy-a-methyl-1H-1,2,4-triazol-1-ethanol,
   α-(5-Methyl-1,3-dioxan-5-yl)-β-[[4-(trifluormethyl)-phenyl]-methylen]-1H-1,2,4-triazol-1-ethanol,
   (5RS,6RS)-6-Hydroxy-2,2,7,7-tetramethyl-5-(1H,2,4-triazol-1-yl)-3-octanon,
   (E)-a-(Methoxyimino)-N-methyl-2-phenoxy-phenylacetamid,
   1-(2,4-Dichlorphenyl)-2-(1H-1,2,4-triazol-1-yl)-ethanon-O-(phenylmethyl)-oxim,
   1-(2-Methyl-1-naphthalenyl)-1H-pyrrol-2,5-dion,
   1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlorphenyl)-1,3-dioxolan-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)-methoxy]-phenyl]-ethenyl]-1H-imidazol,
   1-Methyl-5-nonyl-2-(phenylmethyl)-3-pyrrolidinol,
   2';6'-Dibrom-2-methyl-4'-trifluormethoxy-4'-trifluor-methyl-1,3-thiazol-5-carboxanilid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlormethyl)-1,3,4-thiadiazol,
   2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
   2-Aminobutan,
   2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid,
   2-Phenylphenol(OPP),
   3,4-Dichlor-1-[4-(difluomethoxy)-phenyl]-1H-pyrrol-2,5-dion,
   3,5-Dichlor-N-[cyan[(1-methyl-2-propynyl)-oxy]-methyl]-benzamid,
   3-(1,1-Dimethylpropyl-1-oxo-1H-inden-2-carbonitril,
   3-[2-(4-Chlorphenyl)-5-ethoxy-3-isoxazolidinyl]-pyridin,
   4-Chlor-2-cyan-N,N-dimethyl-5-(4-methylpheny)-1H-imidazol-1-sulfonamid,
   4-Methyl-tetrazolo[1,5-a]quinazolin-5(4H)-on,
   8-Hydroxychinolinsulfat,
   9H-Xanthen-9-carbonsäure-2-[(phenylamino)-carbonyl]-hydrazid,
   bis-(1-Methylethyl)-3-methyl-4-[(3-methylbenzoyl)-oxy]-2,5-thiophendicarboxylat,
   cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholinhydrochlorid,
   Ethyl-[(4-chlorphenyl)-azo]-cyanoacetat,
   Kaliumhydrogencarbonat,
   Methantetrathiol-Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,6-Dimethylphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2-Trichlor-1-[(chloracetyl)-amino]-ethyl]-benzamid,
   N-[3-Chlor-4,5-bis-(2-propinyloxy)-phenyl]-N'-methoxy-methanimidamid,
   N-Formyl-N-hydroxy-DL-alanin -Natriumsalz,
   O,O-Diethyl-[2-(dipropylamino)-2-oxoethyl]-ethylphosphoramidothioat,
   O-Methyl-S-phenyl-phenylpropylphosphoramidothioat,
   S-Methyl-1,2,3-benzothiadiazol-7-carbothioat,
   spiro[2H]-1-Benzopyran-2,1'(3'H)-isobenzofuran]-3'-on,
   4-[3,4-Dimethoxyphenyl)-3-(4-fluorphenyl)-acryloyl]-morpholin
**Bakterizide:**
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
   Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, Bistrifluron, BPMC, Bromophos A, Bufencarb, Buprofezin, Butathiofos, Butocarboxim, Butylpyridaben,
   Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Chromafenozide, Cis-Resmethrin, Cispermethrin, Clocythrin, Cloethocarb, Clofentezine, Clothianidine, Cyanophos, Cycloprene, Cycloprothrin, Cyfluthrin, Cyhalothrin, Cyhexatin, Cypermethrin, Cyromazine,
   Deltamethrin, Demeton M, Demeton S, Demeton-S-methyl, Diafenthiuron, Diazinon, Dichlorvos, Dicofol, Diflubenzuron, Dimethoat, Dimethylvinphos, Diofenolan, Disulfoton, Docusat-sodium, Dofenapyn,
   Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
   Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazuron, Flubrocythrinate, Flucycloxuron, Flucythrinate, Flufenoxuron, Flumethrin, Flutenzine, Fluvalinate, Fonophos, Fosmethilan, Fosthiazate, Fubfenprox, Furathiocarb,
   Granuloseviren
   Halofenozide, HCH, Heptenophos, Hexaflumuron, Hexythiazox, Hydroprene,
   Imidacloprid, Indoxacarb, Isazofos, Isofenphos, Isoxathion, Ivermectin,
   Kempolyederviren
   Lambda-cyhalothrin, Lufenuron
   Malathion, Mecarbam, Metaldehyd, Methamidophos, Metharhizium anisopliae, Metharhizium flavoviride, Methidathion, Methiocarb, Methoprene, Methomyl, Methoxyfenozide, Metolcarb, Metoxadiazone, Mevinphos, Milbemectin, Milbemycin, Monocrotophos,
   Naled, Nitenpyram, Nithiazine, Novaluron
   Omethoat, Oxamyl, Oxydemethon M
   Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen,
   Quinalphos,
   Ribavirin
   Salithion, Sebufos, Silafluofen, Spinosad, Spirodiclofen, Sulfotep, Sulprofos,
   Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon Thetacypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
   Vamidothion, Vaniliprole, Verticillium lecanii
   YI 5302
   Zeta-cypermethrin, Zolaprofos
   (1R-cis)-[5-(Phenylmethyl)-3-furanyl]-methyl-3-[(dihydro-2-oxo-3(2H)-furanyliden)-methyl]-2,2-dimethylcyclopropancarboxylat
   (3-Phenoxyphenyl)-methyl-2,2,3,3-tetramethylcyclopropanecarboxylat
   1-[(2-Chlor-5-thiazolyl)methyl]tetrahydro-3,5-dimethyl-N-nitro-1,3,5-triazin-2(1H)-imin
   2-(2-Chlor-6-fluorphenyl)-4-[4-(1,1-dimethylethyl)phenyl]-4,5-dihydro-oxazol
   2-(Acetlyoxy)-3-dodecyl-1,4-naphthalindion
   2-Chlor-N-[[[4-(1-phenylethoxy)-phenyl]-amino]-carbonyl]-benzamid
   2-Chlor-N-[[[4-(2,2-dichlor-1,1-difluorethoxy)-phenyl]-amino]-carbonyl]-benzamid
   3-Methylphenyl-propylcarbamat
   4-[4-(4-Ethoxyphenyl)-4-methylpentyl]-1-fluor-2-phenoxy-benzol
   4-Chlor-2-(1,1-dimethylethyl)-5-[[2-(2,6-dimethyl-4-phenoxyphenoxy)ethyl]thio]-3(2H)-pyridazinon
   4-Chlor-2-(2-chlor-2-methylpropyl)-5-[(6-iod-3-pyridinyl)methoxy]-3(2H)-pyridazinon
   4-Chlor-5-[(6-chlor-3-pyridinyl)methoxy]-2-(3,4-dichlorphenyl)-3(2H)-pyridazinon
   Bacillus thuringiensis strain EG-2348
   Benzoesäure [2-benzoyl-1-(1,1-dimethylethyl)-hydrazid
   Butansäure 2,2-dimethyl-3-(2,4-dichlorphenyl)-2-oxo-1-oxaspiro[4.5]dec-3-en-4-yl-ester
   [3-[(6-Chlor-3-pyridinyl)methyl]-2-thiazolidinyliden]-cyanamid
   Dihydro-2-(nitromethylen)-2H-1,3-thiazine-3(4H)-carboxaldehyd
   Ethyl-[2-[[1,6-dihydro-6-oxo-1-(phenylmethyl)-4-pyridazinyl]oxy]ethyl]-carbamat
   N-(3,4,4-Trifluor-1-oxo-3-butenyl)-glycin
   N-(4-Chlorphenyl)-3-[4-(difluormethoxy)phenyl]-4,5-dihydro-4-phenyl-1H-pyrazol-1-carboxamid
   N-[(2-Chlor-5-thiazolyl)methyl]-N'-methyl-N"-nitro-guanidin
   N-Methyl-N'-(1-methyl-2-propenyl)-1,2-hydrazindicarbothioamid
   N-Methyl-N'-2-propenyl-1,2-hydrazindicarbothioamid
   O,O-Diethyl-[2-(dipropylamino)-2-oxoethyl]-ethylphosphoramidothioat
   N-Cyanomethyl-4-trifluormethyl-nicotinamid
   3,5-Dichlor-1-(3,3-dichlor-2-propenyloxy)-4-[3-(5-trifluormethylpyridin-2-yloxy)-propoxy]-benzol

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sproßpilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10 000 g/ha, vorzugsweise zwischen 10 und 1 000 g/ha. Bei der Saatgutbehandlung liegen die Aufwandmengen an Wirkstoff im Allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10 000 g/ha, vorzugsweise zwischen 1 und 5 000 g/ha.

Wie bereits oben erwähnt, können mit erfindungsgemäßen Wirkstoffen Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetic Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Emteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft mit den erfindungsgemäßen Verbindungen der allgemeinen Formel (I) behandelt werden. Die bei den Wirkstoffen oben angegebenen Vorzugsbereiche gelten auch für die Behandlung dieser Pflanzen.

Besonders hervorgehoben sei die Pflanzenbehandlung mit den im vorliegenden Text speziell aufgeführten Verbindungen.

Die Erfindung wird durch die folgenden Beispiele veranschaulicht.

### Herstellungsbeispiele

### Beispiel 1

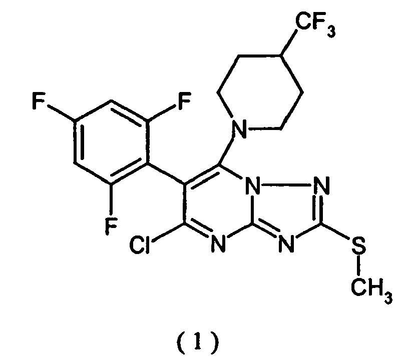

### Verfahren (a)

In eine Lösung von 1 g (2,378 mMol) 5,7-Dichlor-2-(methylsulfanyl)-6-(2,4,6-trifluorphenyl)[1,2,4]triazolo[1,5-a]pyrimidin und 0,419 g (2,378 mMol) 4-Trifluormethylpiperidin in 20 ml Dichlormethan werden 0,277 g Triethylamin gegeben. Das Gemisch wird 18 Stunden bei Raumtemperatur gerührt. Danach wird das Reaktionsgemisch mit soviel 1N Salzsäure versetzt und gerührt, bis der pH-Wert der Mischung bei 1-2 liegt (ca. 50 ml). Die organische Phase wird abgetrennt, über Natriumsulfat getrocknet und unter vermindertem Druck eingeengt. Der Rückstand wird mit Diisopropylether verrührt und abgesaugt. Man erhält 1,1 g (83,4 % der Theorie) 5-Chlor-2-(methylsulfanyl)-7-[4-(trifluormethyl)-1-piperidinyl]-6-(2,4,6-trifluorphenyl)-[1,2,4]triazolo[1,5-a]pyrimidin.
HPLC: logP = 4,10

### Beispiel 2

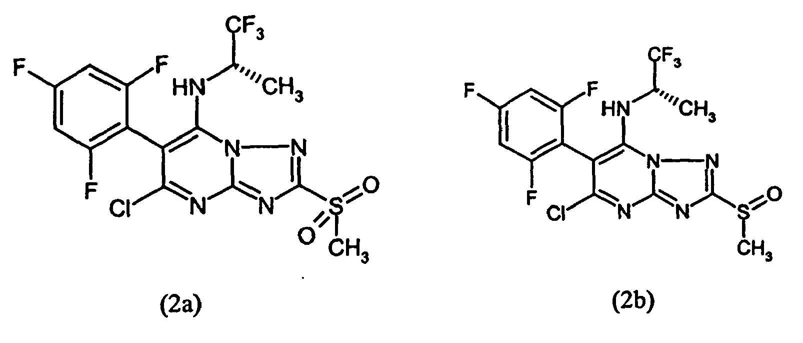

### Verfahren (c)

Zu einer Lösung von 1 g (2,264 mMol) 5-Chlor-2-(methylsulfanyl)-N-[(1S)-2,2,2-trifluor-1-methylethyl]-6-(2,4,6-trifluorphenyl)[1,2,4]triazolo[1,5-a]pyrimidin-7-amin in 20 ml Dichlormethan gibt man 0,1 g Ammoniummolybdat und 0,17 ml 98 bis 100 %ige Ameisensäure. Hierzu tropft man langsam 0,59 ml (6,8 mMol) 35 %iges Wasserstoffperoxid und rührt 16 Stunden. Die Reaktionsmischung wird dann mit 50 ml Wasser versetzt. Danach wird die organische Phase abgetrennt, mit 50 ml 10 %iger wässriger Natriumhydrogensulfitlösung gewaschen, über Natriumsulfat getrocknet und unter vermindertem Druck eingeengt. Der Rückstand wird mit Diisopropylether verrührt und abgesaugt. Das Rohprodukt wird zunächst mit n-Hexan/Essigsäureethylester (1:1) und zuletzt mit reinem Essigsäureethylester an Kieselgel chromatografiert. Man erhält 260 mg (24 % der Theorie) 5-Chlor-2-(methylsulfonyl)-N-[(1S)-2,2,2-trifluor-1-methylethyl]-6-(2,4,6-trifluorphenyl)-[1,2,4]triazolo[1,5-a]pyrimidin-7-amin (2a) und 360 mg (34% der Theorie) 5-Chlor-2-(methylsulfinyl)-N-[(1S)-2,2,2-trifluor-1-methylethyl]-6-(2,4,6-trifluorphenyl)-[1,2,4]triazolo[1,5-a]pyrimidin-7-amin (2b).
2a: HPLC: logP = 2,94
2b: HPLC: logP = 2,50

Nach den zuvor angegebenen Methoden werden auch die in der nachstehenden Tabelle 1 aufgeführten Verbindungen der Formel (I) erhalten.

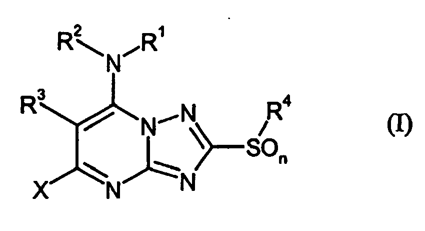

**Tabelle 1**

| **Bsp. Nr.** | **R**^{**1**} **(***)** | **R**^{**2**} | **R**^{**3**} | **R**^{**4**} | **n** | **X** | **logP (**)** | **Fp.: (°C)** |
|---|---|---|---|---|---|---|---|---|
| 3 | Cyclopentyl | -H | 2-Chlorphenyl | -CH₃ | 0 | Cl | | 119-122 |
| 4 | Cyclopentyl | -H | 2,6-Dichlorphenyl | -CH₃ | 0 | Cl | 4,17 | |
| 5 | -CH₂-CH₂-CH(CH₃)-CH₂-CH₂- | * | 2,6-Dichlorphenyl | -CH₃ | 0 | Cl | 4,9 | |
| 6 | Cyclopentyl | -H | 2,6-Dichlorphenyl | -CH₃ | 2 | Cl | 3,35 | |
| 7 | -CH₂-CH₂-CH(CH₃)-CH₂-CH₂- | * | 2,6-Dichlorphenyl | -CH₃ | 2 | Cl | 3,73 | |
| 8 | 2,2,2-Trifluor-1-methylethyl | -H | 2,6-Dichlorphenyl | -CH₃ | 0 | Cl | 3,82 | |
| 9 | 2,2,2-Trifluor-1-methylethyl | -H | 2,4,6-Trifluorphenyl | -CH₃ | 0 | Cl | 3,58 | |
| 10 | -CH₂-CH₂-CF₃ | -H | 2,4,6-Trifluorphenyl | -CH₃ | 0 | Cl | 3,36 | |
| 11 | -C₂H₅ | -C₂H₅ | 2,4,6-Trifluorphenyl | -CH₃ | 0 | Cl | 3,89 | |
| 12 | -CH₂-CF₃ | -H | 2,4,6-Trifluorphenyl | -CH₃ | 0 | Cl | 3,23 | |
| 13 | N-Morpholinyl | -H | 2,4,6-Trifluorphenyl | -CH₃ | 0 | Cl | 2,72 | 202-03 |
| 14 | -NH-CH₂-CF₃ | -H | 2,4,6-Trifluorphenyl | -CH₃ | 0 | Cl | 3,07 | 157-59° |
| 15 | -CH₂-CH₂-O-CH₂-CH₂- | * | 2,4,6-Trifluorphenyl | -CH₃ | 0 | Cl | 2,99 | 208-09 |
| 16 | 1-Piperidinyl | -H | 2,4,6-Trifluorphenyl | -CH₃ | 0 | Cl | 3,81 | 209-11 |
| 17 | -CH₂-CH₂-CH₂-CH₂-CH₂- | * | 2,4,6-Trifluorphenyl | -CH₃ | 0 | Cl | 4,09 | |
| 18 | -NH-CH₂-CF₃ | -H | 2-Chlorphenyl | -CH₃ | 0 | Cl | 3,1 | |
| 19 | Ethylamino | -H | 2,4,6-Trifluorphenyl | -CH₃ | 0 | Cl | 2,93 | 215-18 |
| 20 | 1-Cyclopropylethylamino | -H | 2,4,6-Trifluorphenyl | -CH₃ | 0 | Cl | 3,74 | |
| 21 | -CH₂-C(CH₃)=CH₂ | -C₂H₅ | 2,4,6-Trifluorphenyl | -CH₃ | 0 | Cl | 4,43 | |
| 22 | -i-Propyl | -H | 2,4,6-Trifluorphenyl | -CH₃ | 0 | Cl | 3,36 | |
| 23 | -NH-CH₃ | -CH₃ | 2,4,6-Trifluorphenyl | -CH₃ | 0 | Cl | 3,08 | 213-14 |
| 24 | -i-Propyl | -H | 2-Chlorphenyl | -CH₃ | 0 | Cl | 3,43 | |
| 25 | n-Propyl | -H | 2-Chlorphenyl | -CH₃ | 0 | Cl | 3,4 | |
| 26 | -CH₂-CH₂-CH₂-CH₂- | * | 2-Chlorphenyl | -CH₃ | 0 | Cl | 3,53 | |
| 27 | -CH₂-CH₂-CH₂-CH₂-CH₂- | * | 2-Chlorphenyl | -CH₃ | 0 | Cl | 4,21 | |
| 28 | -CH₂-CH₂-O-CH₂-CH₂- | * | 2-Chlorphenyl | -CH₃ | 0 | Cl | 2,98 | |
| 29 | t-Butylamino | -H | 2,4,6-Trifluorphenyl | -CH₃ | 0 | Cl | 3,66 | 216-17 |
| 30 | n-Butylamino | -H | 2,4,6- Trifluorphenyl | -CH₃ | 0 | Cl | 3,63 | 227-28 |
| 31 | -NH-CH₂-CH(CH₃)₂ | -H | 2,4,6-Trifluorphenyl | -CH₃ | 0 | Cl | 3,66 | 226-28 |
| 32- | 2-Hydroxyethylamino | -H | 2,4,6-Trifluorphenyl | -CH₃ | 0 | Cl | 2,14 | |
| 33 | -i-Propyl | -H | 2-Chlor-6-Fluorphenyl | -CH₃ | 0 | Cl | 3,42 | |
| 34 | -CH₂-CH(CH₃)-O-CH(CH₃)-CH₂- | * | 2-Chlorphenyl | -CH₃ | 0 | Cl | 3,79 | |
| 35 | 2,2,2-Trifluor-1-methylethyl | -H | 2-Chlorphenyl | -CH₃ | 0 | Cl | 3,63 | |
| 36 | -N=CH-N=CH- | * | 2-Chlorphenyl | -CH₃ | 0 | Cl | 2,73 | |
| 37 | 2,2,2-Trifluor-1-methylethyl | -H | 2-Chlorphenyl | -CH₃ | 0 | Cl | 3,64 | |
| 38 | 2,2,2-Trifluor-1-methylethyl | -H | 2-Chlor-6-Fluorphenyl | -CH₃ | 0 | Cl | 3,54 | |
| 39 | -CH₂-C(CH₃)=CH₂ | -H | 2,4,6-Trifluorphenyl | -CH₃ | 0 | Cl | 3,34 | |
| 40 | -CH₂-CN | -H | 2,4,6-Trifluorphenyl | -CH₃ | 0 | Cl | 2,48 | |
| 41 | -i-Propyl | -H | 2,6-Dichlor-3-fluor-5-trifluormethylphenyl | -CH₃ | 0 | Cl | 4,26 | 173-75 |
| 42 | Cyclohexylmethylamino | -H | 2,4,6-Trifluorphenyl | -CH₃ | 0 | Cl | 4,41 | 178-81 |
| 43 | 2,2,2-Trifluor-1-methylethyl | -H | 2,6-Dichlor-3-fluor-5-trifluormethylphenyl | -CH₃ | 0 | Cl | 4,46 | 168-70 |
| 44 | i-Propylamino | -H | 2,6-Dichlor-3-fluor-5-trifluormethylphenyl | -CH₃ | 0 | Cl | 4,24 | 175-80 |
| 45 | -CH₂-CN | -H | Pentafluorphenyl | -CH₃ | 0 | Cl | 2,89 | |
| 46 | -CH₂-CH₂-CH₂-CH₂-CH₂- | * | Pentafluorphenyl | -CH₃ | 0 | Cl | 4,43 | |
| 47 | n-Propyl | -H | Pentafluorphenyl | -CH₃ | 0 | Cl | 3,78 | |
| 48 | Cyclopentyl | -H | Pentafluorphenyl | -CH₃ | 0 | Cl | 4,28 | |
| 49 | -C₂H₅ | -C₂H₅ | Pentafluorphenyl | -CH₃ | 0 | Cl | 4,25 | |
| 50 | Cyclopropyl | -H | Pentafluorphenyl | -CH₃ | 0 | Cl | 3,52 | |
| 51 | Cyclopropylmethyl | -H | Pentafluorphenyl | -CH₃ | 0 | Cl | 3,86 | |
| 52 | -i-Propyl | -H | Pentafluorphenyl | -CH₃ | 0 | Cl | 3,81 | |
| 53 | -CH₂-CF₃ | -H | Pentafluorphenyl | -CH₃ | 0 | Cl | 3,62 | |
| 54 | -CH₂-CH₂-CH(CF₃)-CH₂-CH₂- | * | Pentafluorphenyl | -CH₃ | 0 | Cl | 4,42 | |
| 55 | 2,2,2-Trifluor-1-methylethyl | -H | Pentafluorphenyl | -CH₃ | 0 | Cl | 3,97 | |
| 56 | N-Morpholinyl | -H | Pentafluorphenyl | -CH₃ | 0 | Cl | 3,22 | |
| 57 | Dimethylamino | -H | Pentafluorphenyl | -CH₃ | 0 | Cl | 3,57 | |
| 58 | N-Morpholinyl | -H | 2,4,6-Trifluorphenyl | -CH₃ | 1 | Cl | 1,99 | |
| 59 | N-Morpholinyl | -H | 2,4,6-Trifluorphenyl | -CH₃ | 2 | Cl | 2,37 | 140-42 Zer. |
| 60 | -O-CH₃ | -CH₃ | 2-Chlorphenyl | -CH₃ | 0 | Cl | 3,37 | |
| 61 | -N=CH₂ | -CH₃ | 2,4,6-Trifluorphenyl | -CH₃ | 2 | Cl | 2,61 | 185-88 |
| 62 | 2-Methoxyethyl | -C₂H₅ | 2,4,6-Trifluorphenyl | -CH₃ | 0 | Cl | 3,61 | |
| 63 | -N=CH₂ | -CH₃ | 2,4,6-Trifluorphenyl | -CH₃ | 1 | Cl | 2,14 | |
| 64 | -CH₂-CH₂-N(CH₃)-CH₂-CH₂- | * | 2,4,6-Trifluorphenyl | -CH₃ | 0 | Cl | 1,47 | |
| 65 | -CH₂-CH₂-CH₂-CH₂- | * | 2,4,6-Trifluorphenyl | -CH₃ | 0 | Cl | 3,45 | |
| 66 | n-Propyl | -H | 2,4,6-Trifluorphenyl | -CH₃ | 0 | Cl | 3,34 | |
| 67 | Cyclopentyl | -H | 2,4,6-Trifluorphenyl | -CH₃ | 0 | Cl | 3,87 | |
| 68 | 2-Methoxyethyl | -H | 2,4,6-Trifluorphenyl | -CH₃ | 0 | Cl | 2,88 | |
| 69 | Cyclopropyl | -H | 2,4,6-Trifluorphenyl | -CH₃ | 0 | Cl | 3,11 | |
| 70 | -CH₂-CH₂-S-CH₂-CH₂- | * | 2,4,6-Trifluorphenyl | -CH₃ | 0 | Cl | 3,66 | |
| 71 | Cyclopropylmethyl | -H | 2,4,6-Trifluorphenyl | -CH₃ | 0 | Cl | 3,44 | |
| 72 | -CH₃ | -CH₃ | 2-Chlorphenyl | -CH₃ | 0 | Cl | | |
| 73 | -C₂H₅ | -H | 2-Chlorphenyl | -CH₃ | 0 | Cl | | |
| 74 | -C₂H₅ | -C₂H₅ | 2-Chlorphenyl | -CH₃ | 0 | Cl | | |
| 75 | 2-Methoxyethyl | -H | 2-Chlorphenyl | -CH₃ | 0 | Cl | | |
| 76 | -CH₃ | -H | 2-Chlorphenyl | -CH₃ | 0 | Cl | | |
| 77 | Cyclopropylmethyl | -H | 2-Chlorphenyl | -CH₃ | 0 | Cl | | |
| 78 | -CH₂-CH₂-S-CH₂-CH₂- | * | 2-Chlorphenyl | -CH₃ | 0 | Cl | | |
| 79 | -CH₂-CH₂-CH(CF₃)-CH₂-CH₂- | * | 2-Chlorphenyl | -CH₃ | 0 | Cl | | |
| 80 | Pyrrolidin-1-yl | -H | 2-Chlorphenyl | -CH₃ | 0 | Cl | | |
| 81 | Cyclohexyl | -H | 2-Chlorphenyl | -CH₃ | 0 | Cl | | |
| 82 | 1-Cyclohexylethyl | -H | 2-Chlorphenyl | -CH₃ | 0 | Cl | | |
| 83 | -CH₂-CF₃ | -H | 2-Chlorphenyl | -CH₃ | 0 | Cl | | |
| 84 | i-Butoxy | -H | 2,4,6-Trifluorphenyl | -CH₃ | 0 | Cl | 1,83 | |
| 85 | -O-C₂H₅ | -H | 2,4,6-Trifluorphenyl | -CH₃ | 0 | Cl | 2,99 | |
| 86 | 4-Trifluormethylbenzyloxy | -H | 2,4,6-Trifluorphenyl | -CH₃ | 0 | Cl | 4,06 | |
| 87 | -O-CH(CH₃)-CH₂-CH₃ | -H | 2,4,6-Trifluorphenyl | -CH₃ | 0 | Cl | 3,6 | |
| 88 | Allyloxy | -H | 2,4,6-Trifluorphenyl | -CH₃ | 0 | Cl | 3,15 | |
| 89 | t-Butoxy | -H | 2,4,6-Trifluorphenyl | -CH₃ | 0 | Cl | 3,52 | |
| 90 | -O-CH₃ | -H | 2,4,6-Trifluorphenyl | -CH₃ | 0 | Cl | 2,74 | |
| 91 | 2,2,2-Trifluor-1-methylethyl | -H | 3-Chlor-4-fluorphenyl | -CH₃ | 0 | Cl | 3,93 | |
| 92 | -i-Propyl | -H | 3-Chlor-4-fluorphcnyl | -CH₃ | 0 | Cl | 3,66 | |
| 93 | 2,2,2-Trifluor-1-methylethyl | -H | 2-Chlor-6-Fluorphenyl | -CH₃ | 0 | Cl | 3,63 | |
| 94 | -CH₂-CH₂-N(CH₃)-CH₂-CH₂- | * | 2-Chlor-6-Fluorphenyl | -CH₃ | 0 | Cl | 1,47 | |
| 95 | -CH₂-CH₂-CH₂-CH₂- | * | 2-Chlor-6-Fluorphenyl | -CH₃ | 0 | Cl | 3,52 | |
| 96 | -CH₂-CN | -H | 2-Chlor-6-Fluorphenyl | -CH₃ | 0 | Cl | 2,47 | |
| 97 | -CH₂-CH₂-CH₂-CH₂-CH₂- | * | 2-Chlor-6-Fluorphenyl | -CH₃ | 0 | Cl | 4,2 | |
| 98 | n-Propyl | -H | 2-Chlor-6-Fluorphenyl | -CH₃ | 0 | Cl | 3,37 | |
| 99 | Cyclopentyl | -H | 2-Chlor-6-Fluorphenyl | -CH₃ | 0 | Cl | 3,95 | |
| 100 | -C₂H₅ | -C₂H₅ | 2-Chlor-6-Fluorphenyl | -CH₃ | 0 | Cl | 3,97 | |
| 101 | 2-Methoxyethyl | -H | 2-Chlor-6-Fluorphenyl | -CH₃ | 0 | Cl | 2,89 | |
| 102 | Cyclopropyl | -H | 2-Chlor-6-Fluorphenyl | -CH₃ | 0 | Cl | 3,16 | |
| 103 | -CH₂-CH₂-S-CH₂-CH₂- | * | 2-Chlor-6-Fluorphenyl | -CH₃ | 0 | Cl | 3,75 | |
| 104 | -CH₂-CF₃ | -H | 2-Chlor-6-Fluorphenyl | -CH₃ | 0 | Cl | 3,22 | |
| 105 | -CH₂-CH₂-CH(CF₃)-CH₂-CH₂- | * | 2-Chlor-6-Fluorphenyl | -CH₃ | 0 | Cl | 4,22 | |
| 106 | Cyclopropylmethyl | -H | 2-Chlor-6-Fluorphenyl | -CH₃ | 0 | Cl | 3,47 | |
| 107 | -CH₂-C(CH₃)=CH₂ | -H | 2-Chlor-6-Fluorphenyl | -CH₃ | 0 | Cl | 3,38 | |
| 108 | -CH₂-CH₂-CF₃ | -H | 2-Chlor-6-Fluorphenyl | -CH₃ | 0 | Cl | 3,38 | |
| 109 | 1-Cyclohexylethyl | -H | 2-Chlor-6-Fluorphenyl | -CH₃ | 0 | Cl | 4,97 | |
| 110 | Cyclohexyl | -H | 2-Chlor-6-Fluorphenyl | -CH₃ | 0 | Cl | 4,24 | |
| 111 | 2-Butyl | -H | 2-Chlor-6-Fluorphenyl | -CH₃ | 0 | Cl | 3,74 | |
| 112 | N-Morpholinyl | -H | 2-Chlor-6-Fluorphenyl | -CH₃ | 0 | Cl | 2,73 | |
| 113 | Pyrrolidin-1-yl | -H | 2-Chlor-6-Fluorphenyl | -CH₃ | 0 | Cl | 3,58 | |
| 114 | 1-Cyclopropylethylamino | -H | 2-Chlor-6-Fluorphenyl | -CH₃ | 0 | Cl | 3,76 | |
| 115 | AB2 | -H | 2-Chlor-6-Fluorphenyl | -CH₃ | 0 | Cl | 4,45 | |
| 116 | i-Propylamino | -H | 2,4,6-Trifluorphenyl | -CH₃ | 0 | Cl | 3,29 | |
| 117 | t-Butylamino | -H | 2,4,6-Trifluorphenyl | -CH₃ | 2 | Cl | 3,44 | |
| 118 | -CH₂-CH₂-CH=C(CH₃)-CH₂- | * | 2,4,6-Trifluorphenyl | -CH₃ | 0 | Cl | 4,19 | |
| 119 | -CH₂-CH₂-CH=CH-CH₂- | * | 2,4,6-Trifluorphenyl | -CH₃ | 0 | Cl | 3,78 | |
| 120 | -CH₃ | -CH₃ | Pentafluorphenyl | -CH₃ | 0 | Cl | 3,54 | |
| 121 | -CH₂-CH(CH₃)-O-CH(CH₃)-CH₂- | * | Pentafluorphenyl | -CH₃ | 0 | Cl | 4,15 | |
| 122 | -C₂H₅ | -H | Pentafluorphenyl | -CH₃ | 0 | Cl | 3,51 | |
| 123 | -CH₂-CH₂-O-CH₂-CH₂- | * | Pentafluorphenyl | -CH₃ | 0 | Cl | 3,46 | |
| 124 | 2-Methoxyethyl | -H | Pentafluorphenyl | -CH₃ | 0 | Cl | 3,4 | |
| 125 | -CH₃ | -H | Pentafluorphenyl | -CH₃ | 0 | Cl | 3,15 | |
| 126 | -CH₂-CH₂-S-CH₂-CH₂- | * | Pentafluorphenyl | -CH₃ | 0 | Cl | 4,08 | |
| 127 | n-Propylamino | -H | Pentafluorphenyl | -CH₃ | 0 | Cl | 3,86 | |
| 128 | AB2 | -H | Pentafluorphenyl | -CH₃ | 0 | Cl | 4,97 | |
| 129 | -NH-CH₂-CF₂-CHF₂ | -H | Pentafluorphenyl | -CH₃ | 0 | Cl | 3,56 | |
| 130 | -NH-CH₂-CF₃ | -H | Pentafluorphenyl | -CH₃ | 0 | Cl | 3,55 | |
| 131 | i-Butoxy | -H | 2,6-Difluorphenyl | -CH₃ | 0 | Cl | 3,5 | |
| 132 | -O-C₂H₅ | -H | 2,6-Difluorphenyl | -CH₃ | 0 | Cl | 2,77 | |
| 133 | 3-Chlorbenzyloxy | -H | 2,6-Difluorphenyl | -CH₃ | 0 | Cl | 3,8 | |
| 134 | 4-Chlorbenzyloxy | -H | 2,6-Difluorphenyl | -CH₃ | 0 | Cl | 3,81 | |
| 135 | 4-Fluorbenzyloxy | -H | 2,6-Difluorphenyl | -CH₃ | 0 | Cl | 3,47 | |
| 136 | -O-CH(CH₃)-CH₂-CH₃ | -H | 2,6-Difluorphenyl | -CH₃ | 0 | Cl | 3,39 | |
| 137 | Allyloxy | -H | 2,6-Difluorphenyl | -CH₃ | 0 | Cl | 2,93 | |
| 138 | t-Butoxy | -H | 2,6-Difluorphenyl | -CH₃ | 0 | Cl | 3,3 | |
| 139 | -O-CH₃ | -H | 2,6-Difluorphenyl | -CH₃ | 0 | Cl | 2,52 | |
| 140 | -O-CH₃ | -CH₃ | 2,6-Difluorphenyl | -CH₃ | 0 | Cl | 3,19 | |
| 141 | Benzyloxy | -H | 2,4,6-Trifluorphenyl | -CH₃ | 0 | Cl | 3,68 | |
| 142 | 3,5-Dichlorbenzyloxy | -H | 2,4,6-Trifluorphenyl | -CH₃ | 0 | Cl | 4,43 | |
| 143 | 2-Chlorbenzyloxy | -H | 2,4,6-Trifluorphenyl | -CH₃ | 0 | Cl | 3,94 | |
| 144 | 4-Chlorbenzyloxy | -H | 2,4,6-Trifluorphenyl | -CH₃ | 0 | Cl | 4 | |
| 145 | 4-Fluorbenzyloxy | -H | 2,4,6-Trifluorphenyl | -CH₃ | 0 | Cl | 3,65 | |
| 146 | -n-Butoxy | -H | 2,4,6-Trifluorphenyl | -CH₃ | 0 | Cl | 3,73 | |
| 147 | 2,6-Dichlorbenzyloxy | -H | 2,4,6-Trifluorphenyl | -CH₃ | 0 | Cl | 4,07 | |
| 148 | -O-CH₃ | -CH₃ | 2,4,6-Trifluorphenyl | -CH₃ | 0 | Cl | 3,43 | |
| 149 | -CH₂-CH(CH₃)-O-CH(CH₃)-CH₂- | * | 2,4,6-Trifluorphenyl | -CH₃ | 0 | Cl | 3,7 | |
| 150 | -CH₃ | -CH₃ | 2-Fluorphenyl | -CH₃ | 0 | Cl | | |
| 151 | -CH₂-CH₂-CH₂-CH₂- | * | 2-Fluorphenyl | -CH₃ | 0 | Cl | | |
| 152 | -CH₂-CH₂-CH₂-CH₂-CH₂- | * | 2-Fluorphenyl | -CH₃ | 0 | Cl | | |
| 153 | n-Propyl | -H | 2-Fluorphenyl | -CH₃ | 0 | Cl | | |
| 154 | Cyclopentyl | -H | 2-Fluorphenyl | -CH₃ | 0 | Cl | | |
| 155 | -i-Propyl | -H | 2-Fluorphenyl | -CH₃ | 0 | Cl | | |
| 156 | -C₂H₅ | -C₂H₅ | 2-Fluorphenyl | -CH₃ | 0 | Cl | | |
| 157 | 2-Methoxyethyl | -H | 2-Fluorphenyl | -CH₃ | 0 | Cl | | |
| 158 | -CH₃ | -H | 2-Fluorphenyl | -CH₃ | 0 | Cl | | |
| 159 | Cyclopropyl | -H | 2-Fluorphenyl | -CH₃ | 0 | Cl | | |
| 160 | -CH₂-CH₂-CH(CF₃)-CH₂-CH₂- | * | 2-Fluorphenyl | -CH₃ | 0 | Cl | | |
| 161 | Cyclopropylmethyl | -H | 2-Fluorphenyl | -CH₃ | 0 | Cl | | |
| 162 | 2,2,2-Trifluor-1-methylethyl | -H | 2-Fluorphenyl | -CH₃ | 0 | Cl | | |
| 163 | -CH₂-C(CH₃)=CH₂ | -H | 2-Fluorphenyl | -CH₃ | 0 | Cl | | |
| 164 | -CH2-CH₂-CF₃ | -H | 2-Fluorphenyl | -CH₃ | 0 | Cl | | |
| 165 | 1-Cyclohexylethyl | -H | 2-Fluorphenyl | -CH₃ | 0 | Cl | | |
| 166 | Cyclohexyl | -H | 2-Fluorphenyl | -CH₃ | 0 | Cl | | |
| 167 | 2-Butyl | -H | 2-Fluorphenyl | -CH₃ | 0 | Cl | | |
| 168 | -NH-CH₂-CH(CH₃)₂ | -H | 2,6-Dichlor-3-fluor-5-trifluormethylphenyl | -CH₃ | 0 | Cl | 4,6 | |
| 169 | i-Butoxy | -H | 2-Fluorphenyl | -CH₃ | 0 | Cl | 3,52 | |
| 170 | -O-C₂H₅ | -H | 2-Fluorphenyl | -CH₃ | 0 | Cl | 2,76 | |
| 171 | 2-Chlorbenzyloxy | -H | 2-Fluorphenyl | -CH₃ | 0 | Cl | 3,78 | |
| 172 | 3-Chlorbenzyloxy | -H | 2-Fluorphenyl | -CH₃ | 0 | Cl | 3,83 | |
| 173 | 4-Chlorbenzyloxy | -H | 2-Fluorphenyl | -CH₃ | 0 | Cl | 3,85 | |
| 174 | 4-Fluorbenzyloxy | -H | 2-Fluorphenyl | -CH₃ | 0 | Cl | 3,48 | |
| 175 | -O-CH(CH₃)-CH₂-CH₃ | -H | 2-Fluorphenyl | -CH₃ | 0 | Cl | 3,42 | |
| 176 | Allyloxy | -H | 2-Fluorphenyl | -CH₃ | 0 | Cl | 2,92 | |
| 177 | t-Butoxy | -H | 2-Fluorphenyl | -CH₃ | 0 | Cl | 3,33 | |
| 178 | -O-CH₃ | -H | 2-Fluorphenyl | -CH₃ | 0 | Cl | 2,49 | |
| 179 | -O-CH₃ | -CH₃ | 2-Fluorphenyl | -CH₃ | 0 | Cl | 3,09 | |
| 180 | O-i-Propyl | -H | 2-Fluorphenyl | -CH₃ | 0 | Cl | 3,05 | |
| 181 | -CH₂-C(CH₃)=CH₂ | -C₂H₅ | 2,5-Difluorphenyl | -CH₃ | 0 | Cl | 4,25 | |
| 182 | -NH₂ | i-Butyl | 2,6-Dichlor-3-fluor-5-trifluormethylphenyl | -CH₃ | 0 | Cl | 4,46 | |
| 183 | 3-Trifluormethylbenzyloxy | -H | 2,4,6-Trifluorphenyl | -CH₃ | 0 | Cl | 4,04 | |
| 184 | Cyclohexyl | -H | 2,4,6-Trifluorphenyl | -CH₃ | 0 | Cl | 4,18 | |
| 185 | -CH₂-CH(CH₃)-(7-CH(CH₃) CH₂- | * | 2,6-Difluorphenyl | -CH₃ | 0 | Cl | 3,53 | |
| 186 | -CH₂-CH₂-CH₂-CH₂- | * | 2,6-Difluorphenyl | -CH₃ | 0 | Cl | 3,26 | |
| 187 | -CH₂-CN | -H | 2,6-Difluorphenyl | -CH₃ | 0 | Cl | 2,29 | |
| 188 | -CH₂-CH₂-CH₂-CH₂-CH₂- | * | 2,6-Difluorphenyl | -CH₃ | 0 | Cl | 3,9 | |
| 189 | n-Propyl | -H | 2,6-Difluorphenyl | -CH₃ | 0 | Cl | 3,15 | |
| 190 | Cyclopentyl | -H | 2,6-Difluorphenyl | -CH₃ | 0 | Cl | 3,69 | |
| 191 | -i-Propyl | -H | 2,6-Difluorphenyl | -CH₃ | 0 | Cl | 3,18 | |
| 192 | -C₂H₅ | -C₂H₅ | 2,6-Difluorphenyl | -CH₃ | 0 | Cl | 3,7 | |
| 193 | -CH₂-CH₂-O-CH₂-CH₂- | * | 2,6-Difluorphenyl | -CH₃ | 0 | Cl | 2,8 | |
| 194 | 2-Methoxyethyl | -H | 2,6-Difluorphenyl | -CH₃ | 0 | Cl | 2,68 | |
| 195 | Cyclopropyl | -H | 2,6-Difluorphenyl | -CH₃ | 0 | Cl | 2,91 | |
| 196 | -CH₂-CH₂-S-CH₂-CH₂- | * | 2,6-Difluorphenyl | -CH₃ | 0 | Cl | 3,49 | |
| 197 | -CH₂-CF₃ | -H | 2,6-Difluorphenyl | -CH₃ | 0 | Cl | 3,01 | |
| 198 | -CH₂-CH₂-CH(CF₃)-CH₂-CH₂- | * | 2,6 Difluorphenyl | -CH₃ | 0 | Cl | 3,97 | |
| 199 | Cyclopropylmethyl | -H | 2,6-Difluorphenyl | -CH₃ | 0 | Cl | 3,24 | |
| 200 | -CH₂-C(CH₃)=CH₂ | -H | 2,6-Difluorphenyl | -CH₃ | 0 | Cl | 3,15 | |
| 201 | -CH₂CH₂-CF₃ | -H | 2,6-Difluorphenyl | -CH₃ | 0 | Cl | 3,18 | |
| 202 | 1-Cyclohexylethyl | -H | 2,6-Difluorphenyl | -CH₃ | 0 | Cl | 4,7 | |
| 203 | Cyclohexyl | -H | 2,6-Difluorphenyl | -CH₃ | 0 | Cl | 3,97 | |
| 204 | 2-Butyl | -H | 2,6-Difluorphenyl | -CH₃ | 0 | Cl | 3,49 | |
| 205 | 3-Trifluormethylcyclohexyl | -H | 2,6-Difluorphenyl | -CH₃ | 0 | Cl | 3,98 | |
| 206 | 2-Trifluormethylcyclohexyl | -H | 2,6-Difluorphenyl | -CH₃ | 0 | Cl | 4,06 | |
| 207 | 3,5-bis-Trifluormethylcyclohexyl | -H | 2,6-Difluorphenyl | -CH₃ | 0 | Cl | 4,14 | |
| 208 | 4-Trifluormethylcyclohexyl | -H | 2,6-Difluorphenyl | -CH₃ | 0 | Cl | 4 | |
| 209 | -CH₂-C(CH₃)=CH₂ | -C₂H₅ | 2,4-Diflu phenyl | -CH₃ | 0 | Cl | 4,36 | |
| 210 | -CH₃ | -CH₃ | 2,4,6-Tri phenyl | -CH₃ | 0 | Cl | 3,15 | |
| 211 | -C₂H₅ | -H | 2,4,6-Trifluorphenyl | -CH₃ | 0 | Cl | 3,03 | |
| 212 | -CH₃ | -H | 2,4,6-Trifluorphenyl | -CH₃ | 0 | Cl | 2,68 | |
| 213 | -CH₂-CH₂-CH=CH-CH₂- | * | 2-Chlor-6-Fluorphenyl | -CH₃ | 0 | Cl | 3,89 | |
| 214 | 2-Hydroxypropyl | -H | 2-Chlor-6-Fluorphenyl | -CH₃ | 0 | Cl | 2,38 | |
| 215 | -CH₂ | n-Propyl | 2,4,6-Trifluorphenyl | -CH₃ | 0 | Cl | 3,28 | 210-11 |
| 216 | -CH₂-C(CH₃)=CH₂ | -C₂H₅ | 2-Chlor-6-Fluorphenyl | -CH₃ | 0 | Cl | 4,56 | |
| 217 | -NH-CH₂-CH(CH₃)₂ | -H | 2,6-Dichlorphenyl | -CH₃ | 0 | Cl | 3,96 | 213-15 |
| 219 | -NH₂ | i-Butyl | 4-Chlor-3-fluorphenyl | -CH₃ | 0 | Cl | 3,91 | 155-7 |
| 220 | -NH₂ | i-Butyl | 2,4-Difluorphenyl | -CH₃ | 0 | Cl | 3,58 | 162-64 |
| 223 | -CH₂-C(CH₃)=CH₂ | -C₂H₅ | 2-Chlorphenyl | -CH₃ | 0 | Cl | 4,57 | |
| 224 | -CH₃ | -CH₃ | 2-Chlor-6-Fluorphenyl | -CH₃ | 0 | Cl | 3,23 | |
| 225 | -CH₂-CH(CH₃)-O-CH(CH₃)-CH₂- | * | 2-Chlor-6-Fluorphenyl | -CH₃ | 0 | Cl | 3,84 | |
| 226 | -C₂H₅ | -H | 2-Chlor-6-Fluorphenyl | -CH₃ | 0 | Cl | 3,06 | |
| 227 | -C(CH₃)₂-CF₃ | -H | 2-Chlor-6-Fluorphenyl | -CH₃ | 0 | Cl | 4,19 | |
| 228 | -CH₃ | -H | 2-Chlor-6-Fluorphenyl | -CH₃ | 0 | Cl | 2,71 | |
| 229 | -CH(CF₃)-CH₂-CH₂-CH₂-CH₂- | * | 2-Chlor-6-Fluorphenyl | -CH₃ | 0 | Cl | 4,43 | |
| 230 | 2,2,2-Trifluor-1-methylethyl | -H | 2-Chlor-6-Fluorphenyl | -CH₃ | 0 | Cl | 3,61 | |
| 231 | Dimethylamino | -H | 2-Chlor-6-Fluorphenyl | -CH₃ | 0 | Cl | 3,18 | |
| 232 | -NH-CH₃ | -CH₃ | 2-Chlor-6-Fluorphenyl | -CH₃ | 0 | Cl | 3,12 | |
| 233 | Ethylamino | -H | 2-Chlor-6-Fluorphenyl | -CH₃ | 0 | Cl | 2,99 | |
| 234 | 1-Ethyl-1-propylamino | -H | 2-Chlor-6-Fluorphenyl | -CH₃ | 0 | Cl | 4,08 | |
| 235 | n-Butylamino | -H | 2-Chlor-6-Fluorphenyl | -CH₃ | 0 | Cl | 3,73 | |
| 236 | -NH-CH₂-CF₂-CHF₂ | -H | 2-Chlor-6-Fluorphenyl | -CH₃ | 0 | Cl | 3,21 | |
| 237 | Allylamino | -H | 2-Chlor-6-Fluorphenyl | -CH₃ | 0 | Cl | 3,19 | |
| 238 | -NH-CH₂-CF₃ | -H | 2-Chlor-6-Fluorphenyl | -CH₃ | 0 | Cl | 3,12 | |
| 239 | -N(CH₃)-COOCH₃ | -H | 2-Chlor-6-Fluorphenyl | -CH₃ | 0 | Cl | 2,47 | |
| 240 | 4-Trifluormethylcyclohexyl | -H | 2,4,6-Trifluorphenyl | -CH₃ | 0 | Cl | 4,21 | |
| 241 | 2-Methoxyethyl | n-Propyl | 2,4,6-Trifluorphenyl | -CH₃ | 0 | Cl | 4,03 | |
| 242 | -CH₂-CH₂-NH₂ | -i-Propyl | 2,4,6-Trifluorphenyl | -CH₃ | 0 | Cl | 1,61 | |
| 243 | -CH₂-CH(OH)-CH₂-CH₂- | * | 2,4,6-Trifluorphenyl | -CH₃ | 0 | Cl | 2,38 | |
| 244 | -CH₂-CH₂-CH₂-CH(CH₃)- | * | 2,4,6-Trifluorphenyl | -CH₃ | 0 | Cl | 3,88 | |
| 245 | -CH₂-CH(NH₂)-CH₂-CH₂- | * | 2,4,6-Trifluorphenyl | -CH₃ | 0 | Cl | 1,51 | |
| 246 | AB8 | * | 2,4,6-Trifluorphenyl | -CH₃ | 0 | Cl | 2,22 | |
| 247 | AB9 | * | 2,4,6-Trifluorphenyl | -CH₃ | 0 | Cl | 1,56 | |
| 248 | -CH₂-CH₂-CH(OH)-CH₂-CH₂- | * | 2,4,6-Trifluorphenyl | -CH₃ | 0 | Cl | 2,5 | |
| 249 | AB10 | * | 2,4,6-Trifluorphenyl | -CH₃ | 0 | Cl | 3,43 | |
| 250 | -CH₂-CH₂-CH₂-CH₂-CH(CH₃) | * | 2,4,6-Trifluorphenyl | -CH₃ | 0 | Cl | 4,45 | |
| 251 | -CH₂CH₂-CH₂CH(CH₃)-CH₂- | * | 2,4,6-Trifluorphenyl | -CH₃ | 0 | Cl | 4,51 | |
| 252 | -CH₂-CH₂-CH(CH₃)-CH₂-CH₂- | * | 2,4,6-Trifluorphenyl | -CH₃ | 0 | Cl | 4,51 | |
| 253 | -CH₂-CH₂-C(CH₃)₂-CH₂-CH₂- | * | 2,4,6-Trifluorphenyl | -CH₃ | 0 | Cl | 4,78 | |
| 254 | AB11 | * | 2,4,6-Trifluorphenyl | -CH₃ | 0 | Cl | 1,68 | |
| 255 | -CH₂-CH₂-N(CH₃)₂ | -CH₃ | 2,4,6-Trifluorphenyl | -CH₃ | 0 | Cl | 1,64 | |
| 256 | 3-(Dimethylamino)-propyl | -CH₃ | 2,4,6-Trifluorphenyl | -CH₃ | 0 | Cl | 1,74 | |
| 257 | -CH₂-CH₂-N(CH₃)₂ | -C₂H₅ | 2,4,6-Trifluorphenyl | -CH₃ | 0 | Cl | 1,82 | |
| 258 | -CH₂CH₂OH | -C₂H₅ | 2,4,6-Trifluorphenyl | -CH₃ | 0 | Cl | 2,32 | |
| 259 | -CH₂-CH₂-NH₂ | -C₂H₅ | 2,4,6-Trifluorphenyl | -CH₃ | 0 | Cl | 1,54 | |
| 260 | -CH₂-CH₂-NH₂ | n-Propyl | 2,4,6-Trifluorphenyl | -CH₃ | 0 | Cl | 1,66 | |
| 261 | 3-Aminopropyl | n-Propyl | 2,4,6-Trifluorphenyl | -CH₃ | 0 | Cl | 1,72 | |
| 262 | Benzyloxy | -H | 2,6-Difluorphenyl | -CH₃ | 0 | Cl | 3,49 | |
| 263 | 3,5-Dichlorbenzyloxy | -H | 2,6-Difluorphenyl | -CH₃ | 0 | Cl | 4,33 | |
| 264 | 4-Trifluormethylbenzyloxy | -H | 2,6-Difluorphenyl | -CH₃ | 0 | Cl | 3,97 | |
| 265 | 2-Chlorbenzyloxy | -H | 2,6-Difluorphenyl | -CH₃ | 0 | Cl | 3,77 | |
| 266 | 3-Trifluormethylbenzyloxy | -H | 2,6-Difluorphenyl | -CH₃ | 0 | Cl | 3,94 | |
| 267 | -n-Butoxy | -H | 2,6-Difluorphenyl | -CH₃ | 0 | Cl | 3,53 | |
| 268 | 2,6-Dichlorbenzyloxy | -H | 2,6-Difluorphenyl | -CH₃ | 0 | Cl | 3,92 | |
| 269 | Benzyloxy | -H | 2-Fluorphenyl | -CH₃ | 0 | Cl | 3,49 | |
| 270 | 3,5-Dichlorbenzyloxy | -H | 2-Fluorphenyl | -CH₃ | 0 | Cl | 4,37 | |
| 271 | -O-n-Propyl | -H | 2-Fluorphenyl | -CH₃ | 0 | Cl | 4,35 | |
| 272 | 4-Trifluormethylbenzyloxy | -H | 2-Fluorphenyl | -CH₃ | 0 | Cl | 4,02 | |
| 273 | 3-Trifluormethylbenzyloxy | -H | 2-Fluorphenyl | -CH₃ | 0 | Cl | 3,99 | |
| 274 | -n-Butoxy | -H | 2-Fluorphenyl | -CH₃ | 0 | Cl | 3,57 | |
| 275 | 2-Hexahydropyranyloxy | -H | 2-Fluorphenyl | -CH₃ | 0 | Cl | 3,2 | |
| 276 | 2,6-Dichlorbenzyloxy | -H | 2-Fluorphenyl | -CH₃ | 0 | Cl | 4,03 | |
| 277 | i-Butyl | -H | 2,4,6-Trifluorphenyl | -CH₃ | 0 | Cl | 3,66 | |
| 278 | n-Butyl | -H | 2,4,6-Trifluorphenyl | -CH₃ | 0 | Cl | 3,71 | |
| 279 | -CH₂-C(CH₃)₃ | -H | 2,4,6-Trifluorphenyl | -CH₃ | 0 | Cl | 4,06 | |
| 280 | Propargyl | -CH₃ | 2,4,6-Trifluorphenyl | -CH₃ | 0 | Cl | 3,28 | |
| 281 | 1,3-Dioxolan-2-ylmethyl | -CH₃ | 2,4,6-Trifluorphenyl | -CH₃ | 0 | Cl | 3,23 | |
| 282 | Allyl | -CH₃ | 2,4,6-Trifluorphenyl | -CH₃ | 0 | Cl | 3,76 | |
| 283 | -CH₂-CH₂-CN | -CH₃ | 2,4,6-Trifluorphenyl | -CH₃ | 0 | Cl | 2,83 | |
| 284 | -CH₂-CH(OCH₃)₂ | -CH₃ | 2,4,6-Trifluorphenyl | -CH₃ | 0 | Cl | 3,36 | |
| 285 | (2-Furyl)methyl | -CH₃ | 2,4,6-Trifluorphenyl | -CH₃ | 0 | Cl | 3,77 | |
| 286 | i-Butyl | -CH₃ | 2,4,6-Trifluorphenyl | -CH₃ | 0 | Cl | 4,25 | |
| 287 | 2-Methoxyethyl | -CH₃ | 2,4,6-Trifluorphenyl | -CH₃ | 0 | Cl | 3,27 | |
| 288 | -CH₂-C(CH₃)=CH₂ | -CH₃ | 2,4,6-Trifluorphenyl | -CH₃ | 0 | Cl | 4,11 | |
| 289 | n-Butyl | -CH₃ | 2,4,6-Trifluorphenyl | -CH₃ | 0 | Cl | 4,31 | |
| 290 | i-Butyl | -H | Pentafluorphenyl | -CH₃ | 0 | Cl | 4,09 | |
| 291 | -CH₂-CH₂-CF₃ | -H | Pentafluorphenyl | -CH₃ | 0 | Cl | 3,74 | |
| 292 | Cyclohexyl | -H | Pentafluorphenyl | -CH₃ | 0 | Cl | 4,59 | |
| 293 | 1-Cyclohexylethyl | -H | Pentafluorphenyl | -CH₃ | 0 | Cl | 5,23 | |
| 294 | -CH₂-C(CH₃)=CH₂ | -H | Pentafluorphenyl | -CH₃ | 0 | Cl | 3,78 | |
| 295 | 4-Trifluormethylcyclohexyl | -H | Pentafluorphenyl | -CH₃ | 0 | Cl | 4,53 | |
| 296 | -CH(CH₃)-CH₂-CH(CH₃)₂ | -H | Pentafluorphenyl | -CH₃ | 0 | Cl | 4,76 | |
| 297 | -CH₂-CH₂-N(CH₃)₂ | -CH₃ | Pentafluorphenyl | -CH₃ | 0 | Cl | 1,82 | |
| 298 | 1,3-Dioxolan-2-ylmethyl | -CH₃ | Pentafluorphenyl | -CH₃ | 0 | Cl | 3,64 | |
| 299 | -CH₂-CH₂-CN | -CH₃ | Pentafluorphenyl | -CH₃ | 0 | Cl | 3,19 | |
| 300 | -CH₂-CH(OCH₃)₂ | -CH₃ | Pentafluorphenyl | -CH₃ | 0 | Cl | 3,78 | |
| 301 | (2-Furyl)methyl | -CH₃ | Pentafluorphenyl | -CH₃ | 0 | Cl | 4,11 | |
| 302 | i-Butyl | -CH₃ | Pentafluorphenyl | -CH₃ | 0 | Cl | 4,58 | |
| 303 | 2-Methoxyethyl | -CH₃ | Pentafluorphenyl | -CH₃ | 0 | Cl | 3,69 | |
| 304 | -CH₂-C(CH₃)=CH₂ | -CH₃ | Pentafluorphenyl | -CH₃ | 0 | Cl | 4,43 | |
| 305 | -CH₂-CH₂-N(CH₃)₂ | -C₂H₅ | Pentafluorphenyl | -CH₃ | 0 | Cl | 1,97 | |
| 306 | (2-Tetrahydrofuryl)methyl | -C₂H₅ | Pentafluorphenyl | -CH₃ | 0 | Cl | 4,31 | |
| 307 | 2-Methoxyethyl | -C₂H₅ | Pentafluorphenyl | -CH₃ | 0 | Cl | 4,05 | |
| 308 | -CH₂-CH(CH₃)-O-CH(CH₃)-CH₂- | * | 2-Fluorphenyl | -CH₃ | 0 | Cl | 3,5 | |
| 309 | -C₂H₅ | -H | 2-Fluorphenyl | -CH₃ | 0 | Cl | 2,83 | |
| 310 | -CH₂-CN | -H | 2-Fluorphenyl | -CH₃ | 0 | Cl | 2,27 | |
| 311 | -CH₂-CH₂-O-CH₂-CH₂- | * | 2-Fluorphenyl | -CH₃ | 0 | Cl | 2,78 | |
| 312 | -C(CH₃)₂-CH₂-COCH₃ | -H | 2-Fluorphenyl | -CH₃ | 0 | Cl | 3,2 | |
| 313 | -C₂H₅ | -H | 2,6-Difluorphenyl | -CH₃ | 0 | Cl | 2,86 | |
| 314 | -C(CH₃)₂-CF₃ | -H | 2,6-Difluorphenyl | -CH₃ | 0 | Cl | 4,05 | |
| 315 | -CH₃ | -H | 2,6-Difluorphenyl | -CH₃ | 0 | Cl | 2,53 | |
| 316 | -CH(CF₃)-CH₂-CH₂-CH₂-CH₂- | * | 2,6-Difluorphenyl | -CH₃ | 0 | Cl | 4,05 | |
| 317 | 2,2,2-Trifluor-1-methylethyl | -H | 2,6-Difluorphenyl | -CH₃ | 0 | Cl | 3,27 | |
| 318 | -CH₃ | -CH₃ | 2,6-Difluorphenyl | -CH₃ | 0 | Cl | 3 | |
| 319 | i-Butoxy | -H | Pentafluorphenyl | -CH₃ | 0 | Cl | 4,17 | |
| 320 | -O-C₂H₅ | -H | Pentafluorphenyl | -CH₃ | 0 | Cl | 3,43 | |
| 321 | Benzyloxy | -H | Pentafluorphenyl | -CH₃ | 0 | Cl | 4,11 | |
| 322 | 3,5-Dichlorbenzyloxy | -H | Pentafluorphenyl | -CH₃ | 0 | Cl | 4,75 | |
| 323 | 2,4-Dichlorbenzyloxy | -H | Pentafluorphenyl | -CH₃ | 0 | Cl | 4,73 | |
| 324 | 4-Trifluormethylbenzyloxy | -H | Pentafluorphenyl | -CH₃ | 0 | Cl | 4,41 | |
| 325 | 2-Chlorbenzyloxy | -H | Pentafluorphenyl | -CH₃ | 0 | Cl | 4,3 | |
| 326 | 3-Chlorbenzyloxy | -H | Pentafluorphenyl | -CH₃ | 0 | Cl | 4,33 | |
| 327 | 4-Chlorbenzyloxy | -H | Pentafluorphenyl | -CH₃ | 0 | Cl | 4,31 | |
| 328 | 4-Fluorbenzyloxy | -H | Pentafluorphenyl | -CH₃ | 0 | Cl | 4,04 | |
| 329 | -O-CH(CH₃)-CH₂-CH₃ | -H | Pentafluorphenyl | -CH₃ | 0 | Cl | 4,06 | |
| 330 | 3-Trifluormethylbenzyloxy | -H | Pentafluorphenyl | -CH₃ | 0 | Cl | 4,37 | |
| 331 | -n-Butoxy | -H | Pentafluorphenyl | -CH₃ | 0 | Cl | 4,39 | |
| 332 | Allyloxy | -H | Pentafluorphenyl | -CH₃ | 0 | Cl | 3,59 | |
| 333 | t-Butoxy | -H | Pentafluorphenyl | -CH₃ | 0 | Cl | 3,98 | |
| 334 | 2-Hexahydropyranyloxy | -H | Pentafluorphenyl | -CH₃ | 0 | Cl | 3,82 | |
| 335 | 2,6-Dichlorbenzyloxy | -H | Pentafluorphenyl | -CH₃ | 0 | Cl | 4,23 | |
| 336 | -O-CH₃ | -H | Pentafluorphenyl | -CH₃ | 0 | Cl | 3,15 | |
| 337 | -O-CH₃ | -CH₃ | Pentafluorphenyl | -CH₃ | 0 | Cl | 3,85 | |
| 338 | O-i-Propyl | -H | Pentafluorphenyl | -CH₃ | 0 | Cl | 3,71 | |
| 339 | 2,2,2-Trifluor-1-methylethyl | -H | 2,4-Difluorphenyl | -CH₃ | 0 | Cl | 3,52 | |
| 340 | 1-Cyclopropylethyl | -H | 2,4,6-Trifluorphenyl | -CH₃ | 0 | Cl | 3,71 | |
| 341 | i-Butyl | -H | 2,6-Difluorphenyl | -CH₃ | 0 | Cl | 3,46 | |
| 342 | n-Butyl | -H | 2,6-Difluorphenyl | -CH₃ | 0 | Cl | 3,5 | |
| 343 | -CH₂-C(CH₃)₃ | -H | 2,6-Difluorphenyl | -CH₃ | 0 | Cl | 3,88 | |
| 344 | -CH(CH₃CH₂-CH(CH₃)₂ | -H | 2,6-Difluorphenyl | -CH₃ | 0 | Cl | 4,19 | |
| 345 | Propargyl | -CH₃ | 2,6-Difluorphenyl | -CH₃ | 0 | Cl | 3,1 | |
| 346 | Allyl | -CH₃ | 2,6-Difluorphenyl | -CH₃ | 0 | Cl | 3,59 | |
| 347 | -CH₂-CH₂-CN | -CH₃ | 2,6-Difluorphenyl | -CH₃ | 0 | Cl | 2,66 | |
| 348 | -CH₂-CH(OCH₃)₂ | -CH₃ | 2,6-Difluorphenyl | -CH₃ | 0 | Cl | 3,16 | |
| 349 | (2-Furyl)methyl | -CH₃ | 2,6-Difluorphenyl | -CH₃ | 0 | Cl | 3,58 | |
| 350 | i-Butyl | -CH₃ | 2,6-Difluorphenyl | -CH₃ | 0 | Cl | 4,06 | |
| 351 | 2-Methoxyethyl | -CH₃ | 2,6-Difluorphenyl | -CH₃ | 0 | Cl | 3,08 | |
| 352 | -CH₂-C(CH₃)=CH₂ | -CH₃ | 2,6-Difluorphenyl | -CH₃ | 0 | Cl | 3,94 | |
| 353 | n-Butyl | -CH₃ | 2,6-Difluorphenyl | -CH₃ | 0 | Cl | 4,13 | |
| 354 | -CH₂-CH₂-N(CH₃)₂ | -C₂H₅ | 2,6-Difluorphenyl | -CH₃ | 0 | Cl | 1,66 | |
| 355 | Allyl | -C₂H₅ | 2,6-Difluorphenyl | -CH₃ | 0 | Cl | 3,91 | |
| 356 | (2-Furyl)methyl | -C₂H₅ | 2,6-Difluorphenyl | -CH₃ | 0 | Cl | 3,89 | |
| 357 | (2-Tetrahydrofuryl)methyl | -C₂H₅ | 2,6-Difluorphenyl | -CH₃ | 0 | Cl | 3,67 | |
| 358 | -CH₂-CH₂-CN | -C₂H₅ | 2,6-Difluorphenyl | -CH₃ | 0 | Cl | 2,93 | |
| 359 | 2-Methoxyethyl | -C₂H₅ | 2,6-Difluorphenyl | -CH₃ | 0 | Cl | 3,4 | |
| 360 | -CH₂-COOC₂H₅ | -C₂H₅ | 2,6-Difluorphenyl | -CH₃ | 0 | Cl | 3,38 | |
| 361 | n-Butyl | -C₂H₅ | 2,6-Difluorphenyl | -CH₃ | 0 | Cl | 4,47 | |
| 362 | -CH₂CH₂OH | n-Propyl | 2,6-Difluorphenyl | -CH₃ | 0 | Cl | 2,65 | |
| 363 | 3-Aminopropyl | n-Propyl | 2,6-Difluorphenyl | -CH₃ | 0 | Cl | 1,55 | |
| 364 | Cyclopropylmethyl | n-Propyl | 2,6-Difluorphenyl | -CH₃ | 0 | Cl | 4,49 | |
| 365 | (2-Tetrahydropyranyl)methyl | n-Propyl | 2,6-Difluorphenyl | -CH₃ | 0 | Cl | 4,64 | |
| 366 | (2-Tetrahydrofuryl)methyl | n-Propyl | 2,6-Difluorphenyl | -CH₃ | 0 | Cl | 4,06 | |
| 367 | 2,2-Diethoxy-ethyl | n-Butyl | 2,4-Difluorphenyl | -CH₃ | 0 | Cl | 5,03 | |
| 368 | 2,2,2-Trifluor-1-methylethyl | -H | 2,5-Difluorphenyl | -CH₃ | 0 | Cl | 3,43 | |
| 369 | -O-CH₃ | -H | 2,4-Difluorphenyl | -CH₃ | 0 | Cl | 2,66 | |
| 370 | n-Butyl | -C₂H₅ | Pentafluorphenyl | -CH₃ | 0 | Cl | 4,98 | |
| 371 | Cyclopropylmethyl | n-Propyl | Pentafluorphenyl | -CH₃ | 0 | Cl | 5,01 | |
| 372 | (2-Tetrahydropyranyl)methyl | n-Propyl | Pentafluorphenyl | -CH₃ | 0 | Cl | 5,29 | |
| 373 | (2-Tetrahydrofuryl)methyl | n-Propyl | Pentafluorphenyl | -CH₃ | 0 | Cl | 4,72 | |
| 374 | 2-Methoxyethyl | n-Propyl | Pentafluorphenyl | -CH₃ | 0 | Cl | 4,42 | |
| 375 | -CH₂-CH(OH)-CH₂-CH₂- | ***** | Pentafluorphenyl | -CH₃ | 0 | Cl | 2,72 | |
| 376 | -CH₂-CH₂-CH₂-CH(CH₃)- | * | Pentafluorphenyl | -CH₃ | 0 | Cl | 4,21 | |
| 377 | AB8 | * | Pentafluorphenyl | -CH₃ | 0 | Cl | 2,56 | |
| 378 | AB9 | ***** | Pentafluorphenyl | -CH₃ | 0 | Cl | 1,79 | |
| 379 | -CH₂-CH₂-CH(OH)CH₂-CH₂- | * | Pentafluorphenyl | -CH₃ | 0 | Cl | 2,84 | |
| 380 | n-Butyl | -H | 2,4-Difluorphenyl | -CH₃ | 0 | Cl | 3,62 | |
| 381 | -CH₂COOC₂H₅ | -CH₃ | 2,4,6-Trifluorphenyl | -CH₃ | 0 | Cl | 3,31 | |
| 382 | -CH₂-CN | -CH₃ | 2,4,6-Trifluorphenyl | -CH₃ | 0 | Cl | 2,85 | |
| 383 | -CH₂-COOCH₃ | -CH₃ | 2,4,6-Trifluorphenyl | -CH₃ | 0 | Cl | 2,98 | |
| 384 | -CH₂-CH₂-Cl | -CH₃ | 2,4,6-Trifluorphenyl | -CH₃ | 0 | Cl | 3,61 | |
| 385 | -CH₂-CH₂-O-CH=CH₂ | -CH₃ | 2,4,6-Trifluorphenyl | -CH₃ | 0 | Cl | 2,45 | |
| 386 | 2-Butyl | -H | 2,4,6-Trifluorphenyl | -CH₃ | 0 | Cl | 3,74 | |
| 387 | -CH=C(CH₃)₂ | -C₂H₅ | 2,4,6-Trifluorphenyl | -CH₃ | 0 | Cl | 2,18 | |
| 388 | Allyl | -C₂H₅ | 2,4,6-Trifluorphenyl | -CH₃ | 0 | Cl | 4,14 | |
| 389 | (2-Furyl)methyl | -C₂H₅ | 2,4,6-Trifluorphenyl | -CH₃ | 0 | Cl | 4,14 | |
| 390 | (2-Tetrahydrofuryl)methyl | -C₂H₅ | 2,4,6-Trifluorphenyl | -CH₃ | 0 | Cl | 3,93 | |
| 391 | -CH₂-CH₂-CN | -C₂H₅ | 2,4,6-Trifluorphenyl | -CH₃ | 0 | Cl | 3,15 | |
| 392 | -CH₂-COOC₂H₅ | -C₂H₅ | 2,4,6-Trifluorphenyl | -CH₃ | 0 | Cl | 3,65 | |
| 393 | n-Butyl | -C₂H₅ | 2,4,6-Trifluorphenyl | -CH₃ | 0 | Cl | 4,7 | |
| 394 | -CH₂CH₂OH | n-Propyl | 2,4,6-Trifluorphenyl | -CH₃ | 0 | Cl | 2,96 | |
| 395 | Cyclopropylmethyl | n-Propyl | 2,4,6-Trifluorphenyl | -CH₃ | 0 | Cl | 4,73 | |
| 396 | (2-Tetrahydropyranyl)methyl | n-Propyl | 2,4,6-Trifluorphenyl | -CH₃ | 0 | Cl | 4,91 | |
| 397 | -CH₂-CH₂-COOC₂H₅ | n-Propyl | 2,4,6-Trifluorphenyl | -CH₃ | 0 | Cl | 3,47 | |
| 398 | (2-Tetrahydrofuryl)methyl | n-Propyl | 2,4,6-Trifluorphenyl | -CH₃ | 0 | Cl | 4,34 | |
| 399 | 2-Thienylmethyl | n-Propyl | 2,4,6-Trifluorphenyl | -CH₃ | 0 | Cl | 4,76 | |
| 400 | -C(CH₃)₂-CF₃ | -H | 2,4,6-Trifluorphenyl | -CH₃ | 0 | Cl | 4,07 | |
| 401 | 1-Cyclohexylethyl | -H | 2,4,6-Trifluorphenyl | -CH₃ | 0 | Cl | 4,93 | |
| 402 | 3-Trifluormethylcyclohexyl | -H | 2,4,6-Trifluorphenyl | -CH₃ | 0 | Cl | 4,21 | |
| 403 | 2-Trifluormethylcyclohexyl | -H | 2,4,6-Trifluorphenyl | -CH₃ | 0 | Cl | 4,31 | |
| 404 | -CH(CH₃)-CH₂-CH(CH₃)₂ | -H | 2,4,6-Trifluorphenyl | -CH₃ | 0 | Cl | 4,43 | |
| 405 | 3,5-bis-Trifluormethylcyclohexyl | -H | 2,4,6-Trifluorphenyl | -CH₃ | 0 | Cl | 4,37 | |
| 406 | -CH₂-COOC₂H₅ | Cyclopropyl | 2,4,6-Trifluorphenyl | -CH₃ | 0 | Cl | 3,76 | |
| 407 | -CH(CF₃)-CH₂-CH₂-CH₂- | * | 2,4,6-Trifluorphenyl | -CH₃ | 0 | Cl | 4,07 | |
| 408 | -CH₂-CH₂-CO-CH₂-CH₂- | * | 2,4,6-Trifluorphenyl | -CH₃ | 0 | Cl | 2,84 | |
| 409 | -CH₂-CH(CH₃)-CH₂-CH(CH₃)-CH₂- | * | 2,4,6-Trifluorphenyl | -CH₃ | 0 | Cl | 4,87 | |
| 410 | -CH₂-CH(OH)-CH₂-CH₂-CH₂- | * | 2,4,6-Trifluorphenyl | -CH₃ | 0 | Cl | 2,74 | |
| 411 | -CH₂-CH₂-CH(COCH₃)-CH₂-CH₂- | * | 2,4,6-Trifluorphenyl | -CH₃ | 0 | Cl | 3,2 | |
| 412 | -CH₂CH=C(C₂H₅)-CH₂CH₂- | * | 2,4,6-Trifluorphenyl | -CH₃ | 0 | Cl | 3,85 | |
| 413 | -CH(CH₂NH₂)-CH₂-CH₂-CH₂-CH₂- | * | 2,4,6-Trifluorphenyl | -CH₃ | 0 | Cl | 4,68 | |
| 414 | -CH₂-CH₂-CH(COOCH₃)-CH₂-CH₂- | * | 2,4,6-Trifluorphenyl | -CH₃ | 0 | Cl | 3,94 | |
| 415 | -CH₂-CH₂-CHBr-CH₂-CH₂- | * | 2,4,6-Trifluorphenyl | -CH₃ | 0 | Cl | 3,51 | |
| 416 | -CH(COOCH₃)-CH₂-CH₂-CH₂-CH₂- | * | 2,4,6-Trifluorphenyl | -CH₃ | 0 | Cl | 4,16 | |
| 417 | -CH₂-CH₂-CHF-CH₂-CH₂- | * | 2,4,6-Trifluorphenyl | -CH₃ | 0 | Cl | 3,61 | |
| 418 | AB12 | * | 2,4,6-Trifluorphenyl | -CH₃ | 0 | Cl | 3,98 | |
| 419 | -CH₂-CH₂-CH(NH-COCH₃)-CH₂-CH₂- | * | 2,4,6-Trifluorphenyl | -CH₃ | 0 | Cl | 2,44 | |
| 420 | -CH(CF₃)-CH₂-CH₂-CH₂-CH₂- | * | 2,4,6-Trifluorphenyl | -CH₃ | 0 | Cl | 4,42 | |
| 421 | AB14 | * | 2,4,6-Trifluorphenyl | -CH₃ | 0 | Cl | 4,36 | |
| 422 | 3-Aminopropyl | n-Propyl | Pentafluorphenyl | -CH₃ | 0 | Cl | 1,93 | |
| 423 | 2-Thienylmethyl | n-Propyl | Pentafluorphenyl | -CH₃ | 0 | Cl | 5,05 | |
| 424 | -CH₂-CH₂-NH₂ | -i-Propyl | Pentafluorphenyl | -CH₃ | 0 | Cl | 1,83 | |
| 425 | -CH₂-CH₂-CN | -i-Propyl | Pentafluorphenyl | -CH₃ | 0 | Cl | 3,82 | |
| 426 | -CH₂-COOC₂H₅ | Cyclopropyl | Pentafluorphenyl | -CH₃ | 0 | Cl | 4,13 | |
| 427 | -CH(CF₃)-CH₂-CH₂-CH₂- | * | Pentafluorphenyl | -CH₃ | 0 | Cl | 4,39 | |
| 428 | -CH₂-CH₂-CH₂-CH₂- | * | Pentafluorphenyl | -CH₃ | 0 | Cl | 3,86 | |
| 429 | n-Butyl | -H | Pentafluorphenyl | -CH₃ | 0 | Cl | 4,19 | |
| 430 | -CH₂-C(CH₃)₃ | -H | Pentafluorphenyl | -CH₃ | 0 | Cl | 4,5 | |
| 431 | 2-Butyl | -H | Pentafluorphenyl | -CH₃ | 0 | Cl | 4,16 | |
| 432 | 3,5-bis-Trifluormethyleyclohexyl | -H | Pentafluorphenyl | -CH₃ | 0 | Cl | 4,69 | |
| 433 | Propargyl | -CH₃ | Pentafluorphenyl | -CH₃ | 0 | Cl | 3,71 | |
| 434 | -CH₂-COOC₂H₅ | -CH₃ | Pentafluorphenyl | -CH₃ | 0 | Cl | 3,73 | |
| 435 | Allyl | -CH₃ | Pentafluorphenyl | -CH₃ | 0 | Cl | 4,15 | |
| 436 | -CH₂-CN | -CH₃ | Pentafluorphenyl | -CH₃ | 0 | Cl | 3,23 | |
| 437 | -CH₂-CH₂-Cl | -CH₃ | Pentafluorphenyl | -CH₃ | 0 | Cl | 3,92 | |
| 438 | -CH=C(CH₃)₂ | -C₂H₅ | Pentafluorphenyl | -CH₃ | 0 | Cl | 2,5 | |
| 439 | Allyl | -C₂H₅ | Pentafluorphenyl | -CH₃ | 0 | Cl | 4,46 | |
| 440 | (2-Furyl)methyl | -C₂H₅ | Pentafluorphenyl | -CH₃ | 0 | Cl | 4,48 | |
| 441 | -CH₂-CH₂-CN | -C₂H₅ | Pentafluorphenyl | -CH₃ | 0 | Cl | 3,5 | |
| 442 | -CH₂-COOC₂H₅ | -C₂H₅ | Pentafluorphenyl | -CH₃ | 0 | Cl | 4,07 | |
| 443 | -NH-CH₂-CH₂-CH₂-CH₂- | * | 2,4,6-Trifluorphenyl | -CH₃ | 0 | Cl | 3,45 | 175-7 |
| 444 | -CH₂-CH₂-CH(CH₃)CH₂-CH₂- | * | 2-Chlorphenyl | -CH₃ | 0 | Cl | 4,69 | |
| 445 | 2,2,2-Trifluor-1-methylethyl | -H | 2,6-Difluorphenyl | -CH₃ | 0 | Cl | 3,44 | |
| 446 | -CH₂-COOCH₃ | -CH₃ | 2,6-Difluorphenyl | -CH₃ | 0 | Cl | 2,8 | |
| 447 | 1,3-Dioxolan-2-ylmethyl | -CH₃ | 2,6-Difluorphenyl | -CH₃ | 0 | Cl | 3,1 | |
| 448 | -CH₂-CH₂-N(CH₃)₂ | -CH₃ | 2,6-Difluorphenyl | -CH₃ | 0 | Cl | 1,43 | |
| 449 | 3-(Dimethylamino)-propyl | -CH₃ | 2,6-Difluorphenyl | -CH₃ | 0 | Cl | 2,81 | |
| 450 | -CH₂-COOC₂H₅ | -CH₃ | 2,6-Difluorphenyl | -CH₃ | 0 | Cl | 3,14 | |
| 451 | -CH₂-CH₂-Cl | -CH₃ | 2,6-Difluorphenyl | -CH₃ | 0 | Cl | 3,44 | |
| 452 | -CH₂-C(CH₃)=CH₂ | -C₂H₅ | 2,6-Difluorphenyl | -CH₃ | 0 | Cl | 4,27 | |
| 453 | 2-Methoxyethyl | n-Propyl | 2,6-Difluorphenyl | -CH₃ | 0 | Cl | 3,78 | |
| 454 | -CH₂-CH₂-NH₂ | -i-Propyl | 2,6-Difluorphenyl | -CH₃ | 0 | Cl | 1,47 | |
| 455 | -CH₂-CH(OH)-CH₂-CH₂- | * | 2,6-Difluorphenyl | -CH₃ | 0 | Cl | 2,17 | |
| 456 | -CH₂-CH₂-CH₂-CH(CH₃) | * | 2,6-Difluorphenyl | -CH₃ | 0 | Cl | 3,63 | |
| 457 | AB8 | * | 2,6-Difluorphenyl | -CH₃ | 0 | Cl | 2,03 | |
| 458 | -CH(CF₃)-CH₂-CH₂-CH₂- | * | 2,6-Difluorphenyl | -CH₃ | 0 | Cl | 3,78 | |
| 459 | AB9 | * | 2,6-Difluorphenyl | -CH₃ | 0 | Cl | 1,42 | |
| 460 | -CH₂-CH₂-CH=CH-CH₂- | * | 2,6-Difluorphenyl | -CH₃ | 0 | Cl | 3,6 | |
| 461 | AB10 | * | 2,6-Difluorphenyl | -CH₃ | 0 | Cl | 3,19 | |
| 462 | -CH₂-CH(CH₃)CH₂-CH(CH₃)-CH₂- | * | 2,6-Difluorphenyl | -CH₃ | 0 | Cl | 4,69 | |
| 463 | -CH₂-CH₂-CH₂-CH₂-CH(CH₃) | * | 2,6-Difluorphenyl | -CH₃ | 0 | Cl | 4,2 | |
| 464 | -CH₂-CH₂-CH₂-CH(CH₃)-CH₂- | * | 2,6-Difluorphenyl | -CH₃ | 0 | Cl | 4,2 | |
| 465 | -CH₂-CH₂-CH(CH₃)-CH₂-CH₂- | * | 2,6-Difluorphenyl | -CH₃ | 0 | Cl | 4,27 | |
| 466 | -CH₂-CH(OH)-CH₂-CH₂-CH₂- | * | 2,6-Difluorphenyl | -CH₃ | 0 | Cl | 2,49 | |
| 467 | -CH₂-CH₂-C(CH₃)₂-CH₂-CH₂- | * | 2,6-Difluorphenyl | -CH₃ | 0 | Cl | 4,52 | |
| 468 | -CH₂-CH₂-CH(COCH₃)-CH₂-CH₂- | * | 2,6-Difluorphenyl | -CH₃ | 0 | Cl | 2,92 | |
| 469 | -CH₂-CH=C(C₂H₅)-CH₂-CH₂- | * | 2,6-Difluorphenyl | -CH₃ | 0 | Cl | 4,44 | |
| 470 | -CH₂-CH₂-CH=C(CH₃)-CH₂- | * | 2,6-Difluorphenyl | -CH₃ | 0 | Cl | 4,02 | |
| 471 | -CH(CH₂Cl)-CH₂-CH₂-CH₂-CH(CHCl)- | * | 2,6-Difluorphenyl | -CH₃ | 0 | Cl | 3,23 | |
| 472 | -CH₂-CH₂-CH(COOCH₃)-CH₂-CH₂- | * | 2,6-Difluorphenyl | -CH₃ | 0 | Cl | 3,25 | |
| 473 | -CH₂-CH₂-CHBr-CH₂-CH₂- | * | 2,6-Difluorphenyl | -CH₃ | 0 | Cl | 3,91 | |
| 474 | -CH(COOCH₃)-CH₂-CH₂-CH₂-CH₂- | * | 2,6-Difluorphenyl | -CH₃ | 0 | Cl | 3,56 | |
| 475 | AB12 | * | 2,6-Difluorphenyl | -CH₃ | 0 | Cl | 3,71 | |
| 476 | -CH₂-CH₂-CH(NH-COCH₃)-CH₂-CH₂- | * | 2,6-Difluorphenyl | -CH₃ | 0 | Cl | 2,18 | |
| 477 | -CH₂-CH₂-N(CH₃)-CH₂-CH₂- | * | 2,6-Difluorphenyl | -CH₃ | 0 | Cl | 1,4 | |
| 478 | AB14 | * | 2,6-Difluorphenyl | -CH₃ | 0 | Cl | 4,13 | |
| 479 | -CH₂-CH₂-CH₂-CH(CH₃)- | * | 2,4-Difluorphenyl | -CH₃ | 0 | Cl | 3,75 | |
| 480 | AB9 | * | 2,4-Difluorphenyl | -CH₃ | 0 | Cl | 1,47 | |
| 481 | -CH₂-CH₂-CH(OH)-CH₂-CH₂- | * | 2,4-Difluorphenyl | -CH₃ | 0 | Cl | 2,32 | |
| 482 | -CH₂-CH₂-CH₂-CH₂-CH(CH₃)- | * | 2,4-Difluorphenyl | -CH₃ | 0 | Cl | 4,26 | |
| 483 | -CH₂-CH₂-CH₂-CH(CH₃)-CH₂- | * | 2,4-Difluorphenyl | -CH₃ | 0 | Cl | 4,38 | |
| 484 | -CH₂-CH₂-C(CH₃)₂-CH₂-CH₂- | * | 2,4-DiBuorphenyl | -CH₃ | 0 | Cl | 4,6 | |
| 485 | -CH₂-CH₂-CH(COCH₃)-CH₂-CH₂- | * | 2,4-Difluorphenyl | -CH₃ | 0 | Cl | 3,02 | |
| 486 | -CH₂-CH=C(C₂H₅)-CH₂-CH₂- | * | 2,4-Difluorphenyl | -CH₃ | 0 | Cl | 4,45 | |
| 487 | -CH₂-CH₂-CH=C(CH₃)-CH₂- | * | 2,4-Difluorphenyl | -CH₃ | 0 | Cl | 4,09 | |
| 488 | -CH₂-CH₂-CH(COOCH₃)-CH₂-CH₂- | * | 2,4-Difluorphenyl | -CH₃ | 0 | Cl | 3,32 | |
| 489 | -CH₂-CH₂-CH(CF₃)-CH₂-CH₂- | * | 2,4-Difluorphenyl | -CH₃ | 0 | Cl | 3,99 | |
| 490 | -CH₂-CH₂-N(CH₃)-CH₂-CH₂- | * | 2,4-Difluorphenyl | -CH₃ | 0 | Cl | 1,42 | |
| 491 | -CH₂-CH₂-CH₂-CH₂-CH₂- | * | 2,4-Difluorphenyl | -CH₃ | 0 | Cl | 3,97 | |
| 492 | -CH₂-CH₂-S-CH₂-CH₂- | * | 2,4-Difluorphenyl | -CH₃ | 0 | Cl | 3,53 | |
| 493 | -CH(CH₃)-CH₂-CH(CH₃)₂ | -H | 2,4-Difluorphenyl | -CH₃ | 0 | Cl | 4,19 | |
| 494 | -CH(CF₃)-CH₂-CH₂-CH₂- | * | 2,4-Difluorphenyl | -CH₃ | 0 | Cl | 4,12 | |
| 495 | -CH₂-CH₂-CH₂-CH₂- | * | 2,4-Difluorphenyl | -CH₃ | 0 | Cl | 3,45 | |
| 496 | -CH₂-CH₂-CH=CH-CH₂- | * | 2,4-Difluorphenyl | -CH₃ | 0 | Cl | 3,77 | |
| 497 | AB10 | * | 2,4-Difluorphenyl | -CH₃ | 0 | Cl | 3,33 | |
| 498 | -CH₂-CH(CH₃)-CH₂-CH(CH₃)-CH₂- | * | 2,4-Difluorphenyl | -CH₃ | 0 | Cl | 4,88 | |
| 499 | -CH₂-CH₂-CN(CH₃)-CH₂-CH₂- | * | 2,4-Difluorphenyl | -CH₃ | 0 | Cl | 4,46 | |
| 500 | -CH(CH₃)-CH₂-CH₂-CH₂-CH(CH₃)- | * | 2,4-Difluorphenyl | -CH₃ | 0 | Cl | 3,37 | |
| 501 | -CH₂-CH(OH)-CH₂-CH₂-CH₂- | * | 2,4-Difluorphenyl | -CH₃ | 0 | Cl | 2,65 | |
| 502 | -CH₂-CH₂-CHF-CH₂-CH₂- | * | 2,4-Difluorphenyl | -CH₃ | 0 | Cl | 3,51 | |
| 503 | -CH₂-CH(CH₃)-O-CH(CH₃)-CH₂- | * | 2,4-Difluorphenyl | -CH₃ | 0 | Cl | 3,63 | |
| 504 | -CH₂-CH₂-O-CH₂-CH₂- | * | 2,4-Difluorphenyl | -CH₃ | 0 | Cl | 2,93 | |
| 505 | i-Butyl | -H | 2,4-Difluorphenyl | -CH₃ | 0 | Cl | 3,67 | |
| 506 | -CH₂-C(CH₃)₃ | -H | 2,4-Difluorphenyl | -CH₃ | 0 | Cl | 4,07 | |
| 507 | 2-Butyl | -H | 2,4-Difluorphenyl | -CH₃ | 0 | Cl | 3,69 | |
| 508 | -CH₂-CH₂-CF₃ | -H | 2,4-Difluorphenyl | -CH₃ | 0 | Cl | 3,33 | |
| 509 | Cyclopentyl | -H | 2,4-Difluorphenyl | -CH₃ | 0 | Cl | 3,88 | |
| 510 | -i-Propyl | -H | 2,4-Difluorphenyl | -CH₃ | 0 | Cl | 3,36 | |
| 511 | Cyclohexyl | -H | 2,4-Difluorphenyl | -CH₃ | 0 | Cl | 4,16 | |
| 512 | 1-Cyclohexylethyl | -H | 2,4-Difluorphenyl | -CH₃ | 0 | Cl | 4,9 | |
| 513 | Cyclopropyl | -H | 2,4-Difluorphenyl | -CH₃ | 0 | Cl | 3,1 | |
| 514 | -CH₂-CF₃ | -H | 2,4-Difluorphenyl | -CH₃ | 0 | Cl | 3,19 | |
| 515 | Cyclopropylmethyl | -H | 2,4-Difluorphenyl | -CH₃ | 0 | Cl | 3,42 | |
| 516 | -CH₂-C(CH₃)=CH₂ | -H | 2,4-Difluorphenyl | -CH₃ | 0 | Cl | 3,34 | |
| 517 | 3-Trifluormethylcyclohexyl | -H | 2,4-Difluorphenyl | -CH₃ | 0 | Cl | 4,14 | |
| 518 | 2-Trifluormethylcyclohexyl | -H | 2,4-Difluorphenyl | -CH₃ | 0 | Cl | 4,18 | |
| 519 | 4-Trifluormethylcyclohexyl | -H | 2,4-Difluorphenyl | -CH₃ | 0 | Cl | 4,11 | |
| 520 | -CH₂-CH₂-N(CH₃)₂ | -CH₃ | 2,4-Difluorphenyl | -CH₃ | 0 | Cl | 1,58 | |
| 521 | Propargyl | -CH₃ | 2,4-Difluorphenyl | -CH₃ | 0 | Cl | 3,29 | |
| 522 | -CH₂-COOC₂H₅ | -CH₃ | 2,4-Difluorphenyl | -CH₃ | 0 | Cl | 3,32 | |
| 523 | 1,3-Dioxolan-2-ylmethyl | -CH₃ | 2,4-Difluorphenyl | -CH₃ | 0 | Cl | 3,23 | |
| 524 | Allyl | -CH₃ | 2,4-Difluorphenyl | -CH₃ | 0 | Cl | 3,75 | |
| 525 | -CH₂-CH₂-CN | -CH₃ | 2,4-Difluorphenyl | -CH₃ | 0 | Cl | 2,84 | |
| 526 | -CH₂-CN | -CH₃ | 2,4-Difluorphenyl | -CH₃ | 0 | Cl | 2,84 | |
| 527 | (2-Furyl)methyl | -CH₃ | 2,4-Difluorphenyl | -CH₃ | 0 | Cl | 3,78 | |
| 528 | i-Butyl | -CH₃ | 2,4-Difluorphenyl | -CH₃ | 0 | Cl | 4,27 | |
| 529 | -CH₂-CH₂-Cl | -CH₃ | 2,4-Difluorphenyl | -CH₃ | 0 | Cl | 3,56 | |
| 530 | 2-Methoxyethyl | -CH₃ | 2,4-Difluorphenyl | -CH₃ | 0 | Cl | 3,27 | |
| 531 | -CH₂-C(CH₃)=CH₂ | -CH₃ | 2,4-Difluorphenyl | -CH₃ | | 0 Cl | 4,12 | |
| 532 | -CH₂-CH₂-N(CH₃)₂ | -C₂H₅ | 2,4-Difluorphenyl | -CH₃ | | 0 Cl | 1,71 | |
| 533 | Allyl | -C₂H₅ | 2,4-Difluorphenyl | -CH₃ | 0 | Cl | 4,09 | |
| 534 | (2-Tetrahydrofuryl)methyl | -C₂H₅ | 2,4-Difluorphenyl | -CH₃ | 0 | Cl | 3,95 | |
| 535 | -CH₂-CH₂-CN | -C₂H₅ | 2,4-Difluorphenyl | -CH₃ | | 0 Cl | 3,12 | |
| 536 | 2-Methoxyethyl | -C₂H₅ | 2,4-Difluorphenyl | -CH₃ | 0 | Cl | 3,57 | |
| 537 | -CH₂-COOC₂H₅ | -C₂H₅ | 2,4-Difluorphenyl | -CH₃ | 0 | Cl | 3,62 | |
| 538 | Cyclopropylmethyl | n-Propyl | 2,4-Difluorphenyl | -CH₃ | 0 | Cl | 4,65 | |
| 539 | (2-Tetrahydrofuryl)methyl | n-Propyl | 2,4-Difluorphenyl | -CH₃ | 0 | Cl | 4,32 | |
| 540 | 2-Methoxyethyl | n-Propyl | 2,4-Difluorphenyl | -CH₃ | 0 | Cl | 3,99 | |
| 541 | -CH₂-COOC₂H₅ | Cyclopropyl | 2,4-Difluorphenyl | -CH₃ | 0 | Cl | 3,87 | |
| 542 | -CH₂-CH(OH)-CH₂-CH₂- | * | 2,4-Difluorphenyl | -CH₃ | 0 | Cl | 2,33 | |
| 543 | 1,2-Dimethylpropyl | -H | 2,6-Difluorphenyl | -CH₃ | 0 | Cl | 3,83 | |
| 544 | 1,2-Dimethylpropyl | -H | 2-Fluorphenyl | -CH₃ | 0 | Cl | 3,83 | |
| 545 | 2-Thienylmethyl | n-Propyl | 2,6-Difluorphenyl | -CH₃ | 0 | Cl | 4,61 | |
| 546 | -CH₂-COOC₂H₅ | Cyclopropyl | 2,6-Difluorphenyl | -CH₃ | 0 | Cl | 3,58 | |
| 547 | -CH₂-CH(NH₂)-CH₂-CH₂- | * | 2,6-Difluorphenyl | -CH₃ | 0 | Cl | 1,39 | |
| 548 | -CH₂-CH₂-CHF-CH₂-CH₂- | * | 2,6-Difluorphenyl | -CH₃ | 0 | Cl | 3,45 | |
| 549 | -CH₂-CH₂-CO-CH₂-CH₂- | * | 2,6-Difluorphenyl | -CH₃ | 0 | Cl | 2,68 | |
| 550 | -CH₂-CH₂-CH=CH-CH₂- | * | Pentafluorphenyl | -CH₃ | 0 | Cl | 4,2 | |
| 551 | AB10 | * | Pentafluorphenyl | -CH₃ | 0 | Cl | 3,86 | |
| 552 | -CH₂-CH(CH₃)-CH₂-CH(CH₃)-CH₂- | * | Pentafluorphenyl | -CH₃ | 0 | Cl | 5,31 | |
| 553 | -CH₂-CH₂-CH₂-CH₂-CH(CH₃)- | * | Pentafluorphenyl | -CH₃ | 0 | Cl | 4,83 | |
| 554 | -CH₂-CH₂-CH₂-CH(CH₃)-CH₂- | * | Pentafluorphenyl | -CH₃ | 0 | Cl | 4,89 | |
| 555 | -CH₂-CH₂-CH(CH₃)-CH₂-CH₂- | * | Pentafluorphenyl | -CH₃ | 0 | Cl | 4,88 | |
| 556 | -CH₂-CH(OH)-CH₂-CH₂-CH₂- | * | Pentafluorphenyl | -CH₃ | 0 | Cl | 3,14 | |
| 557 | -CH₂-CH₂-C(CH₃)₂-CH₂-CH₂- | * | Pentafluorphenyl | -CH₃ | 0 | Cl | 5,13 | |
| 558 | -CH₂-CH₂-CH(COCH₃)-CH₂-CH₂- | * | Pentafluorphenyl | -CH₃ | 0 | Cl | 3,6 | |
| 559 | -CH₂-CH=C(C₂H₅)-CH₂-CH₂- | * | Pentafluorphenyl | -CH₃ | 0 | Cl | 5 | |
| 560 | -CH₂-CH₂-CH=C(CH₃)-CH₂- | * | Pentafluorphenyl | -CH₃ | 0 | Cl | 4,6 | |
| 561 | -CH₂-CH₂-CH(COOCH₃)-CH₂-CH₂- | * | Pentafluorphenyl | -CH₃ | 0 | Cl | 3,89 | |
| 562 | -CH₂-CH₂-CHBr-CH₂-CH₂- | * | Pentafluorphenyl | -CH₃ | 0 | Cl | 4,5 | |
| 563 | -CH₂-CH₂-CF₂-CH₂-CH₂. | * | Pentafluorphenyl | -CH₃ | | 0 Cl | 4,12 | |
| 564 | AB12 | * | Pentafluorphenyl | -CH₃ | 0 | Cl | 4,36 | |
| 565 | AB11 | * | Pentafluorphenyl | -CH₃ | 0 | Cl | 1,91 | |
| 566 | -CH₂-CH₂-CH(NH-COCH₃)-CH₂-CH₂- | * | Pentafluorphenyl | -CH₃ | 0 | Cl | 2,79 | |
| 567 | AB14 | * | Pentafluorphenyl | -CH₃ | 0 | Cl | 4,67 | |

| | | | | | | | | |
|---|---|---|---|---|---|---|---|---|
| ** Die Bestimmung der logP-Werte erfolgte gemäß EEC-Directive 79/831 Annex V. A8 durch HPLC (Gradientenmethode, Acetonitril/0,1 % wässrige Phosphorsäure) * R¹ und R² bilden gemeinsam mit dem Stickstoffatom, an das sie gebunden sind, einen heterocyclischen Ring. *** Die Abkürzungen AB2, AB8, AB9 AB10, AB11, AB12, AB14 und AB29 haben die folgenden Bedeutungen: | | | | | | | | |

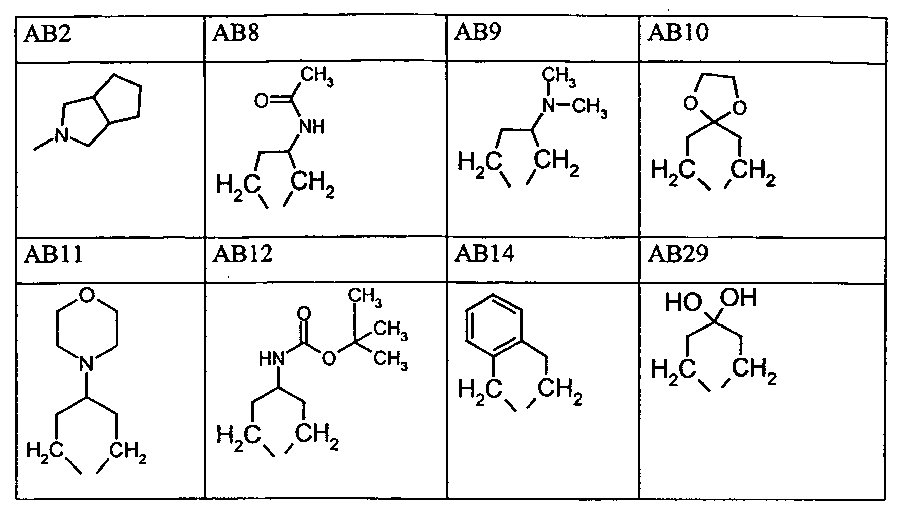

### Herstellung von Ausgangsstoffen

### Beispiel 568

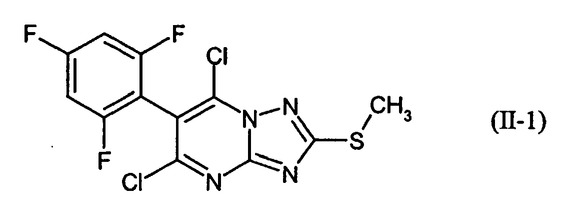

31,3 g (95,35 mMol) 2-Methylsulfanyl-6-(2,4,6-trifluor-phenyl)-[1,2,4]triazolo[1,5-a]pyrimidin-5,7-diol werden bei Raumtemperatur in 140 ml Phosphoroxychlorid gelöst, portionsweise mit 14,1 g Phosphorpentachlorid versetzt und anschließend 16 Stunden unter Rückfluss erhitzt. Flüchtige Bestandteile der Reaktionsmischung unter vermindertem Druck abdestilliert. Der Rückstand wird mit 200 ml Wasser versetzt und mit 100 ml Dichlormethan extrahiert. Die organische Phase wird über Natriumsulfat getrocknet und an Kieselgel zunächst mit Essigsäureethylester/n-Hexan (1:3) und zuletzt mit reinem Essigsäureethylester chromatografiert. Man erhält 18 g (50 % der Theorie) 5,7-Dichlor-2-(methylsulfanyl)-6-(2,4,6-trifluorphenyl)[1,2,4]triazolo[1,5-a]pyrimidin.
HPLC: logP = 3,43

Nach der zuvor angegebenen Methode werden auch die in der folgenden Tabelle 2 aufgeführten Dihalogen-triazolopyrimidine der Formel (II) erhalten.

**Tabelle 2**

| | | | | | | | |
|---|---|---|---|---|---|---|---|
| 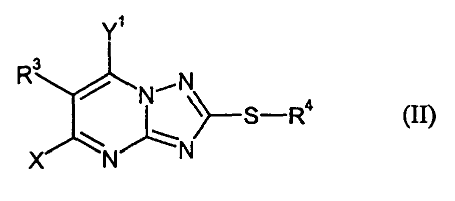 | | | | | | | |

| Bsp.Nr. | Verb. | R³ | R⁴ | X | Y¹ | logP | Fp. (°C) |
|---|---|---|---|---|---|---|---|
| 569 | II-2 | 2-Chlorphenyl | -CH3 | -Cl | -Cl | | 159-164 |
| 570 | II-3 | 2-Chlorphenyl | -i-Propyl | -Cl | -Cl | | |
| 571 | II-4 | 2-Chlorphenyl | 4-Chlorbenzyl | -Cl | -Cl | | |
| 572 | II-5 | 2,6-Dichlorphenyl | -CH₃ | -Cl | -Cl | 3,86 | |

### Beispiel 573

### Verfahren (f), erste Stufe

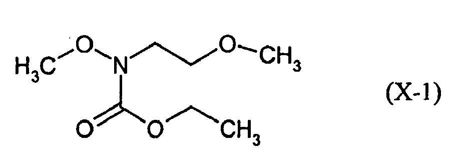

1000 mg N-Methoxy-carbaminsäureethylester werden in 10,0 ml Dimethylformamid vorgelegt und portionsweise mit 403 mg Natriumhydrid versetzt, wobei die Temperatur durch Kühlung auf 30°C eingestellt wurde. Die Reaktionsmischung wird für 2 Stunden bei 30°C gerührt und anschließend mit 3500 mg 2-Bromethyl-methylether versetzt. Die Reaktionsmischung wird für 18 Stunden bei 20°C bis 25°C gerührt und anschließend in 20 ml Wasser eingerührt. Die erhaltene Reaktionsmischung wird unter vermindertem Druck zur Trockne eingeengt und viermal mit je 30 ml Dichlormethan extrahiert. Die organische Extrakte werden über Natriumsulfat getrocknet, filtriert und unter vermindertem Druck zu Trockne eingeengt.

Man erhält 1200 mg (N-Methoxy-N-methoxyethylcarbaminsäure-ethylester (Reinheit 77,6 %, Ausbeute 62,6 %).

### Verfahren (f), zweite Stufe:

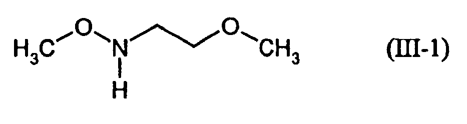

200 mg (N-Methoxy-N-methoxyethyl)-carbaminsäureethylester werden in 4,0 ml wässrigem Ethanol (59 %ig) vorgelegt, mit 240,6 mg Kaliumhydroxid versetzt und für 18 Stunden bei 40°C gerührt. Die Reaktionsmischung wird dann in 50 ml Wasser eingerührt, dreimal mit je 20 ml Diethylether und dreimal mit je 20 ml Dichlormethan extrahiert. Die vereinigten organischen Phasen werden zweimal mit je 20 ml Wasser gewaschden, getrocknet und bei 20°C unter vermindertem Druck auf ein Volumen von 20 ml eingeengt.

Die erhaltene Lösung wird unter Eiskühlung mit 2 ml Salzsäure versetzt, 1 Stunde bei Raumtemperatur gerührt und bei 20°C unter vermindertem Druck zur Trockne eingeengt.

Man erhält 140 mg N-Methoxy-N-methoxyethylamin-hydrochlorid (Ausbeute 87,6 %).

### Beispiel 574

### Verfahren (g), erste Stufe:

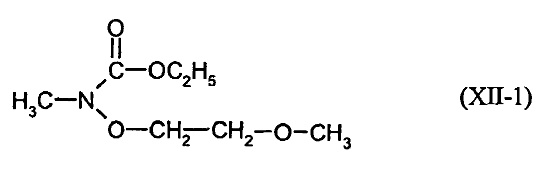

Ein Gemisch aus 1000 mg N-Hydroxy-N-methyl-carbaminsäure-ethylester, 1166 mg 2-Bromethyl-methylether und 10 ml Ethanol wird unter Rühren auf Rückflusstemperatur erhitzt und dann tropfenweise mit einer Lösung von 493 mg Kaliumhydroxid in 5 ml Ethanol versetzt. Man kocht das Reaktionsgemisch 10 Stunden unter Rückfluss und arbeitet dann auf, indem man das Reaktionsgemisch filtriert und das Filtrat unter vermindertem Druck einengt. Der verbleibende Rückstand wird mit einem Gemisch aus Wasser und Essigsäureethylester versetzt. Die organische Phase wird abgetrennt, mit gesättigter, wässriger Ammoniumchlorid-Lösung und dann mit Wasser gewaschen. Anschließend wird die organische Phase über Natriumsulfat getrocknet und unter vermindertem Druck eingeengt. Man erhält auf diese Weise 0,7 g eines Produktes, das gemäß Gaschromatogramm zu 83 % aus (N-Methyl-N-methoxyethoxy)-carbaminsäure-ethylester besteht. Die Ausbeute errechnet sich danach zu 39 % der Theorie.

### Verfahren (g), zweite Stufe:

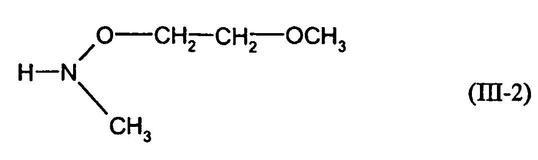

Ein Gemisch aus 200 mg (N-Methyl-N-methoxyethoxy)-carbaminsäure-ethylester, 4 ml Ethanol und 4 ml Wasser wird mit 240,6 mg pulverisiertem Kaliumhydroxid versetzt und 42 Stunden bei 40°C gerührt. Das Reaktionsgemisch wird danach in 50 ml Wasser eingerührt, dann dreimal mit je 20 ml Diethylether und anschließend dreimal mit je 20 ml Methylenchlorid extrahiert. Die vereinigten organischen Phasen werden zweimal mit je 20 ml Wasser gewaschen, über Natriumsulfat getrocknet und bei Raumtemperatur unter vermindertem Druck auf ein Volumen von 20 ml eingeengt. Die erhaltene Lösung wird unter Eiskühlung mit 1 ml etherischer Salzsäure versetzt. Die sich abscheidenden Kristalle werden abfiltriert und getrocknet. Man erhält auf diese Weise 190 mg an N-Methyl-N-methoxyethoxyamin-hydrochlorid.

### Beispiel 575

### Verfahren (h), erste Stufe:

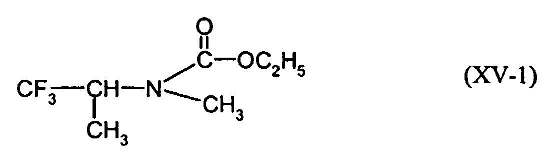

Ein Gemisch aus 2000 mg N-(2,2,2-Trifluor-1-methyl-ethyl)-carbaminsäure-ethylester und 20 ml Tetrahydrofuran wird bei Raumtemperatur unter Rühren mit 475 mg Natriumhydroxid versetzt. Danach wird unter Rühren bei Raumtemperatur eine Lösung von 4600 mg Iodmethan in 10 ml Tetrahydrofuran hinzugetropft. Das Reaktionsgemisch wird 16 Stunden bei 50°C gerührt und dann mit Wasser versetzt. Man extrahiert dreimal mit je 20 ml Methylenchlorid, trocknet die vereinigten organischen Phasen über Natriumsulfat und engt unter vermindertem Druck ein. Man erhält 1000 mg eines Produktes, das gemäß Gaschromatogramm zu 75 % aus N-(2,2,2-Trifluor-1-methyl-ethyl)-N-methyl-carbaminsäure-ethylester besteht. Die Ausbeute errechnet sich danach zu 34,86 %.

### Verfahren (h), zweite Stufe:

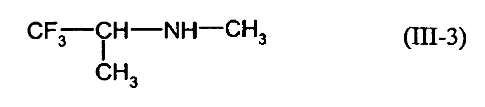

Ein Gemisch aus 1000 mg N-(2,2,2-Trifluor-1-methyl-ethyl)-N-methyl-carbaminsäure-ethylester, 20 ml Ethanol und 20 ml Wasser wird mit 1070 mg pulverisierten Kaliumhydroxid versetzt und 66 Stunden bei 40°C gerührt. Danach wird das Reaktionsgemisch mit Wasser verdünnt und dreimal mit je 20 ml eines Gemisches extrahiert, das zu gleichen Teilen aus Methylenchlorid und Diethylether besteht. Die vereinigten organischen Phasen werden über Natriumsulfat getrocknet und dann bei Raumtemperatur unter leicht vermindertem Druck eingeengt. Die erhaltene Lösung wird unter Eiskühlung mit etherischer Salzsäure versetzt und 60 Stunden bei Raumtemperatur gerührt. Nach dem Einengen unter vermindertem Druck erhält man 280 mg an N-(2,2,2-Trifluor-1-methyl-ethyl)-N-methylamin-hydrochlorid. Die Ausbeute errechnet sich danach zu 34 % der Theorie.

### Beispiel 576

### Verfahren (i):

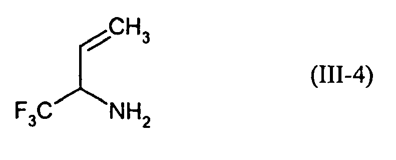

600 mg N-(1-Trifluormethyl-2-propen)-carbaminsäurebenzylester werden in 8,0 ml 16 %iger Salzsäure für 1,5 Stunden unter Rückfluss erhitzt. Nach dem Abkühlen auf 20°C wird zweimal mit je 20 ml Diethylether extrahiert.

Die verbleibende wässrige Phase wird unter vermindertem Druck zur Trockne eingeengt und dreimal mit je 10 ml Methanol versetzt. Nach Entfernen des Methanols unter vermindertem Druck werden 310 mg an (1-Trifluormethyl-prop-2-en)-amin-hydrochlorid isoliert. Die Ausbeute errechnet sich danach zu 82,9 % der Theorie.

Nach dem zuvor angegebenen Methoden lassen sich auch die in den folgenden Tabellen angegebenen Carbamate herstellen.

**Tabelle 3**

| | | | |
|---|---|---|---|
| 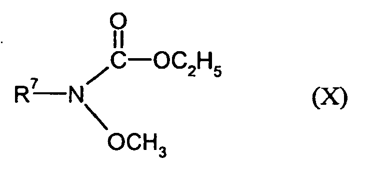 | | | |

| **Beispiel-Nr.** | **Verb.-Nr.** | **R**^{**7**} | **logP** |
|---|---|---|---|
| 577 | X-2 | 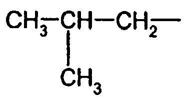 | 2,38 |
| 578 | X-3 | 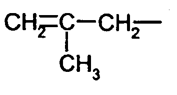 | 2,06 |

**Tabelle 4**

| | | | |
|---|---|---|---|
| 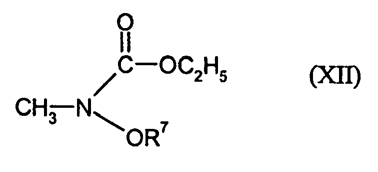 | | | |

| **Beispiel-Nr.** | **Verb.-Nr.** | **R**^{**7**} | **Physikalische Konst.** |
|---|---|---|---|
| 579 | XII-22 | 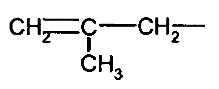 | |

**Tabelle 5**

| | | | |
|---|---|---|---|
| 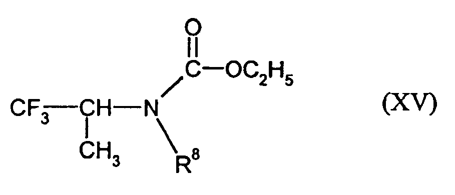 | | | |

| **Beispiel-Nr.** | **Verb.-Nr.** | **R**^{**8**} | **Physikalische Konst.** |
|---|---|---|---|
| 580 | XV-2 | -C₂H₅ | ¹H-NMR (400 MHz, CD₃CN): δ (ppm) = 1.13 (t, CH₃CH₂N), 1,21 (t, CH₃CHCF₃), 1,23 (t, CH₃CH₂O), 3,20 (m, CH₂N, CHCF₃), 4,1 (q, CH₃CH₂O). |

Nach den zuvor angegebenen Methoden lassen sich auch die in der folgenden Tabelle aufgeführten Amine herstellen.

**Tabelle 6**

| | | | | |
|---|---|---|---|---|
| 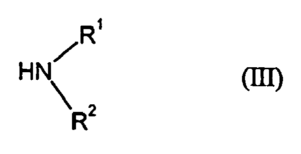 | | | | |

| **Bsp.-Nr.** | **Verb.-Nr.** | **R**^{**1**} | **R**^{**2**} | **Physikal. Konst.** |
|---|---|---|---|---|
| 581 | III-5 | 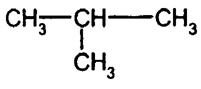 | -OCH₃ | ¹H-NMR (400 MHz, CD₃CN): δ (ppm) = 1,03 (d, (CH₃)₂CH), 3,06 (d, CH₂), 3,28 (b, (CH₃)₂CH, 4,01 (s, OCH₃) |
| 582 | III-6 | 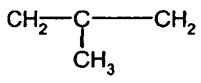 | -OCH₃ | ¹H-NMR (400 MHz, DMSO): δ (ppm) = 1,76 (s, CH₃-(CCH₂)CH₂), 3,29 (b, NH, CH₃(CCH₂)CH₂, OCH₃), 7,89, 5,02 (2 s, CH₃(CCH₂)CH₂). |
| 583 | III-7 | 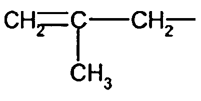 | -CH₃ | |
| 584 | III-8 | 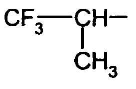 | -C₂H₅ | ¹H-NMR (400 MHz, DMSO): δ (ppm) = 1,06 (m, CH₃CH₂N, CH₃CHCF₃), 3,20 (m, CH₂N), 4,1 (m, CHCF₃) |

Die in den Beispielen 581-584 aufgeführten Amine wurden jeweils in Form ihrer Hydrochloride isoliert und charakterisiert.

### Beispiel 585

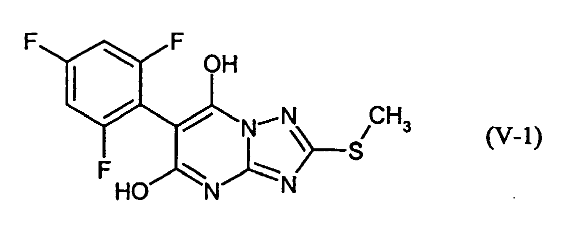

5 g (19,1 mMol) 2-(2,4,6-trifluor-phenyl)malonsäuredimethylester und 2,5 g (19,1 mMol) 5-Methylsulfanyl-1H-[1,2,4]triazol-3-ylamin werden in 5 ml Tri-n-Butylamin 6 Stunden auf 180°C erhitzt, wobei entstehendes Methanol abdestilliert wird. Das Tri-n-Butylamin wird unter stark vermindertem Druck abdestilliert. Man erhält 7,9 g (99,7 % der Theorie) rohes 79 %iges 2-Methylsulfanyl-6-(2,4,6-trifluorphenyl)-[1,2,4]triazolo[1,5-a]pyrimidin-5,7-diol, das ohne Reinigung weiter umgesetzt wird.
HPLC: logP = 0,47

### Verwendungsbeispiele:

### Beispiel A

### Podosphaera-Test (Apfel) / protektiv

| | |
|---|---|
| Lösungsmittel : | 24,5 Gewichtsteile Aceton |
| | 24,5 Gewichtsteile Dimethylacetamid |
| Emulgator : | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Sporensuspension des Apfelmehltauerregers Podosphaera leucotricha inokuliert. Die Pflanzen werden dann im Gewächshaus bei ca. 23°C und einer relativen Luftfeuchtigkeit von ca. 70 % aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen und Versuchsergebnisse gehen aus der folgenden Tabelle hervor:

**Tabelle A**

| Podosphaera-Test (Apfel) / protektiv | | | |
|---|---|---|---|
| Wirkstoff | | Aufwandmenge an Wirkstoff in g/ha | Wirkungsgrad in % |
| Erfindungsgemäß: | | | |
| 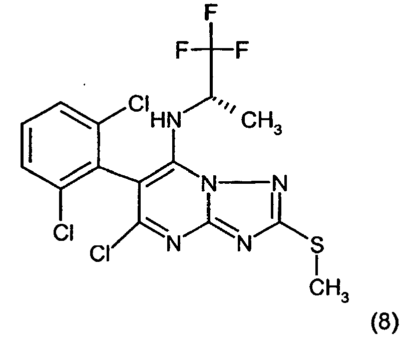 | | 100 | 100 |
| 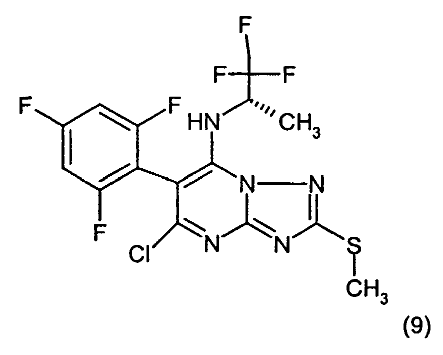 | | 100 | 94 |
| 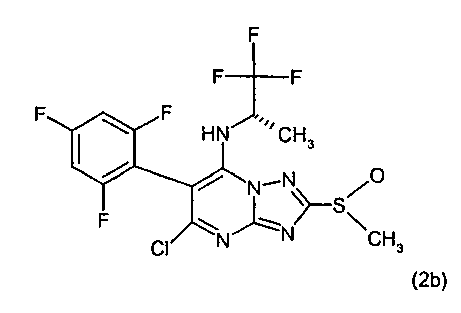 | | 100 | 98 |

### Beispiel B

### Venturia - Test (Apfel) / protektiv

| | |
|---|---|
| Lösungsmittel : | 24,5 Gewichtsteile Aceton |
| | 24,5 Gewichtsteile Dimethylacetamid |
| Emulgator : | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100 % relativer Luftfeuchtigkeit in einer Inkubations-kabine.

Die Pflanzen werden dann im Gewächshaus bei ca. 21°C und einer relativen Luftfeuchtigkeit von ca. 90 % aufgestellt.

12 Tage nach der Inokulation erfolgt die Auswertung. Dabei bedeutet 0 % ein Wirkungsgrad, der demjenigen der Kontrolle entspricht, während ein Wirkungsgrad von 100% bedeutet, dass kein Befall beobachtet wird.

Wirkstoffe, Aufwandmengen und Versuchsergebnisse gehen aus der folgenden Tabelle hervor.

**Tabelle B**

| Venturia-Test (Apfel) / protektiv | | | |
|---|---|---|---|
| Wirkstoff | | Aufwandmenge an Wirkstoff in g/ha | Wirkungsgrad in % |
| Erfindungsgemäß: | | | |
| 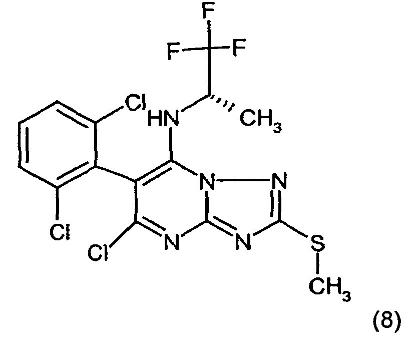 | | 100 | 100 |
| 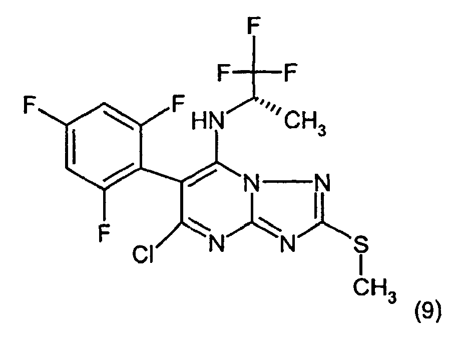 | | 100 | 100 |
| 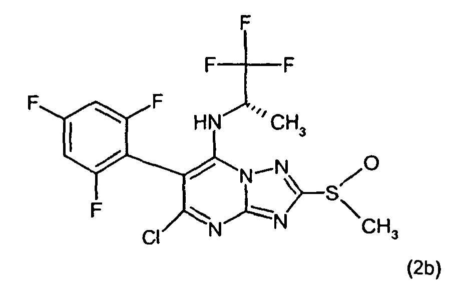 | | 100 | 100 |

### Beispiel C

### In vitro-Test zur Bestimmung der ED₅₀ an Mikroorganismen

| | |
|---|---|
| Lösungsmittel: | 1 ml Methanol |
| Emulgator: | 6 mg ethoxyliertes Tristyrylphenol |

Zur Herstellung einer zweckmäßigen Wirkstoffzubereitung vermischt man 20 µg Wirkstoff mit den oben angegebenen Mengen an Lösungsmittel und Emulgator und verdünnt das Konzentrat mit dem angegebenen Lösungsmittel/Emulgator-Gemisch auf die jeweils gewünschte Konzentration.

Jeweils 10 µl der Zubereitung werden in die Kavitäten von Mikrotiterplatten einpipettiert. Nachdem das Lösungsmittel verdampft ist, werden je Kavität jeweils 200 µl eines Potato-Dextrose Mediums hinzugefügt, das zuvor mit der jeweils gewünschten Konzentration an Sporen bzw. Myzel des zu prüfenden Mikroorganismus versetzt worden war. Die resultierenden Konzentrationen an Wirkstoff in den Kavitäten betragen

| | | |
|---|---|---|
| | 0,1 | ppm |
| | 1 | ppm |
| | 10 | ppm |
| bzw. | 100 | ppm. |

Die resultierende Konzentration an Emulgator beträgt jeweils 300 ppm.

Zur Inkubation werden die Mikrotiterplatten anschließend 3 bis 5 Tage auf einem Schüttler bei einer Temperatur von 22°C bewegt, bis in der unbehandelten Kontrolle ein ausreichendes Wachstum des jeweiligen Mikroorganismus feststellbar ist.

Die Auswertung erfolgt photometrisch bei einer Wellenlänge von 620 nm. Aus den Messdaten für die verschiedenen Konzentrationen wird die Wirkstoffdosis berechnet, die zu einer 50 %igen Hemmung des Pilzwachstums (ED₅₀) gegenüber der unbehandelten Kontrolle führt.

Wirkstoffe und Versuchsergebnisse gehen aus der folgenden Tabelle hervor.

**Tabelle C In vitro-Test zur ED₅₀-Bestimmung bei Mikroorganismen**

| Wirkstoff | Mikroorganismus | ED₅₀-Wert |
|---|---|---|
| Erfindungsgemäß: | | |
| 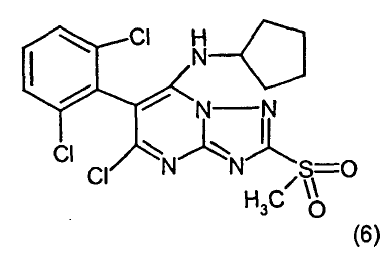 | Pyricularia oryzae | 0,24 |
| 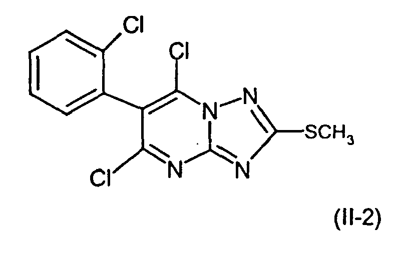 | Pyricularia oryzae | 0,33 |

## Patentansprüche

1. Triazolopyrimidine der Formel (I), 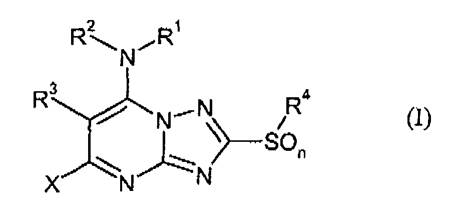 wobei die Symbole und Indizes folgende Bedeutung haben:
R¹ steht für Amino;
für gegebenenfalls durch Halogen, Cycloalkyl, Cyano, Phenyl, Heterocyclyl, Alkoxy mit 1 bis 4 Kohlenstoffatomen, Alkylthio mit 1 bis 4 Kohlenstoff atomen, Oxo, Hydroxoimino und/oder Alkoximino mit 1 bis 4 Kohlenstoff atomen substituiertes Alkyl mit 1 bis 6 Kohlenstoffatomen,
für gegebenenfalls durch Hydroxy, Alkenyloxy mit 2 bis 4 Kohlenstoffatomen, Alkoxycarbonyl mit 1 bis 4 Kohlenstoffatomen substituiertes Alkyl, mit 1 bis 6 Kohlenstoffatomen,
für gegebenenfalls durch Halogen, Cycloalkyl, Cyano, Phenyl und/oder Heterocyclyl substituiertes Alkenyl mit 2 bis 6 Kohlenstoffatomen,
für gegebenenfalls durch Halogen, Cycloalkyl, Cyano, Phenyl und/oder Heterocyclyl substituiertes Alkinyl mit 2 bis 6 Kohlenstoffatomen,
für gegebenenfalls durch Halogen, Cycloalkyl, Cyano, Phenyl und/oder Heterocyclyl substituiertes Cycloalkyl mit 3 bis 7 Kohlenstoffatomen,
für gegebenenfalls durch Halogen, Cyloalkyl, Cyano, Phenyl und/oder Heterocyclyl substituiertes Alkoxy mit 1 bis 7 Kohlenstoffatomen,
für gegebenenfalls durch Halogen, Cyloalkyl, Cyano, Phenyl und/oder Heterocyclyl substituiertes Alkenyloxy mit 2 bis 6 Kohlenstoffatomen,
für gegebenenfalls durch Halogen, Cyloalkyl, Cyano, Phenyl und/oder Heterocyclyl substituiertes Alkinyloxy mit 2 bis 6 Kohlenstoffatomen,
für gegebenenfalls durch Halogen, Cyloalkyl, Cyano, Phenyl und/oder Heterocyclyl substituiertes Cycloalkyloxy mit 3 bis 7 Kohlenstoffatomen,
für gegebenenfalls durch Halogen, Cyloalkyl, Cyano, Phenyl und/oder Heterocyclyl substituiertes Alkylamino mit 1 bis 7 Kohlenstoffatomen,
für gegebenenfalls durch Halogen, Cyloalkyl, Cyano, Phenyl und/oder Heterocyclyl substituiertes Dialkylamino mit 1 bis 7 Kohlenstoffatomen in jedem der Alkylreste,
für gegebenenfalls durch Halogen, Cyloalkyl, Cyano, Phenyl und/oder Heterocyclyl substituiertes Alkenylamino mit 2 bis 6 Kohlenstoffatomen,
für gegebenenfalls durch Halogen, Cyloalkyl, Cyano, Phenyl und/oder Heterocyclyl substituiertes Alkinylamino mit 2 bis 6 Kohlenstoffatomen,
für gegebenenfalls durch Halogen, Cyloalkyl, Cyano, Phenyl und/oder Heterocyclyl substituiertes Cycloalkylamino mit 3 bis 7 Kohlenstoffatomen,
für gegebenenfalls durch Halogen, Cyloalkyl, Cyano, Phenyl und/oder Heterocyclyl substituiertes N-Cycloalkyl-N-alkyl-amino mit 3 bis 7 Kohlenstoffatomen im Cycloalkylteil und 1 bis 7 Kohlenstoffatomen in Alkylteil,
für gegebenenfalls durch Halogen, Cyloalkyl, Cyano, Phenyl und/oder Heterocyclyl substituiertes Alkylidenamino mit 2 bis 6 Kohlenstoffatomen,
für gegebenenfalls durch Halogen, Alkyl, Cycloalkyl, Cyano, Phenyl und/oder Heterocyclyl substituiertes Heterocyclyl mit 5 oder 6 Ringgliedern oder für -S-R⁵, worin
R⁵ für gegebenenfalls durch Halogen, Cycloalkyl, Cyano, Phenyl und/oder Heterocyclyl substituiertes Alkyl mit 1 bis 6 Kohlenstoffatomen,
für gegebenenfalls durch Halogen, Cycloalkyl, Cyano, Phenyl und/oder Heterocyclyl substituiertes Alkenyl mit 2 bis 6 Kohlenstoffatomen,
für gegebenenfalls durch Halogen, Cycloalkyl, Cyano, Phenyl und/oder Heterocyclyl substituiertes Alkinyl mit 2 bis 6 Kohlenstoffatomen oder
für gegebenenfalls durch Halogen, Cycloalkyl, Cyano, Phenyl und/oder Heterocyclyl substituiertes Cycloalkyl mit 3 bis 7 Kohlenstoffatomen steht,
wobei die zuvor genannten Heterocyclyl-Reste einfach bis dreifach, gleichartig oder verschieden substituiert sein können durch Halogen, Alkyl mit 1 bis 4 Kohlenstoffatomen, Halogenalkyl mit 1 oder 2 Kohlenstoffatomen und 1 bis 5 Halogenatomen, Alkoxy mit 1 bis 4 Kohlenstoffatomen, Alkylthio mit 1 bis 4 Kohlenstoffatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen und/oder Phenyl,
und wobei die zuvor genannten Phenyl-Reste einfach bis dreifach, gleichartig oder verschieden substituiert sein können durch
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen,
oder einfach substituiert sein können durch zweifach in ortho-Stellung verknüpftes Alkylen mit 3 oder 4 Kohlenstoffatomen oder Dioxyalkylen mit 1 oder 2 Kohlenstoffatomen, wobei diese Reste einfach bis vierfach, gleichartig oder verschieden substituiert sein können durch Halogen, Alkyl mit 1 bis 4 Kohlenstoffatomen und/oder Halogenalkyl mit 1 bis 4 Kohlenstoffaromen und 1 bis 9 gleichen oder verschiedenen Halogenatomen;
R² steht für Wasserstoff,
für gegebenenfalls durch Halogen, Alkoxy mit 1 bis 4 Kohlenstoffatomen, Alkylthio mit 1 bis 4 Kohlenstoffatomen, Oxo, Hydroximino, Alkoximino mit 1 bis 4 Kohlenstoffatomen und/oder Cycloalkyl mit 3 bis 6 Kohlenstoffatomen substituiertes Alkyl mit 1 bis 4 Kohlenstoffatomen,
für gegebenenfalls durch Halogen und/oder Cycloalkyl mit 3 bis 6 Kohlenstoffatomen substituiertes Alkenyl mit 2 bis 4 Kohlenstoffatomen oder
für gegebenenfalls durch Halogen und/oder Cycloalkyl mit 3 bis 6 Kohlenstoffatomen substituiertes Alkinyl mit 2 bis 4 Kohlenstoffatomen oder
für gegebenenfalls durch Halogen und/oder Alkyl mit 1 bis 4 Kohlenstoffatomen substituiertes Cycloalkyl mit 3 bis 6 Kohlenstoffatomen steht; oder
R¹ und R² stehen gemeinsam mit dem Stickstoffatom, an das sie gebunden sind, für einen 3- bis 6-gliedrigen heterocyclischen Ring, der einfach bis dreifach, gleichartig oder verschieden substituiert sein kann durch
Alkyl mit 1 bis 4 Kohlenstoffatomen,
Halogenalkyl mit 1 bis 4 Kohlenstoffatomen und 1 bis 9 gleichen oder verschiedenen Halogenatomen,
Fluor, Chlor, Brom, Iod, Hydroxy, Cyano, Oxo;
Alkylcarbonyl mit 1 bis 4 Kohlenstoffatomen,
Alkoxycarbonyl mit 1 bis 4 Kohlenstoffatomen,
Aminoalkyl mit 1 bis 4 Kohlenstoffatomen,
Alkylcarbonylamino mit 1 bis 4 Kohlenstoffatomen oder
an dem ein Phenylring anelliert ist;
R³ steht für Phenyl, das einfach bis fünffach, gleichartig oder verschieden substituiert sein kann durch
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen,
oder einfach substituiert sein kann durch zweifach in ortho-Stellung verknüpftes Alkylen mit 3 oder 4 Kohlenstoffatomen oder Dioxyalkylen mit 1 oder 2 Kohlenstoffatomen, wobei diese Reste einfach bis vierfach, gleichartig oder verschieden substituiert sein können durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen;
R⁴ steht für gegebenenfalls durch Halogen und/oder Cyano substituiertes Alkyl mit 1 bis 6 Kohlenstoffatomen,
für gegebenenfalls durch Halogen und/oder Cyano substituiertes Alkenyl mit 3 bis 6 Kohlenstoffatomen,
für gegebenenfalls durch Halogen und/oder Cyano substituiertes Alkinyl mit 3 bis 6 Kohlenstoffatomen oder
für gegebenenfalls durch Halogen substituiertes Aralkyl mit 6 bis 10 Kohlenstoffatomen im Arylteil und 1 bis 4 Kohlenstoffatomen im Arylteil und 1 bis 4 Kohlenstoffatomen im Alkylteil steht;
X steht für Fluor, Chlor oder Brom;
n steht für 0, 1 oder 2.

2. Triazolopyrimidine der Formel (I) gemäß Anspruch 1, wobei die Symbole und Indizes folgende Bedeutung haben:
R¹ steht für Methyl, Ethyl, n-Propyl, i-Propyl, n-, i-, s- oder t-Butyl, Methoxymethyl, 2-Methoxy-ethyl, Methylthio-methyl, 2-Methylthio-methyl, Hydroximino-methyl, Methoximino-methyl, Acetylmethyl, 2-(Hydroximino)-propyl, 2-(Methoximino)propyl,
oder
für 2-Ethoxycarbonyl-ethyl, 2-Vinyloxy-ethyl, 2-Hydroxyimino-ethyl, 2-Methoxyimino-ethyl, 2-Hydroxyethyl, 2-Chlorethyl, 2-Methyl-1-propyl, 1,2-Dimethylpropyl, Thienylmethyl, amino;
oder
für Allyl, 2-Methyl-prop-2-enyl, Propargyl, 2,2,2-Trifluorethyl, 1-(Trifluormethyl)-ethyl, 3,3,3-Trifluorpropyl, Cyclopentyl, Cyclohexyl, Cyclopropylmethyl, Cyclobutylmethyl, Cyclopentylmethyl oder Cyclohexylmethyl, Methoxy, Ethoxy, n- oder i-Propoxy, n-, i-, s- oder t-Butoxy, Allyloxy, Propargyloxy, Cyclopropyloxy, Cyclobutyloxy, Cyclopentyloxy oder Cyclohexyloxy, Difluormethoxy, Trifluormethoxy, Difluorchlormethoxy, Trifluorethoxy, Methylamino, Ethylamino, n- oder i-Propylamino, n-, i-, s- oder t-Butylamino, Dimethylamino, Diethylamino, Trifluorethylamino, Cyclohexylmethylamino, 2-Cyanethylamino, Allylamino, 1-Cyclopropylethylamino, Cyclopropylamino, Cyclobutylamino, Cyclopentylamino, Cyclohexylamino, 1-Methylethylidenamino, Benzyloxy, Piperidinyl, Morpholinyl, Pyridylmethoxy, Thiazolylmethoxy oder für -S-R⁵ steht, worin
R⁵ für Methyl, Ethyl, n- oder i-Propyl, Difluormethyl, Difluorchlormethyl, Dichlorfluormethyl oder Trifluormethyl steht,
wobei die zuvor genannten Thiazolyl- und Pyridyl-Reste im Falle von Thiazolyl einfach oder zweifach und im Falle von Pyridyl einfach bis dreifach, jeweils gleichartig oder verschieden substituiert sein können durch Fluor, Chlor Brom, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n-odcr i-Propylthio, Difluormethoxy, Trifluormethoxy, Difluorchlormethoxy, Trifluorethoxy, Difluormethylthio, Difluorchlormethylthio, Dichlorfluormethylthio, Trifluormethylthio und/oder Phenyl,
und wobei das zuvor genannte Benzyloxy im Phenylteil einfach bis dreifach, gleichartig oder verschieden substituiert sein kann durch
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Methylamino, Ethylamino, n- oder i-Propylamino, Dimethylamino, Diethylamino, Acetyl, Propionyl, Acctyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl,
oder einfach substituiert sein kann durch zweifach in ortho-Stellung verknüpftes Propan-1,3-diyl, Methylendioxy oder Ethylendioxy, wobei diese Reste einfach bis vierfach, gleichartig oder verschieden substituiert sein können durch Fluor, Chlor, Methyl, Ethyl, n-Propyl, i-Propyl und/oder Trifluormethyl;
R² steht für Wasserstoff, Methyl, Ethyl, n- oder 1-Propyl, n-, i-, s- oder t-Butyl, Cyclopropyl, Methoxy-methyl, 2-Methoxy-ethyl, Methylthio-methyl, 2-Methylthio-methyl, Hydroximino-methyl, Methoximino-methyl, Acetylmethyl, 2-Hydroximino-propyl, 2-Methoximino-propyl,
oder
für Allyl, Propargyl, 2,2,2-Trifluorethyl, 1-(1,1,1-Trifluormethyl)ethyl, Cyclopropylmethyl, Cyclobutylmethyl, Cyclopentylmethyl oder Cyclohexylmethyl; oder
R¹ und R² stehen gemeinsam mit dem Stickstoffatom, an das sie gebunden sind,
für gegebenenfalls durch Methyl, Ethyl oder Trifluorethyl substituiertes Pyrrolyl, Piperidinyl, Morpholinyl oder Piperazinyl,
für einfach bis dreifach durch Fluor, Chlor, Brom, Hydroxy, Oxo, Methoxy, Amino, Methylamino, Dimethylamino, Acetylamino, Chlormethyl, Dichlormethyl, Fluormethyl, Trifluormethyl, Aminomethyl, Methoxycarbonyl, Methylcarbonyl, Morpholinyl, 1,3-Dioxolanyl, tert.-Butoxycarbonylamino substituiertes Piperidinyl,
für durch Phenyl anelliertes Piperidinyl,
für unsubstituiertes oder einfach bis dreifach durch Hydroxy, Dimethylaminom, Acetylamino, 1,3-Propandiyl substituiertes Pyrrolidinyl,
für unsubstituiertes oder durch Methyl substituiertes Piperazin, Tetrahydrothiazin oder Tetrahydropyridin,
R³ steht für Phenyl, das einfach bis dreifach, gleichartig oder verschieden substituiert sein kann durch
Fluor, Chlor, Brom, Cyano, Nitro, Formyl, Methyl, Ethyl, n- oder i-Propyl, n-, i-, s- oder t-Butyl, Allyl, Propargyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Allyloxy, Propargyloxy, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Iodpropargyloxy, Methylamino, Ethylamino, n- oder i-Propylamino, Dimethylamino, Diethylamino, Acetyl, Propionyl, Acetyloxy, Methoxycarbonyl, Ethoxycarbonyl, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl,
oder einfach substituiert sein kann durch zweifach in ortho-Stellung verknüpftes Propan-1,3-diyl, Methylendioxy oder Ethylendioxy, wobei diese Reste einfach bis vierfach, gleichartig oder verschieden substituiert sein können durch Fluor, Chlor, Methyl, Ethyl, n-Propyl i-Propyl und/oder Trifluormethyl;
R⁴ steht für Methyl, Ethyl, n- oder i-Propyl, n-, i-, s- oder t-Butyl, Allyl, Propargyl, Benzyl oder Chlorbenzyl;
X steht für Fluor oder Chlor.

3. Triazolopyrimidine der Formel (1) gemäß Anspruch 2, wobei
R3 für 2-substituiertes, 2,4-disubstituiertes, 2,6-disubstituiertes oder 2,4,6-trisubstituiertes Phenyl steht.

4. Triazolopyrimidine der Formel (1) gemäß Anspruch 2, wobei
R⁴ für Methyl und
X für Chlor steht.

5. Triazolopyrimidine der Formel (I) gemäß Anspruch 2, wobei
R¹ und R² gemeinsam mit dem Stickstoffatom, an welches sie gebunden sind, für gegebenenfalls substituiertes Piperidinyl stehen.

6. Triazolopyrimidine der Formel (I) gemäß Anspruch 2, wobei
R² für Wasserstoff, Methyl, Ethyl oder iso-Propyl steht.

7. Verfahren zur Herstellung von Triazolopyrimidinen der Formel (1) gemäß einem oder mehreren der Ansprüche 1 bis 6, **dadurch gekennzeichnet, dass** man
a) Dihalogen-triazolopyrimidine der Formel 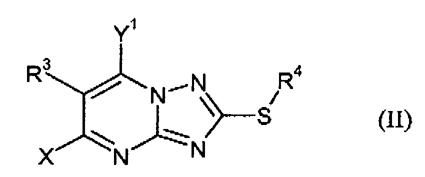 in welcher
R³, R⁴ und X die oben angegebenen Bedeutungen haben und
Y¹ für Halogen steht,
mit Aminen der Formel 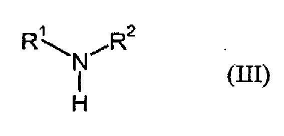 in welcher
R¹ und R² die oben angegebenen Bedeutungen haben,
gegebenenfalls in Gegenwart eines Verdünnungsmittels und gegebenenfalls in Gegenwart eines Säureakzeptors umsetzt,
oder
b) Triazolopyrimidine der Formel 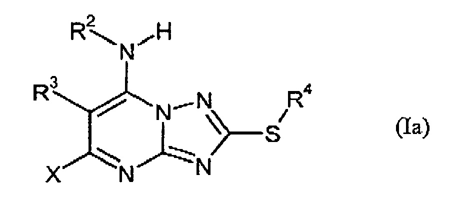 in welcher
R², R³, R⁴ und X die oben angegebenen Bedeutungen haben,
mit Sulfensäurehalogeniden der Formel
Y²-S-R⁵ (IV)
in welcher
R⁵ die oben angegebenen Bedeutungen hat und
Y² für Halogen steht,
gegebenenfalls in Gegenwart eines Verdünnungsmittels und gegebenenfalls in Gegenwart eines Säureakzeptors umsetzt,
oder
c) Triazolopyrimidine der Formel 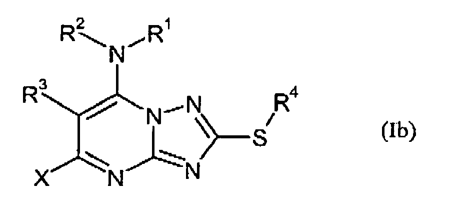 in welcher
R¹, R², R³, R⁴ und X die oben angegebenen Bedeutungen haben,
mit Sauerstoff abgebenden Oxidationsmitteln gegebenenfalls in Gegenwart eines Verdünnungsmittels und gegebenenfalls in Gegenwart eines Katalysators umsetzt,
und gegebenenfalls an die so erhaltenen Verbindungen der Formel (1), in denen
R¹ für Amino steht,
eine Säure addiert.

8. Mittel zur Bekämpfung von unerwünschten Mikroorganismen, **gekennzeichnet durch** einen Gehalt an mindestens einem Triazolopyrimidin der Formel (I) gemäß einem oder mehreren der Ansprüche 1 bis 6 bzw. an einem Säureadditions-Salz davon neben Streckmitteln und/oder oberflächenaktiven Stoffen.

9. Verwendung von Triazolopyrimidinen der Formel (1) gemäß einem oder mehreren der Ansprüche 1 bis 6 bzw. von deren Säureadditions-Salzen zur Bekämpfung von unerwünschten Mikroorganismen.

10. Verfahren zur Bekämpfung von unerwünschten Mikroorganismen, **dadurch gekennzeichnet, dass** man Triazolopyrimidine der Formel (T) gemäß einem oder mehreren der Ansprüche 1 bis 6 bzw. deren Säureadditions-Salze auf die unerwünschten Mikroorganismen und/oder deren Lebensraum ausbringt.

11. Verfahren zur Herstellung von Mitteln zur Bekämpfung von unerwünschten Mikroorganismen, **dadurch gekennzeichnet, dass** man Triazolopyrimidine der Formel (I) gemäß einem oder mehreren der Ansprüche 1 bis 6 bzw. deren Säureadditions-Salze mit Streckmitteln und/oder oberflächenaktiven Stoffen vermischt.

## Claims

1. Triazolopyrimidines of the formula (I) 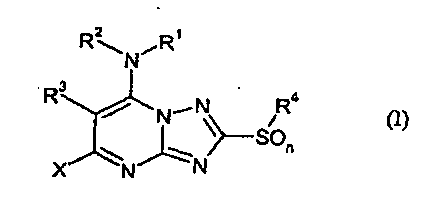 where the symbols and indices are as defined below:
R¹ represents amino;
represents alkyl having 1 to 6 carbon atoms which is optionally substituted by halogen, cycloalkyl, cyano, phenyl, heterocyclyl, alkoxy having 1 to 4 carbon atoms, alkylthio having 1 to 4 carbon atoms, oxo, hydroximino and/or alkoximino having 1 to 4 carbon atoms,
represents alkyl having 1 to 6 carbon atoms which is optionally substituted by hydroxyl, alkenyloxy having 2 to 4 carbon atoms, alkoxycarbonyl having 1 to 4 carbon atoms,
represents alkenyl having 2 to 6 carbon atoms which is optionally substituted by halogen, cycloalkyl, cyano, phenyl and/or heterocyclyl,
represents alkynyl having 2 to 6 carbon atoms which is optionally substituted by halogen, cycloalkyl, cyano, phenyl and/or heterocyclyl,
represents cycloalkyl having 3 to 7 carbon atoms which is optionally substituted by halogen, cycloalkyl, cyano, phenyl and/or heterocyclyl,
represents alkoxy having 1 to 7 carbon atoms which is optionally substituted by halogen, cycloalkyl, cyano, phenyl and/or heterocyclyl,
represents alkenyloxy having 2 to 6 carbon atoms which is optionally substituted by halogen, cycloalkyl, cyano, phenyl and/or heterocyclyl,
represents alkynyloxy having 2 to 6 carbon atoms which is optionally substituted by halogen, cycloalkyl, cyano, phenyl and/or heterocyclyl,
represents cycloalkyloxy having 3 to 7 carbon atoms which is optionally substituted by halogen, cycloalkyl, cyano, phenyl and/or heterocyclyl,
represents alkylamino having 1 to 7 carbon atoms which is optionally substituted by halogen, cycloalkyl, cyano, phenyl and/or heterocyclyl,
represents dialkylamino having 1 to 7 carbon atoms in each of the alkyl radicals which is optionally substituted by halogen, cycloalkyl, cyano, phenyl and/or heterocyclyl,
represents alkenylamino having 2 to 6 carbon atoms which is optionally substituted by halogen, cycloalkyl, cyano, phenyl and/or heterocyclyl,
represents alkynylamino having 2 to 6 carbon atoms which is optionally substituted by halogen, cycloalkyl, cyano, phenyl and/or heterocyclyl,
represents cycloalkylamino having 3 to 7 carbon atoms which is optionally substituted by halogen, cycloalkyl, cyano, phenyl and/or heterocyclyl,
represents N-cycloalkyl-N-alkylamino having 3 to 7 carbon atoms in the cycloalkyl moiety and 1 to 7 carbon atoms in the alkyl moiety which is optionally substituted by halogen, cycloalkyl, cyano, phenyl and/or heterocyclyl,
represents alkylideneamino having 2 to 6 carbon atoms which is optionally substituted by halogen, cycloalkyl, cyano, phenyl and/or heterocyclyl,
represents heterocyclyl having 5 or 6 ring members which is optionally substituted by halogen, alkyl, cycloalkyl, cyano, phenyl and/or heterocyclyl or represents -S-R⁵, in which
R⁵ represents alkyl having 1 to 6 carbon atoms which is optionally substituted by halogen, cycloalkyl, cyano, phenyl and/or heterocyclyl,
represents alkenyl having 2 to 6 carbon atoms which is optionally substituted by halogen, cycloalkyl, cyano, phenyl and/or heterocyclyl,
represents alkynyl having 2 to 6 carbon atoms which is optionally substituted by halogen, cycloalkyl, cyano, phenyl and/or heterocyclyl, or
represents cycloalkyl having 3 to 7 carbon atoms which is optionally substituted by halogen, cycloalkyl, cyano, phenyl and/or heterocyclyl,
where the heterocyclyl radicals mentioned above may be mono- to trisubstituted by identical or different substituents from the group consisting of halogen, alkyl having 1 to 4 carbon atoms, haloalkyl having 1 or 2 carbon atoms and 1 to 5 halogen atoms, alkoxy having 1 to 4 carbon atoms, alkylthio having 1 to 4 carbon atoms, haloalkoxy having 1 or 2 carbon atoms and 1 to 5 halogen atoms, haloalkylthio having 1 or 2 carbon atoms and 1 to 5 halogen atoms and phenyl,
and where the phenyl radicals mentioned above may be mono- to trisubstituted by identical or different substituents from the group consisting of
halogen, cyano, nitro, amino, hydroxyl, formyl, carboxyl, carbamoyl, thiocarbamoyl;
in each case straight-chain or branched alkyl, alkoxy, alkylthio, alkylsulphinyl or alkylsulphonyl having in each case 1 to 6 carbon atoms;
in each case straight-chain or branched alkenyl or alkenyloxy having in each case 2 to 6 carbon atoms;
in each case straight-chain or branched haloalkyl, haloalkoxy, haloalkylthio, haloalkylsulphinyl or haloalkylsulphonyl having in each case 1 to 6 carbon atoms and 1 to 13 identical or different halogen atoms;
in each case straight-chain or branched haloalkenyl or haloalkenyloxy having in each case 2 to 6 carbon atoms and 1 to 13 identical or different halogen atoms;
in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties;
cycloalkyl having 3 to 6 carbon atoms,
or may be monosubstituted by alkylene having 3 or 4 carbon atoms or dioxyalkylene having 1 or 2 carbon atoms, where these radicals are doubly attached in the ortho-position and may be mono- to tetrasubstituted by identical or different substituents from the group consisting of halogen, alkyl having 1 to 4 carbon atoms and haloalkyl having 1 to 4 carbon atoms and 1 to 9 identical or different halogen atoms;
R² represents hydrogen,
represents alkyl having 1 to 4 carbon atoms which is optionally substituted by halogen, alkoxy having 1 to 4 carbon atoms, alkylthio having 1 to 4 carbon atoms, oxo, hydroximino, alkoximino having 1 to 4 carbon atoms and/or cycloalkyl having 3 to 6 carbon atoms,
represents alkenyl having 2 to 4 carbon atoms which is optionally substituted by halogen and/or cycloalkyl having 3 to 6 carbon atoms or
represents alkynyl having 2 to 4 carbon atoms which is optionally substituted by halogen and/or cycloalkyl having 3 to 6 carbon atoms or
represents cycloalkyl having 3 to 6 carbon atoms which is optionally substituted by halogen and/or alkyl having 1 to 4 carbon atoms; or
R¹ and R² together with the nitrogen atom to which they are attached represent a 3- to 6-membered heterocyclic ring which may be mono- to trisubstituted by identical or different substituents from the group consisting of
alkyl having 1 to 4 carbon atoms,
haloalkyl having 1 to 4 carbon atoms and 1 to 9 identical or different halogen atoms,
fluorine, chlorine, bromine, iodine, hydroxyl, cyano, oxo;
alkylcarbonyl having 1 to 4 carbon atoms,
alkoxycarbonyl having 1 to 4 carbon atoms,
aminoalkyl having 1 to 4 carbon atoms,
alkylcarbonylamino having 1 to 4 carbon atoms or
onto which a phenyl ring is fused;
R³ represents phenyl which may be mono- to pentasubstituted by identical or different substituents from the group consisting of
halogen, cyano, nitro, amino, hydroxyl, formyl, carboxyl, carbamoyl, thiocarbamoyl;
in each case straight-chain or branched alkyl, alkoxy, alkylthio, alkylsulphinyl or alkylsulphonyl having in each case 1 to 6 carbon atoms;
in each case straight-chain or branched alkenyl or alkenyloxy having in each case 2 to 6 carbon atoms;
in each case straight-chain or branched haloalkyl, haloalkoxy, haloalkylthio, haloalkylsulphinyl or haloalkylsulphonyl having in each case 1 to 6 carbon atoms and 1 to 13 identical or different halogen atoms;
in each case straight-chain or branched haloalkenyl or haloalkenyloxy having in each case 2 to 6 carbon atoms and 1 to 13 identical or different halogen atoms;
in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties;
cycloalkyl having 3 to 6 carbon atoms,
or may be monosubstituted by alkylene having 3 or 4 carbon atoms or dioxyalkylene having 1 or 2 carbon atoms, where these radicals are doubly attached in the ortho-position and may be mono- to tetrasubstituted by identical or different substituents from the group consisting of halogen, alkyl having 1 to 4 carbon atoms and haloalkyl having 1 to 4 carbon atoms and 1 to 9 identical or different halogen atoms;
R⁴ represents alkyl having 1 to 6 carbon atoms which is optionally substituted by halogen and/or cyano,
represents alkenyl having 3 to 6 carbon atoms which is optionally substituted by halogen and/or cyano,
represents alkynyl having 3 to 6 carbon atoms which is optionally substituted by halogen and/or cyano or
represents optionally halogen-substituted aralkyl having 6 to 10 carbon atoms in the aryl moiety and 1 to 4 carbon atoms in the aryl moiety and 1 to 4 carbon atoms in the alkyl moiety;
X represents fluorine, chlorine or bromine;
n represents 0, 1 or 2.

2. Triazolopyrimidines of the formula (I) according to Claim 1, where the symbols and indices are as defined below:
R¹ represents methyl, ethyl, n-propyl, i-propyl, n-, i-, s- or t-butyl, methoxymethyl, 2-methoxyethyl, methylthiomethyl, 2-methylthioethyl, hydroximinomethyl, methoximinomethyl, acetylmethyl, 2-(hydroximino)-propyl, 2-(methoximino)propyl,
or
represents 2-ethoxycarbonylethyl, 2-vinyloxyethyl, 2-hydroxyiminoethyl, 2-methoxyiminoethyl, 2-hydroxyethyl, 2-chloroethyl, 2-methyl-1-propyl, 1,2-dimethylpropyl, thienylmethyl, amino;
or
represents allyl, 2-methylprop-2-enyl, propargyl, 2,2,2-trifluoroethyl, 1-(trifluoromethyl)ethyl, 3,3,3-trifluoropropyl, cyclopentyl, cyclohexyl, cyclopropylmethyl, cyclobutylmethyl, cyclopentylmethyl or cyclohexylmethyl, methoxy, ethoxy, n- or i-propoxy, n-, i-, s- or t-butoxy, allyloxy, propargyloxy, cyclopropyloxy, cyclobutyloxy, cyclopentyloxy or cyclohexyloxy, difluoromethoxy, trifluoromethoxy, difluorochloromethoxy, trifluoroethoxy, methylamino, ethylamino, n- or i-propylamino, n-, i-, s- or t-butylamino, dimethylamino, diethylamino, trifluoroethylamino, cyclohexylmethylamino, 2-cyanoethylamino, allylamino, 1-cyclopropylethylamino, cyclopropylamino, cyclobutylamino, cyclopentylamino, cyclohexylamino, 1-methylethylideneamino, benzyloxy, piperidinyl, morpholinyl, pyridylmethoxy, thiazolylmethoxy or represents -S-R⁵, in which
R^{*5*} represents methyl, ethyl, n- or i-propyl, difluoromethyl, difluorochloromethyl, dichlorofluoromethyl or trifluoromethyl,
where the thiazolyl and pyridyl radicals mentioned above may, in the case of thiazolyl, be mono- or disubstituted and, in the case of pyridyl, mono- to trisubstituted, in each case by identical or different substituents from the group consisting of fluorine, chlorine, bromine, methyl, ethyl, n- or i-propyl, n-, i-, s- or t-butyl, methoxy, ethoxy, n- or i-propoxy, n-, i-, s- or t-butoxy, methylthio, ethylthio, n- or i-propylthio, difluoromethoxy, trifluoromethoxy, difluorochloromethoxy, trifluoroethoxy, difluoromethylthio, difluorochloromethylthio, dichlorofluoromethylthio, trifluoromethylthio and phenyl,
and where the benzyloxy mentioned above may be mono- to trisubstituted in the phenyl moiety by identical or different substituents from the group consisting of
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl, trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl, ethoximinoethyl, cyclopropyl, cyclobutyl, cyclopentyl and cyclohexyl,
or may be monosubstituted by propane-1,3-diyl, methylenedioxy or ethylenedioxy, where these radicals are doubly attached in the ortho-position and may be mono- to tetrasubstituted by identical or different substituents from the group consisting of fluorine, chlorine, methyl, ethyl, n-propyl, i-propyl and trifluoromethyl;
R² represents hydrogen, methyl, ethyl, n- or i-propyl, n-, i-, s- or t-butyl, cyclopropyl, methoxymethyl, 2-methoxyethyl, methylthiomethyl, 2-methylthioethyl, hydroximinomethyl, methoximinomethyl, acetylmethyl, 2-hydroximinopropyl, 2-methoximinopropyl,
or
represents allyl, propargyl, 2,2,2-trifluoroethyl, 1-(1,1,1-trifluoromethyl)ethyl, cyclopropylmethyl, cyclobutylmethyl, cyclopentylmethyl or cyclohexylmethyl; or
R¹ and R² together with the nitrogen atom to which they are attached
represent optionally methyl-, ethyl- or trifluoroethyl-substituted pyrrolyl, piperidinyl, morpholinyl or piperazinyl,
represent piperidinyl which is mono- to trisubstituted by fluorine, chlorine, bromine, hydroxyl, oxo, methoxy, amino, methylamino, dimethylamino, acetylamino, chloromethyl, dichloromethyl, fluoromethyl, trifluoromethyl, aminomethyl, methoxycarbonyl, methylcarbonyl, morpholinyl, 1,3-dioxolanyl, tert-butoxycarbonylamino,
represent phenyl-fused piperidinyl,
represent pyrrolidinyl which is unsubstituted or mono- to trisubstituted by hydroxyl, dimethylamino, acetylamino, 1,3-propanediyl,
represent unsubstituted or methyl-substituted piperazine, tetrahydrothiazine or tetrahydropyridine,
R³ represents phenyl which may be mono- to trisubstituted by identical or different substituents from the group consisting of
fluorine, chlorine, bromine, cyano, nitro, formyl, methyl, ethyl, n- or i-propyl, n-, i-, s- or t-butyl, allyl, propargyl, methoxy, ethoxy, n- or i-propoxy, methylthio, ethylthio, n- or i-propylthio, methylsulphinyl, ethylsulphinyl, methylsulphonyl, ethylsulphonyl, allyloxy, propargyloxy, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl, trifluoromethylsulphonyl, trichloroethynyloxy, trifluoroethynyloxy, chloroallyloxy, iodopropargyloxy, methylamino, ethylamino, n- or i-propylamino, dimethylamino, diethylamino, acetyl, propionyl, acetyloxy, methoxycarbonyl, ethoxycarbonyl, hydroximinomethyl, hydroximinoethyl, methoximinomethyl, ethoximinomethyl, methoximinoethyl, ethoximinoethyl, cyclopropyl, cyclobutyl, cyclopentyl and cyclohexyl,
or may be monosubstituted by propane-1,3-diyl, methylenedioxy or ethylenedioxy, where these radicals are doubly attached in the ortho-position and may be mono- to tetrasubstituted by identical or different substituents from the group consisting of fluorine, chlorine, methyl, ethyl, n-propyl, i-propyl and trifluoromethyl;
R⁴ represents methyl, ethyl, n- or i-propyl, n-, i-, s- or t-butyl, allyl, propargyl, benzyl or chlorobenzyl;
X represents fluorine or chlorine.

3. Triazolopyrimidines of the formula (I) according to Claim 2, where
R³ represents 2-substituted, 2,4-disubstituted, 2,6-disubstituted or 2,4,6-trisubstituted phenyl.

4. Triazolopyrimidines of the formula (I) according to Claim 2, where
R⁴ represents methyl and
X represents chlorine.

5. Triazolopyrimidines of the formula (I) according to Claim 2, where
R¹ and R² together with the nitrogen atom to which they are attached represent optionally substituted piperidinyl.

6. Triazolopyrimidines of the formula (I) according to Claim 2, where
R² represents hydrogen, methyl, ethyl or isopropyl.

7. Process for preparing triazolopyrimidines of the formula (I) according to one or more of Claims 1 to 6, **characterized in that**
a) dihalotriazolopyrimidines of the formula 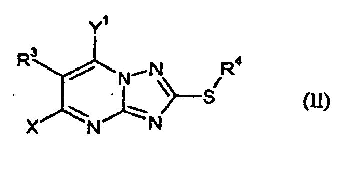 in which
R³, R⁴ and X are as defined above and
Y¹ represents halogen
are reacted with amines of the formula 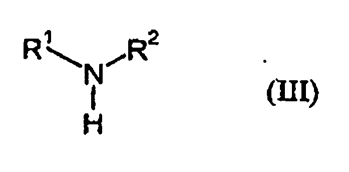 in which
R¹ and R² are as defined above,
if appropriate in the presence of a diluent and if appropriate in the presence of an acid acceptor,
or
b) triazolopyrimidines of the formula 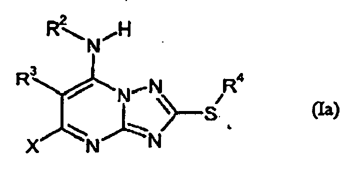 in which
R², R³, R⁴ and X are as defined above
are reacted with sulphenyl halides of the formula
Y²-S-R⁵ (IV)
in which
R⁵ is as defined above and
Y² represents halogen,
if appropriate in the presence of a diluent and if appropriate in the presence of an acid acceptor,
or
c) triazolopyrimidines of the formula 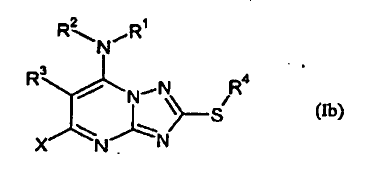 in which
R¹, R², R³, R⁴ and X are as defined above
are reacted with oxygen-releasing oxidizing agents, if appropriate in the presence of a diluent and if appropriate in the presence of a catalyst,
and, if appropriate, an acid is added to the resulting compounds of the formula (I) in which
R¹ is amino.

8. Compositions for controlling unwanted microorganisms, **characterized in that** they comprise at least one triazolopyrimidine of the formula (I) according to one or more of Claims 1 to 6 or an acid addition salt thereof, in addition to extenders and/or surfactants.

9. Use of triazolopyrimidines of the formula (I) according to one or more of Claims 1 to 6 or of acid addition salts thereof for controlling unwanted microorganisms.

10. Method for controlling unwanted microorganisms, **characterized in that** triazolopyrimidines of the formula (I) according to one or more of Claims 1 to 6 or acid addition salts thereof are applied to the unwanted microorganisms and/or their habitat.

11. Process for preparing compositions for controlling unwanted microorganisms, **characterized in that** triazolopyrimidines of the formula (I) according to one or more of Claims 1 to 6 or acid addition salts thereof are mixed with extenders and/or surfactants.

## Revendications

1. Triazolopyrimidines de formule (I), 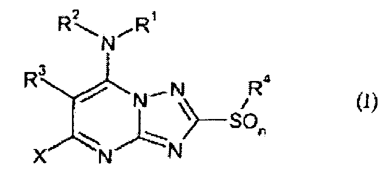 dans laquelle les symboles et les indices ont la définition suivante :
R¹ représente un groupe amino ;
un reste alkyle ayant 1 à 6 atomes de carbone, éventuellement substitué par un halogène, un radical cycloalkyle, cyano, phényle, hétérocyclyle, alkoxy ayant 1 à 4 atomes de carbone, alkylthio ayant 1 à 4 atomes de carbone, oxo, hydroxoimino et/ou alkoximino ayant 1 à 4 atomes de carbone,
un reste alkyle ayant 1 à 6 atomes de carbone, éventuellement substitué par un radical hydroxy, alcényloxy ayant 2 à 4 atomes de carbone, alkoxycarbonyle ayant 1 à 4 atomes de carbone,
un reste alcényle ayant 2 à 6 atomes de carbone, éventuellement substitué par un halogène, un radical cycloalkyle, cyano, phényle et/ou hétérocyclyle,
un reste alcynyle ayant 2 à 6 atomes de carbone, éventuellement substitué par un halogène, un radical cycloalkyle, cyano, phényle et/ou hétérocyclyle,
un reste cycloalkyle ayant 3 à 7 atomes de carbone, éventuellement substitué par un halogène, un radical cycloalkyle, cyano, phényle et/ou hétérocyclyle,
un reste alkoxy ayant 1 à 7 atomes de carbone, éventuellement substitué par un halogène, un radical cycloalkyle, cyano, phényle et/ou hétérocyclyle,
un reste alcényloxy ayant 2 à 6 atomes de carbone, éventuellement substitué par un halogène, un radical cycloalkyle, cyano, phényle et/ou hétérocyclyle,
un reste alcynyloxy ayant 2 à 6 atomes de carbone, éventuellement substitué par un halogène, un radical cycloalkyle, cyano, phényle et/ou hétérocyclyle,
un reste cycloalkyloxy ayant 3 à 7 atomes de carbone, éventuellement substitué par un halogène, un radical cycloalkyle, cyano, phényle et/ou hétérocyclyle,
un reste alkylamino ayant 1 à 7 atomes de carbone, éventuellement substitué par un halogène, un radical cycloalkyle, cyano, phényle et/ou hétérocyclyle,
un reste dialkylamino ayant 1 à 7 atomes de carbone dans chacun des groupes alkyle, éventuellement substitué par un halogène, un radical cycloalkyle, cyano, phényle et/ou hétérocyclyle,
un reste alcénylamino ayant 2 à 6 atomes de carbone, éventuellement substitué par un halogène, un radical cycloalkyle, cyano, phényle et/ou hétérocyclyle,
un reste alcynylamino ayant 2 à 6 atomes de carbone, éventuellement substitué par un halogène, un radical cycloalkyle, cyano, phényle et/ou hétérocyclyle,
un reste cycloalkylamino ayant 3 à 7 atomes de carbone, éventuellement substitué par un halogène, un radical cycloalkyle, cyano, phényle et/ou hétérocyclyle,
un reste N-cycloalkyl-N-alkylamino ayant 3 à 7 atomes de carbone dans la partie cycloalkyle et 1 à 7 atomes de carbone dans la partie alkyle, éventuellement substitué par un halogène, un radical cycloalkyle, cyano, phényle et/ou hétérocyclyle,
un reste alkylidène-amino ayant 2 à 6 atomes de carbone, éventuellement substitué par un halogène, un radical cycloalkyle, cyano, phényle et/ou hétérocyclyle,
un reste hétérocyclyle à noyau de 5 ou 6 chaînons, éventuellement substitué par un halogène, un radical alkyle, cycloalkyle, cyano, phényle et/ou hétérocyclyle, ou un groupe -S-R⁵, dans lequel
R⁵ désigne un reste alkyle ayant 1 à 6 atomes de carbone, éventuellement substitué par un halogène,
un radical cycloalkyle, cyano, phényle et/ou hétérocyclyle,
un reste alcényle ayant 2 à 6 atomes de carbone, éventuellement substitué par un halogène, un radical cycloalkyle, cyano, phényle et/ou hétérocyclyle,
un reste alcynyle ayant 2 à 6 atomes de carbone, éventuellement substitué par un halogène, un radical cycloalkyle, cyano, phényle et/ou hétérocyclyle, ou bien
un reste cycloalkyle ayant 3 à 7 atomes de carbone, éventuellement substitué par un halogène, un radical cycloalkyle, cyano, phényle et/ou hétérocyclyle,
les restes hétérocyclyle mentionnés ci-dessus pouvant être substitués une à trois fois, identiques ou différentes, par un halogène, un radical alkyle ayant 1 à 4 atomes de carbone, halogénalkyle ayant 1 ou 2 atomes de carbone et 1 à 5 atomes d'halogène, un radical alkoxy ayant 1 à 4 atomes de carbone, un radical alkylthio ayant 1 à 4 atomes de carbone, un radical halogénalkoxy ayant 1 ou 2 atomes de carbone et 1 à 5 atomes d'halogène, un radical halogénalkylthio ayant 1 ou 2 atomes de carbone et 1 à 5 atomes d'halogène et/ou un radical phényle,
et les restes phényle mentionnés ci-dessus pouvant être substitués une à trois fois, identiques ou différentes, par
un halogène, un radical cyano, nitro, amino, hydroxy, formyle, carboxy, carbamoyle, thiocarbamoyle ;
un radical alkyle, alkoxy, alkylthio, alkylsulfinyle ou alkylsulfonyle ayant chacun 1 à 6 atomes de carbone et chacun étant linéaire ou ramifié ;
un radical alcényle ou alcényloxy ayant chacun 2 à 6 atomes de carbone et chacun étant linéaire ou ramifié ;
un radical halogénalkyle, halogénalkoxy, halogénalkylthio, halogénalkylsulfinyle ou halogénalkylsulfonyle ayant chacun 1 à 6 atomes de carbone et 1 à 13 atomes d'halogène identiques ou différents et chacun étant linéaire ou ramifié ;
un radical halogénalcényle ou halogénalcényloxy ayant chacun 2 à 6 atomes de carbone et 1 à 13 atomes d'halogène identiques ou différents et chacun étant linéaire ou ramifié ;
un radical alkylamino, dialkylamino, alkylcarbonyle, alkylcarbonyloxy, alkoxycarbonyle, alkylsulfonyloxy, hydroximinoalkyle ou alkoximinoalkyle ayant chacun 1 à 6 atomes de carbone dans les parties alkyle individuelles et chacun étant linéaire ou ramifié ;
un radical cycloalkyle ayant 3 à 6 atomes de carbone,
ou bien ils peuvent être substitués une fois par un reste alkylène ayant 3 ou 4 atomes de carbone liés deux fois en position ortho ou un reste dioxyalkylène ayant 1 ou 2 atomes de carbone, ces restes pouvant être substitués une à quatre fois identiques ou différentes par un halogène, un radical alkyle ayant 1 à 4 atomes de carbone et/ou un radical halogénalkyle ayant 1 à 4 atomes de carbone et 1 à 9 atomes d'halogène identiques ou différents ;
R² représente l'hydrogène,
un reste alkyle ayant 1 à 4 atomes de carbone éventuellement substitué par un halogène, un radical alkoxy ayant 1 à 4 atomes de carbone, alkylthio ayant 1 à 4 atomes de carbone, oxo, hydroximino, alkoximino ayant 1 à 4 atomes de carbone et/ou cycloalkyle ayant 3 à 6 atomes de carbone,
un reste alcényle ayant 2 à 4 atomes de carbone, éventuellement substitué par un halogène et/ou un radical cycloalkyle ayant 3 à 6 atomes de carbone, ou bien
un reste alcynyle ayant 2 à 4 atomes de carbone, éventuellement substitué par un halogène et/ou un radical cycloalkyle ayant 3 à 6 atomes de carbone, ou bien
un reste cycloalkyle ayant 3 à 6 atomes de carbone, éventuellement substitué par un halogène et/ou un radical alkyle ayant 1 à 4 atomes de carbone ; ou bien
R¹ et R² forment, conjointement avec l'atome d'azote auquel ils sont liés, un noyau hétérocyclique de 3 à 6 chaînons qui peut être substitué une à trois fois identiques ou différentes par
un radical alkyle ayant 1 à 4 atomes de carbone,
un radical halogénalkyle ayant 1 à 4 atomes de carbone et 1 à 9 atomes d'halogène identiques ou différents,
un radical fluoro, chloro, bromo, iodo, hydroxy, cyano, oxo ;
un radical alkylcarbonyle ayant 1 à 4 atomes de carbone,
un radical alkoxycarbonyle ayant 1 à 4 atomes de carbone,
un radical aminoalkyle ayant 1 à 4 atomes de carbone,
un radical alkylcarbonylamino ayant 1 à 4 atomes de carbone ou bien
il est condensé à un noyau phényle ;
R³ représente un reste phényle qui peut être substitué une à cinq fois identiques ou différentes par
un halogène, un radical cyano, nitro, amino, hydroxy, formyle, carboxy, carbamoyle, thiocarbamoyle ;
un radical alkyle, alkoxy, alkylthio, alkylsulfinyle ou alkylsulfonyle ayant chacun 1 à 6 atomes de carbone et
chacun étant linéaire ou ramifié ;
un radical alcényle ou alcényloxy ayant chacun 2 à 6 atomes de carbone et chacun étant linéaire ou ramifié ;
un radical halogénalkyle, halogénalkoxy, halogénalkylthio, halogénalkylsulfinyle ou halogénalkylsulfonyle ayant chacun 1 à 6 atomes de carbone et 1 à 13 atomes d'halogène identiques ou différents et chacun étant linéaire ou ramifié ;
un radical halogénalcényle ou halogénalcényloxy ayant chacun 2 à 6 atomes de carbone et 1 à 13 atomes d'halogène identiques ou différents et chacun étant linéaire ou ramifié ;
un radical alkylamino, dialkylamino, alkylcarbonyle, alkylcarbonyloxy, alkoxycarbonyle, alkylsulfonyloxy, hydroximinoalkyle ou alkoximinoalkyle ayant chacun 1 à 6 atomes de carbone dans les parties alkyle individuelles et chacun étant linéaire ou ramifié ;
un radical cycloalkyle ayant 3 à 6 atomes de carbone,
ou bien il peut être substitué une fois par un reste alkylène ayant 3 ou 4 atomes de carbone liés deux fois en position ortho ou un reste dioxyalkylène ayant 1 ou 2 atomes de carbone, ces restes pouvant être substitués une à quatre fois identiques ou différentes par un halogène, un radical alkyle ayant 1 à 4 atomes de carbone et/ou halogénalkyle ayant 1 à 4 atomes de carbone et 1 à 9 atomes d'halogène identiques ou différents ;
R⁴ représente un reste alkyle ayant 1 à 6 atomes de carbone, éventuellement substitué par un halogène et/ou un radical cyano,
un reste alcényle ayant 3 à 6 atomes de carbone, éventuellement substitué par un halogène et/ou un radical cyano,
un reste alcynyle ayant 3 à 6 atomes de carbone, éventuellement substitué par un halogène et/ou un radical cyano, ou bien
un reste aralkyle ayant 6 à 10 atomes de carbone dans la partie aryle et 1 à 4 atomes de carbone dans la partie aryle et 1 à 4 atomes de carbone dans la partie alkyle et étant éventuellement substitué par un halogène ;
x représente le fluor, le chlore ou le brome
n a la valeur 0, 1 ou 2.

2. Triazolopyrimidines de formule (I) suivant la revendication 1, formule dans laquelle les symboles et les indices ont la définition suivante :
R¹ représente un reste méthyle, éthyle, n-propyle, isopropyle, n-butyle, isobutyle, sec.-butyle, tertiobutyle, méthoxyméthyle, 2-méthoxy-éthyle, méthylthio-méthyle, 2-méthylthio-méthyle, hydroximino-méthyle, méthoximino-méthyle, acétylméthyle, 2-(hydroximino)propyle, 2-(méthoximino)propyle. ou bien
un reste 2-éthoxycarbonyl-éthyle, 2-vinyloxy-éthyle, 2-hydroxyimino-éthyle, 2-méthoxyimino-éthyle, 2-hydroxyéthyle, 2-chloréthyle, 2-méthyl-1-propyle, 1,2-diméthylpropyle, thiényl-méthyle, amino ;
ou bien
un reste allyle, 2-méthyl-prop-2-ényle, propargyle, 2,2,2-trifluoréthyle, 1-(trifluorométhyl)-éthyle, 3,3,3-trifluoropropyle, cyclopentyle, cyclohexyle, cyclopropylméthyle, cyclobutylméthyle, cyclopentylméthyle ou cyclohexylméthyle, un reste méthoxy, éthoxy, n-propoxy, isopropoxy, n-butoxy, isobutoxy, sec.-butoxy ou tertiobutoxy, allyloxy, propargyloxy, cyclopropyloxy, cyclobutyloxy, cyclopentyloxy ou cyclohexyloxy, un reste difluorométhoxy, trifluorométhoxy, difluorochlorométhoxy, trifluoréthoxy, méthylamino, éthylamino, n-propylamino ou isopropylamino, n-butylamino, isobutylamino, sec.-butylamino ou tertiobutylamino, diméthylamino, diéthylamino, trifluoréthylamino, cyclohexylméthylamino, 2-cyanéthylamino, allylamino, 1-cyclopropyléthylamino, cyclopropylamino, cyclobutylamino, cyclopentylamino, cyclohexylamino, 1-méthyléthylidénamino, benzyloxy, pipéridinyle, morpholinyle, pyridylméthoxy, thiazolylméthoxy ou un groupe -S-R⁵, dans lequel
R⁵ est un reste méthyle, éthyle, n-propyle, isopropyle, difluorométhyle, difluorochlorométhyle, dichlorofluorométhyle ou trifluorométhyle,
les restes thiazolyle et pyridyle mentionnés ci-dessus pouvant être substitués une ou deux fois dans le cas du reste thiazolyle et une à trois fois dans le cas du reste pyridyle, de façon identique ou différente dans chaque cas, par un radical fluoro, chloro, bromo, méthyle, éthyle, n-propyle, isopropyle, n-butyle, isobutyle, sec.-butyle, tertiobutyle, méthoxy, éthoxy, n-propoxy, isopropoxy, n-butoxy, isobutoxy, sec.-butoxy, tertiobutoxy, méthylthio, éthylthio, n-propylthio, isopropylthio, difluorométhoxy, trifluorométhoxy, difluorochlorométhoxy, trifluoréthoxy, difluorométhylthio, difluorochlorométhylthio, dichlorofluorométhylthio, trifluorométhylthio et/ou phényle.
et le reste benzyloxy mentionné ci-dessus pouvant être substitué dans la partie phényle une à trois fois identiques ou différentes par
un radical fluoro, chloro, bromo, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle ou éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle, éthoximinoéthyle, cyclopropyle, cyclobutyle, cyclopentyle ou cyclohexyle,
ou bien il peut être substitué une fois par un reste propane-1,3-diyle lié deux fois en position ortho, un reste méthylènedioxy ou éthylènedioxy, ces restes pouvant être substitués une à quatre fois identiques ou différentes par du fluor, du chlore, un radical méthyle, éthyle, n-propyle, isopropyle et/ou trifluorométhyle ;
R² représente l'hydrogène, un reste méthyle, éthyle, n-propyle, isopropyle, n-butyle, isobutyle, sec.-butyle, tertiobutyle, cyclopropyle, méthoxy-méthyle, 2-méthoxyéthyle, méthylthio-méthyle, 2-méthylthio-méthyle, hydroximinométhyle, méthoximino-méthyle, acétylméthyle, 2-hydroximinopropyle, 2-méthoximino-propyle,
ou bien
un reste allyle, propargyle, 2,2,2-trifluoréthyle, 1-(1,1,1-trifluorométhyl)éthyle, cyclopropylméthyle, cyclobutylméthyle, cyclopentylméthyle ou cyclohexylméthyle ; ou bien
R¹ et R² forment, conjointement avec l'atome d'azote auquel ils sont liés,
un noyau pipéridinyle, morpholinyle, pipérazinyle ou pyrrolyle éventuellement substitué par un radical méthyle, éthyle ou trifluoréthyle,
un noyau pipéridinyle substitué une à trois fois par du fluor, du chlore, du brome, un radical hydroxy, oxo, méthoxy, amino, méthylamino, diméthylamino, acétylamino, chlorométhyle, dichlorométhyle, fluorométhyle, trifluorométhyle, aminométhyle, méthoxycarbonyle, méthylcarbonyle, morpholinyle, 1,3-dioxolannyle, tertiobutoxycarbonylamino,
un noyau pipéridinyle condensé à un noyau phényle,
un noyau pyrrolidinyle non substitué ou substitué une à trois fois par un radical hydroxy, diméthylamino, acétylamino, 1,3-propanediyle,
un noyau pipérazine, tétrahydrothiazine ou tétrahydropyridine non substitué ou substitué par un radical méthyle,
R³ représente un reste phényle qui peut être substitué une à trois fois identiques ou différentes par
du fluor, du chlore, du brome, un radical cyano, nitro, formyle, méthyle, éthyle, n-propyle, isopropyle, n-butyle, isobutyle, sec.-butyle, tertiobutyle, allyle, propargyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, allyloxy, propargyloxy, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, trichloréthynyloxy, trifluoréthynyloxy, chlorallyloxy, iodopropargyloxy, méthylamino, éthylamino, n-propylamino, isopropylamino, diméthylamino, diéthylamino, acétyle, propionyle, acétyloxy, méthoxycarbonyle, éthoxycarbonyle, hydroximinométhyle, hydroximinoéthyle, méthoximinométhyle, éthoximinométhyle, méthoximinoéthyle, éthoximinoéthyle, cyclopropyle, cyclobutyle, cyclopentyle ou cyclohexyle,
ou peut être substitué une fois par un reste propane-1,3-diyle lié deux fois en position ortho, un reste méthylènedioxy ou éthylènedioxy, ces restes pouvant être substitués une à quatre fois identiques ou différentes par du fluor, du chlore, un radical méthyle, éthyle, n-propyle, isopropyle et/ou trifluorométhyle ;
R⁴ représente un reste méthyle, éthyle, n-propyle, isopropyle, n-butyle, isobutyle, sec.-butyle, tertiobutyle, allyle, propargyle, benzyle ou chlorobenzyle ;
X représente le fluor ou le chlore.

3. Triazolopyrimidines de formule (I) suivant la revendication 2, formule dans laquelle
R³ représente un reste phényle monosubstitué en position 2, disubstitué en positions 2, 4, disubstitué en positions 2, 6 ou trisubstitué en positions 2, 4, 6.

4. Triazolopyrimidines de formule (I) suivant la revendication 2, formule dans laquelle
R⁴ est un reste méthyle et
X représente le chlore.

5. Triazolopyrimidines de formule (I) suivant la revendication 2, formule dans laquelle
R¹ et R² forment, conjointement avec l'atome d'azote auquel ils sont liés, un noyau pipéridinyle éventuellement substitué.

6. Triazolopyrimidines de formule (I) suivant la revendication 2, formule dans laquelle
R² représente l'hydrogène, un reste méthyle, éthyle ou isopropyle.

7. Procédé de production de triazolopyrimidines de formule (I) suivant une ou plusieurs des revendications 1 à 6, **caractérisé en ce que** :
(a) on fait réagir des dihalogéno-triazolopyrimidines de formule 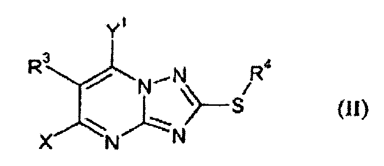 dans laquelle
R³, R⁴ et X ont les définitions indiquées ci-dessus et
Y¹ représente un halogène,
avec des amines de formule 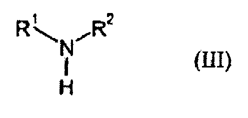 dans laquelle
R¹ et R² ont les définitions indiquées ci-dessus, éventuellement en présence d'un diluant et, le cas échéant, en présence d'un accepteur d'acide,
ou bien
b) on fait réagir des triazolopyrimidines de formule 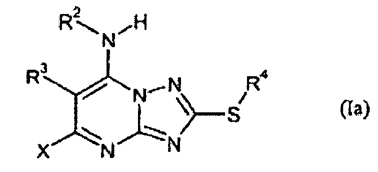 dans laquelle
R², R³, R⁴ et X ont les définitions indiquées ci-dessus,
avec des halogénures d'acides sulféniques de formule
Y²-S-R⁵ (IV)
dans laquelle
R⁵ a les définitions indiquées ci-dessus et
Y² représente un halogène,
éventuellement en présence d'un diluant et, le cas échéant, en présence d'un accepteur d'acide,
ou bien
c) on fait réagir des triazolopyrimidines de formule 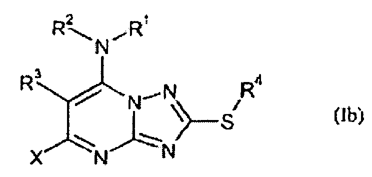 dans laquelle
R¹, R², R³, R⁴ et X ont les définitions indiquées ci-dessus,
avec des agents oxydants cédant de l'oxygène, éventuellement en présence d'un diluant et, le cas échéant, en présence d'un catalyseur,
et on additionne éventuellement un acide sur les composés ainsi obtenus de formule (I), dans lesquels
R¹ représente un groupe amino.

8. Compositions destinées à combattre des micro-organismes indésirables, **caractérisées par** une teneur en au moins une triazolopyrimidine de formule (I) suivant une ou plusieurs des revendications 1 à 6 ou en un sel d'addition d'acide de ce composé, à côté de diluants et/ou d'agents tensioactifs.

9. Utilisation de triazolopyrimidines de formule (I) suivant une ou plusieurs des revendications 1 à 6, ou de leurs sels d'addition d'acide pour combattre des micro-organismes indésirables.

10. Procédé de lutte contre des micro-organismes indésirables, **caractérisé en ce qu'**on épand des triazolopyrimidines de formule (I) suivant une ou plusieurs des revendications 1 à 6, ou leurs sels d'addition d'acide sur les micro-organismes indésirables et/ou sur leur milieu.

11. Procédé de préparation de compositions destinées à combattre des micro-organismes indésirables, **caractérisé en ce qu'**on mélange des triazolopyrimidines de formule (I) suivant une ou plusieurs des revendications 1 à 6, ou leurs sels d'addition d'acide avec des diluants et/ou des agents tensioactifs.
